(19) Europäisches Patentamt
European Patent Office
Office européen des brevets

(11) EP 1 783 221 A1

(12) **EUROPEAN PATENT APPLICATION**

(43) Date of publication:
**09.05.2007 Bulletin 2007/19**

(21) Application number: **06022533.1**

(22) Date of filing: **11.05.2001**

(51) Int Cl.:
***C12N 15/12*** *(2006.01)* ***C07K 14/47*** *(2006.01)*
***C12N 15/62*** *(2006.01)* ***C12N 5/10*** *(2006.01)*
***C12N 9/00*** *(2006.01)* ***C12N 15/86*** *(2006.01)*
***G01N 33/50*** *(2006.01)* ***C07H 15/24*** *(2006.01)*

(84) Designated Contracting States:
**AT BE CH CY DE DK ES FI FR GB GR IE IT LI LU MC NL PT SE TR**

(30) Priority: **11.05.2000 US 569327**

(62) Document number(s) of the earlier application(s) in accordance with Art. 76 EPC:
**01935420.8 / 1 283 884**

(71) Applicant: **MIGENIX Corp.**
**San Diego, CA 92121 (US)**

(72) Inventors:
- **Anderson, Christen M.**
  **Encinitas, CA 92024 (US)**
- **Carroll, Amy K.**
  **San Diego, CA92110 (US)**
- **Davis, Robert E.**
  **San Diego, CA92130 (US)**

(74) Representative: **Gowshall, Jonathan Vallance**
**Forrester & Boehmert**
**Pettenkoferstrasse 20-22**
**80336 München (DE)**

Remarks:
This application was filed on 27.10.2006 as a divisional application to the application mentioned under INID code 62.

(54) **Production of adenine nucleotide translocator (ANT), novel ANT ligands and screening assays therefor**

(57) Compositions and methods are provided for producing adenine nucleotide translocator (ANT) polypeptides and fusion proteins, including the production and use of recombinant expression constructs having a regulated promoter. ANT ligands and compositions and methods for identifying ANT ligands, agents that bind ANT and agents that interact with ANT are also disclosed.

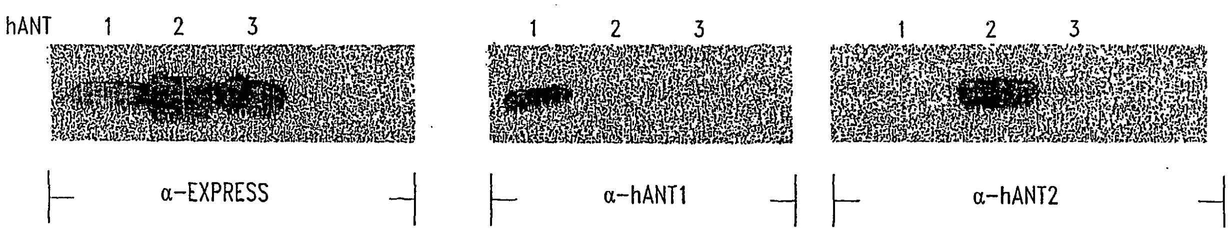


*Fig. 3*

EP 1 783 221 A1

## Description

FIELD OF THE INVENTION

The invention relates to the adenine nucleotide translocator (ANT) protein that is found in mitochondria of eukaryotic cells. More particularly, the invention relates to the production of ANT polypeptides and ANT fusion proteins using recombinant DNA technology; to novel labeled ligands for ANT proteins; and to assays useful for identifying and isolating ANT proteins and for screening compounds that interact with ANT, including high throughput screening.

BACKGROUND OF THE INVENTION

Mitochondria are the main energy source in cells of higher organisms, and these organelles provide direct and indirect biochemical regulation of a wide array of cellular respiratory, oxidative and metabolic processes. These include electron transport chain (ETC) activity, which drives oxidative phosphorylation to produce metabolic energy in the form of adenosine triphosphate (ATP), and which also underlies a central mitochondrial role in intracellular calcium homeostasis.

Mitochondrial ultrastructural characterization reveals the presence of an outer mitochondrial membrane that serves as an interface between the organelle and the cytosol, a highly folded inner mitochondrial membrane that appears to form attachments to the outer membrane at multiple sites, and an intermembrane space between the two mitochondrial membranes. The subcompartment within the inner mitochondrial membrane is commonly referred to as the mitochondrial matrix. (For a review, *see, e.g.*, Ernster et al., 1981 J. Cell Biol. 91:227s.) The cristae, originally postulated to occur as infoldings of the inner mitochondrial membrane, have recently been characterized using three-dimensional electron tomography as also including tube-like conduits that may form networks, and that can be connected to the inner membrane by open, circular (30 nm diameter) junctions (Perkins et al., 1997, *Journal of Structural Biology 119*:260). While the outer membrane is freely permeable to ionic and non-ionic solutes having molecular weights less than about ten kilodaltons, the inner mitochondrial membrane exhibits selective and regulated permeability for many small molecules, including certain cations, and is impermeable to large (> ~10 kDa) molecules.

Altered or defective mitochondrial activity, including but not limited to failure at any step of the ETC, may result in catastrophic mitochondrial collapse that has been termed "permeability transition" (PT) or "mitochondrial permeability transition" (MPT). According to generally accepted theories of mitochondrial function, proper ETC respiratory activity requires maintenance of an electrochemical potential ($\Delta\Psi m$) in the inner mitochondrial membrane by a coupled chemiosmotic mechanism. Altered or defective mitochondrial activity may dissipate this membrane potential, thereby preventing ATP biosynthesis and halting the production of a vital biochemical energy source. In addition, mitochondrial proteins such as cytochrome c may leak out of the mitochondria after permeability transition and may induce the genetically programmed cell suicide sequence known as apoptosis (Wilson, 1998 Cell Death Differen. 5:646-652) or programmed cell death (PCD).

MPT may result from direct or indirect effects of mitochondrial genes, gene products or related downstream mediator molecules and/or extramitochondrial genes, gene products or related downstream mediators, or from other known or unknown causes. Loss of mitochondrial potential therefore may be a critical event in the progression of diseases associated with altered mitochondrial function, including degenerative diseases.

Mitochondrial defects, which may include defects related to the discrete mitochondrial genome that resides in mitochondrial DNA and/or to the extramitochondrial genome, which includes nuclear chromosomal DNA and other extramitochondrial DNA, may contribute significantly to the pathogenesis of diseases associated with altered mitochondrial function. For example, alterations in the structural and/or functional properties of mitochondrial components comprised of subunits encoded directly or indirectly by mitochondrial and/or extramitochondrial DNA, including alterations deriving from genetic and/or environmental factors or alterations derived from cellular compensatory mechanisms, may play a role in the pathogenesis of any disease associated with altered mitochondrial function. A number of degenerative diseases are thought to be caused by, or to be associated with, alterations in mitochondrial function. These include Alzheimer's Disease (AD); diabetes mellitus; Parkinson's Disease; Huntington's disease; dystonia; Leber's hereditary optic neuropathy; schizophrenia; mitochondrial encephalopathy, lactic acidosis, and stroke (MELAS); cancer; psoriasis; hyperproliferative disorders; mitochondrial diabetes and deafness (MIDD) and myoclonic epilepsy ragged red fiber syndrome. The extensive list of additional diseases associated with altered mitochondrial function continues to expand as aberrant mitochondrial or mitonuclear activities are implicated in particular disease processes.

A hallmark pathology of AD and potentially other diseases associated with altered mitochondrial function is the death of selected cellular populations in particular affected tissues, which results from apoptosis (also referred to as "programmed cell death" or PCD) according to a growing body of evidence. Mitochondrial dysfunction is thought to be critical in the cascade of events leading to apoptosis in various cell types (Kroemer et al., FASEB J. 9:1277-87, 1995), and may be a cause of apoptotic cell death in neurons of the AD brain. Altered mitochondrial physiology may be among

the earliest events in PCD (Zamzami et al., J. Exp. Med. 182:367-77, 1995; Zamzami et al., J. Exp. Med 181:1661-72, 1995) and elevated reactive oxygen species (ROS) levels that result from such altered mitochondrial function may initiate the apoptotic cascade (Ausserer et al., Mol Cell. Biol. 14:5032-42, 1994).

Thus, in addition to their role in energy production in growing cells, mitochondria (or, at least, mitochondrial components) participate in apoptosis (Newmeyer et al., 1994, Cell 79:353-364; Liu et al., 1996, Cell 86:147-157). Apoptosis is apparently also required for, *inter alia,* normal development of the nervous system and proper functioning of the immune system. Moreover, some disease states are thought to be associated with either insufficient (*e.g.*, cancer, autoimmune diseases) or excessive (*e.g.*, stroke damage, AD-associated neurodegeneration) levels of apoptosis. For general reviews of apoptosis, and the role of mitochondria therein, see Green and Reed (1998, Science 281:1309-1312), Green (1998, Cell 94:695-698) and Kromer (1997, Nature Medicine 3:614-620). Hence, agents that effect apoptotic events, including those associated with mitochondrial components, might have a variety of palliative, prophylactic and therapeutic uses.

The adenine nucleotide translocase (ANT), a nuclear encoded mitochondrial protein, is reportedly the most abundant protein of the inner mitochondrial membrane, forming dimers that comprise up to 10% of the total mitochondrial protein in highly oxidative tissue like cardiac and skeletal muscle. Several isoforms of ANT are known, as discussed in more detail *infra.* ANT proteins mediate the exchange of ATP synthesized in the mitochondrial matrix for ADP in the cytosol. This nucleotide exchange is the most active transport system in aerobic cells, and is a critical component in maintaining cellular energy metabolism (for a review see Klingenberg, J. Bioenergetics and Biomembranes 25:447-457, 1993).

ANT has also been implicated as an important molecular component of the mitochondrial permeability transition (MPT) pore, a $Ca^{2+}$-regulated inner membrane channel that, as described *supra,* plays an important modulating role in apoptotic processes, as ANT inhibitors (such as atractyloside or bongkrekic acid) induce MPT under certain conditions.

Three human ANT isoforms have been described that appear to differ in their tissue expression patterns, and other mammalian ANT homologues have been described. See, *e.g.,* Wallace et al., 1998 in Mitochondria & Free Radicals in Neurodegenerative Diseases, Beal, Howell and Bodis-Wollner, Eds., Wiley-Liss, New York, pp. 283-307, and references cited therein.

Because the ANT isoforms have peptide sequences that are fairly homologous to each other, attempts to use biochemical protein purification techniques to purify individual isoforms of ANT have not been successful. The present invention overcomes this limitation by providing compositions and methods for the separate production of each individual ANT isoform. Using the human ANT isoforms as an example, non-mammalian host cells comprising an expression construct for a particular huANT isoform are used to overexpress the gene encoding that huANT isoform, resulting in the production of a particular huANT isoform in a host cell that lacks any endogenous huANT.

A particular ANT isoform that has been produced in this manner can be isolated or partially isolated from the proteins (and other biomolecules) of the host cell, thereby producing a composition of matter that (i) comprises a specific isoform of an ANT protein from an organism and (ii) does not comprise any of the other isoforms of ANT from that organism. For brevity's sake, such compositions are referred to herein as being "ANT-x specific," where "x" is a term that distinguishes the ANT of the composition from other ANT isoforms. , For example, an ANT-1 specific composition of matter comprises ANT-1 but lacks ANT-2 and ANT-3.

Unlike previous preparations of ANT proteins, ANT-x specific compositions can be examined and analyzed for biochemical and other properties of a particular ANT isoform (*i.e.,* ANT-x). Non-limiting examples include, for example, determining the biochemical kinetics of one or more ANT isoforms; examining the ability of a specific ANT isoform to form ANT multimers (e.g., dimers, trimers, tetramers, and the like) with itself or with other, separately introduced, ANT isoforms; and determining the ability of a given ANT isoform to alter the ATP/ADP concentration in the mitochondrial matrix and/or the cytoplasm or organelles. Altering the ATP/ADP concentration in the matrix can lead to secondary alterations in various mitochondrial activities, including but not limited to oxidative phosphorylation, rates of TCA cycle flux and/or flux of metabolic intermediates out of or into mitochondria. The present invention provides compositions and methods for determining the biochemical characteristics of specific ANT isoforms and the ability of each isoform to influence mitochondrial functions.

As inner mitochondrial membrane proteins are believed to possess multiple hydrophobic membrane spanning domains, ANT polypeptides may exhibit, *inter alia,* poor intracellular solubility, toxic accumulations and/or the formation of inclusion bodies and other deleterious effects on respiratory homeostasis within a host cell due to ANT biological activity. Consequently, those having ordinary skill in the art have heretofore been unable to produce ANT reliably or in sufficient quantities for a variety of uses, such as those provided herein. For example, successful recombinant expression of mammalian ANT polypeptides in bacterial cells has not been achieved, due to toxic effects of the ectopic protein and its sequestration into inclusion bodies (Miroux et al., 1996 J. Mol. Biol. 260:289). Because of the significance of mitochondria to respiratory, metabolic and apoptotic processes, and in view of the prominent role played by ANT in these and other mitochondrial activities, there is clearly a need for compositions and methods that permit the production of ANT proteins, including ANT fusion proteins; for novel ANT ligands; for methods to identify and isolate ANT proteins;

and for methods of identifying and isolating agents that interact with ANT.

The present invention fulfills these needs and provides other related advantages. These and other aspects of the present invention will become evident upon reference to the following detailed description and attached drawings. In addition, various references are set forth below which describe in more detail certain procedures or compositions (e.g., plasmids, vectors, etc.), and are therefore incorporated by reference in their entireties.

SUMMARY OF THE INVENTION

In its various aspects and embodiments the invention is directed to:

A recombinant expression construct comprising at least one regulated promoter operably linked to a first nucleic acid encoding an adenine nucleotide translocator polypeptide; further comprising at least one additional nucleic acid sequence that regulates transcription; wherein the additional nucleic acid sequence that regulates transcription encodes a repressor of said regulated promoter; wherein the adenine nucleotide translocator polypeptide comprises a human adenine nucleotide translocator polypeptide; wherein the human adenine nucleotide translocator polypeptide is ANT1; wherein the human adenine nucleotide translocator polypeptide is ANT2; wherein the adenine nucleotide translocator polypeptide is expressed as a fusion protein with a polypeptide product of a second nucleic acid sequence; wherein the polypeptide product of said second nucleic acid sequence is an enzyme; wherein said fusion protein localizes to membranes; wherein said membranes are mitochondrial membranes; wherein the adenine nucleotide translocator polypeptide is expressed as a fusion protein with at least one product of a second nucleic acid sequence encoding a polypeptide cleavable by a protease, said adenine nucleotide translocator polypeptide being separable from the fusion protein by cleavage with the protease; a host cell comprising a recombinant expression construct as provided; wherein the host cell is a prokaryotic cell; wherein the host cell is a eukaryotic cell; wherein the eukaryotic cell is a yeast cell, an insect cell or a mammalian cell; wherein the insect cell is an Sf9 cell or a *Trichoplusia ni* cell; that lacks at least one isoform of an endogenous adenine nucleotide translocator; in which expression of at least one gene encoding an endogenous adenine nucleotide translocator isoform is substantially impaired;

A recombinant expression construct comprising at least one promoter operably linked to a nucleic acid molecule comprising a first nucleic acid sequence and a second nucleic acid sequence, said first nucleic acid sequence encoding an animal adenine nucleotide translocator polypeptide wherein the adenine nucleotide translocator polypeptide is expressed as a fusion protein with a polypeptide product of said second nucleic acid sequence; wherein the polypeptide product of said second nucleic acid sequence is an enzyme; wherein said fusion protein localizes to membranes; wherein said membranes are mitochondrial membranes; further comprising at least one additional nucleic acid sequence that regulates transcription; wherein the additional nucleic acid sequence that regulates transcription encodes a repressor of said promoter;

wherein the adenine nucleotide translocator polypeptide comprises a human adenine nucleotide translocator polypeptide; wherein the human adenine nucleotide translocator polypeptide is ANT1; wherein the human adenine nucleotide translocator polypeptide is ANT2; wherein the adenine nucleotide translocator polypeptide is expressed as a fusion protein with at least one product of a second nucleic acid sequence encoding a polypeptide cleavable by a protease, said adenine nucleotide translocator polypeptide being separable from the fusion protein by cleavage with the protease; a host cell comprising a recombinant expression construct as just described; wherein the host cell is a prokaryotic cell; wherein the host cell is a eukaryotic cell; wherein the eukaryotic cell is selected from the group consisting of a yeast cell, an insect cell and a mammalian cell; wherein the insect cell is an Sf9 cell or a *Trichoplusia ni cell;* that lacks at least one isoform of an endogenous adenine nucleotide translocator; in which expression of at least one gene encoding an endogenous adenine nucleotide translocator isoform is substantially impaired; wherein the expression construct is a recombinant viral expression construct;

A method of producing a recombinant adenine nucleotide translocator polypeptide, comprising; culturing a host cell comprising a recombinant expression construct comprising at least one regulated promoter operably linked to a first nucleic acid encoding an adenine nucleotide translocator polypeptide;

A method of producing a recombinant adenine nucleotide translocator polypeptide, comprising culturing a host cell comprising a recombinant expression construct comprising at least one promoter operably linked to a nucleic acid molecule comprising a first nucleic acid sequence and a second nucleic acid sequence, said first nucleic acid sequence encoding an animal adenine nucleotide translocator polypeptide wherein the adenine nucleotide translocator polypeptide is expressed as a fusion protein with a polypeptide product of said second nucleic acid sequence;

A method of producing a recombinant adenine nucleotide translocator polypeptide, comprising culturing a host cell infected with the recombinant viral expression construct as provided above;

An ANT polypeptide produced by the methods just described;

An isolated human adenine nucleotide translocator polypeptide; wherein the human adenine nucleotide translocator polypeptide is recombinant ANT1 or a variant or fragment thereof; wherein the human adenine nucleotide translocator polypeptide is recombinant ANT2 or a variant or fragment thereof;

An isolated human adenine nucleotide translocator fusion protein comprising an adenine translocator polypeptide fused to at least one additional polypeptide sequence; wherein said one additional polypeptide sequence is an enzyme sequence or a variant or fragment thereof; wherein said fusion protein localizes to membranes; wherein said membranes are mitochondrial membranes;

An isolated human adenine nucleotide translocator fusion protein comprising an adenine translocator polypeptide fused to at least one additional polypeptide sequence cleavable by a protease, said adenine nucleotide translocator polypeptide being separable from the fusion protein by cleavage with the protease;

An isolated adenine nucleotide translocator fusion protein comprising a first polypeptide that is an animal adenine translocator polypeptide fused to at least one additional polypeptide sequence; wherein said one additional polypeptide sequence is an enzyme sequence or a variant or fragment thereof; that localizes to membranes; wherein said membranes are mitochondrial membranes;

An isolated recombinant animal adenine nucleotide translocator fusion protein comprising an adenine translocator polypeptide fused to at least one additional polypeptide sequence cleavable by a protease, said adenine nucleotide translocator polypeptide being separable from the fusion protein by cleavage with the protease; wherein the additional polypeptide sequence is a polypeptide having affinity for a ligand;

A method for determining the presence of an ANT polypeptide in a biological sample comprising contacting a biological sample suspected of containing an ANT polypeptide with an ANT ligand under conditions and for a time sufficient to allow binding of the ANT ligand to an ANT polypeptide; and detecting the binding of the ANT ligand to an ANT polypeptide, and therefrom determining the presence of an ANT polypeptide in said biological sample; wherein the adenine nucleotide translocator polypeptide comprises a human adenine nucleotide translocator polypeptide; wherein the human adenine nucleotide translocator polypeptide is ANT1; wherein the human adenine nucleotide translocator polypeptide is ANT2; wherein the ANT ligand comprises atractyloside substituted at 6’ hydroxyl to form an atractyloside derivative; wherein the atractyloside is detectably substituted at the 6’ hydroxyl to form a detectable atractyloside derivative; wherein the detectable atractyloside derivative comprises a radioloabeled substituent; wherein the radiolabeled substituent is selected from the group consisting of $^{125}I$, $^{131}I$, $^{3}H$, $^{14}C$ and $^{35}S$; wherein the detectable atractyloside derivative comprises a fluorescent substituent; wherein the ANT ligand further comprises a $Eu^{3+}$ atom complexed to the atractyloside derivative; wherein the detectable atractyloside derivative comprises covalently bound biotin; wherein the atractyloside molecule is substituted at 6’ hydroxyl with an amine or an amine containing functionality to form an amine modified atractyloside derivative; wherein the atractyloside molecule is a carboxyatractyloside molecule that is substituted at 6’ hydroxyl to form an atractyloside derivative that is a carboxyatractyloside derivative;

A method for isolating ANT from a biological sample, comprising contacting a biological sample suspected of containing an ANT polypeptide with an ANT ligand under conditions and for a time sufficient to allow binding of the ANT ligand to an ANT polypeptide; and recovering the ANT polypeptide, and thereby isolating ANT from a biological sample; wherein the ANT ligand is covalently bound to a solid phase; wherein the ANT ligand is non-covalently bound to a solid phase;

A method for identifying an agent that binds to an ANT polypeptide, comprising contacting a candidate agent with a host cell expressing at least one recombinant ANT polypeptide under conditions and for a time sufficient to permit binding of the agent to said recombinant ANT polypeptide; and detecting binding of said agent to the recombinant ANT; wherein the host cell is a prokaryotic cell; wherein the prokaryotic cell is an *E. coli* cell; wherein the host cell is a eukaryotic cell; wherein the eukaryotic cell is selected from the group consisting of a yeast cell, an insect cell and a mammalian cell; wherein the insect cell is an Sf9 cell or a *Trichoplusia ni* cell; wherein the host cell lacks at least one isoform of an endogenous adenine nucleotide translocator; wherein host cell expression of at least one gene encoding an endogenous adenine nucleotide translocator isoform is substantially impaired;

A method for identifying an agent that binds to an ANT polypeptide, comprising contacting a candidate agent with a biological sample containing at least one recombinant ANT polypeptide under conditions and for a time sufficient to permit binding of the agent to said ANT polypeptide; and detecting binding of said agent to the recombinant ANT polypeptide;

A method for identifying an agent that interacts with an ANT polypeptide comprising contacting a biological sample containing recombinant ANT with a detectable ANT ligand in the presence of a candidate agent; and comparing binding of the detectable ANT ligand to recombinant ANT in the absence of said agent to binding of the detectable ANT ligand to recombinant ANT in the presence of said agent, and therefrom identifying an agent that interacts with an ANT polypeptide;

An ANT ligand comprising atractyloside substituted at the 6’ hydroxyl to form an atractyloside derivative; wherein the atractyloside is detectably substituted at the 6’ hydroxyl to form a detectable atractyloside derivative; wherein the detectable atractyloside derivative comprises a radioloabeled substituent; wherein the radiolabeled substituent is selected from the group consisting of $^{125}I$, $^{131}I$, $^{3}H$, $^{14}C$ and $^{35}S$; wherein the detectable atractyloside derivative comprises a fluorescent substituent; further comprising a $Eu^{3+}$ atom complexed to the atractyloside derivative; wherein the detectable atractyloside derivative comprises covalently bound biotin; wherein the atractyloside molecule is substituted at 6' hydroxyl with an amine or an amine containing functionality to form an amine modified atractyloside derivative; wherein the atractyloside molecule is a carboxyatractyloside molecule that is substituted at 6' hydroxyl to form an atractyloside derivative that is a carboxyatractyloside derivative;

An ANT ligand having the following structure(I):

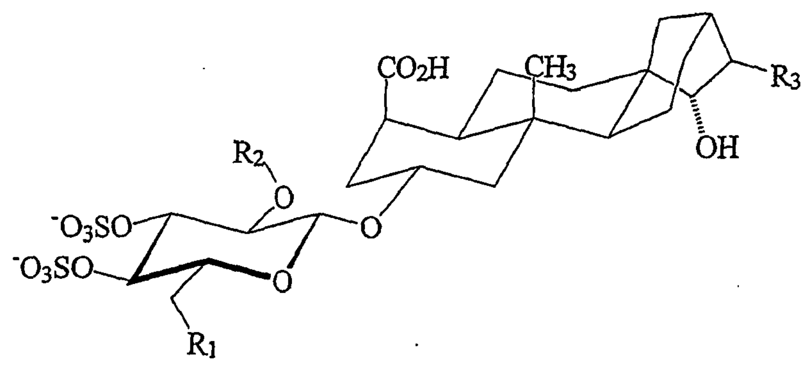

(I)

including stereoisomers and pharmaceutically acceptable salts thereof, wherein $R_1$, $R_2$ and $R_3$ are as identified below;

An assay plate for high throughput screening of candidate agents that bind to at least one ANT polypeptide, comprising an assay plate having a plurality of wells, each of said wells further comprising at least one immobilized recombinant ANT polypeptide or a variant or fragment thereof;

A method of targeting a polypeptide of interest to a mitochondrial membrane, comprising expressing a recombinant expression construct encoding a fusion protein in a host cell, said construct comprising at least one regulated promoter operably linked to a nucleic acid molecule comprising a first nucleic acid sequence and a second nucleic acid sequence, said first nucleic acid sequence encoding an adenine nucleotide translocator polypeptide that is expressed as a fusion protein with a polypeptide product of said second nucleic acid sequence, wherein said second nucleic acid sequence encodes the polypeptide of interest;

A method of targeting a polypeptide of interest to a mitochondrial membrane, comprising expressing a recombinant expression construct encoding a fusion protein in a host cell, said construct comprising at least one promoter operably linked to a nucleic acid molecule comprising a first nucleic acid sequence and a second nucleic acid sequence, said first nucleic acid sequence encoding an animal adenine nucleotide translocator polypeptide that is expressed as a fusion protein with a polypeptide product of said second nucleic acid sequence, wherein said second nucleic acid sequence encodes the polypeptide of interest; a pharmaceutical composition comprising an ANT ligand as just described;

A pharmaceutical composition comprising an agent that binds to an ANT polypeptide identified as just described. A pharmaceutical composition comprising an agent that binds to an ANT polypeptide identified as described above. A pharmaceutical composition comprising an agent that interacts with an ANT polypeptide identified above. A method of treatment comprising administering to a subject any one of the just described the pharmaceutical compositions.

These and other aspects of the present invention will become evident upon reference to the following detailed description and attached drawings. In addition, various references are set forth herein which describe in more detail certain aspects of this invention, and are therefore incorporated by reference in their entireties

BRIEF DESCRIPTION OF THE DRAWINGS

Figure 1 shows the nucleotide sequences of the coding regions of human ANT1 ("ANT1m"), human ANT2 ("ANT2m") and human ANT3 ("ANT3m").

Figure 2 shows the polypeptide sequences of human ANT1 ("ANT1p"), human ANT2 ("ANT2p") and human ANT3 ("ANT3p").

Figure 3 shows that His-tagged ANT fusion proteins corresponding to huANT1, huANT2 and huANT3 are all detected using an ANTI-XPRESS™ Antibody (Invitrogen) (left panel). In contrast, an ANT1-specific antibody detects only the

ANT1 isoform (central panel), and an ANT2-specific antiibody detects only the ANT2 isoform (right panel).

Figure 4 shows binding of $^{125}$I-compound 24 to bovine mitochondria. Symbols: (♦), bovine mitochondria; (■), control (no mitochondria).

Figure 5 shows competition of $^{125}$I-compound 24 binding to bovine mitochondria by unlabeled compound 24(▼), ATR (■) and ADP (▲).

Figure 6 shows competition of $^{125}$I-compound 24 binding to beef heart mitochondria by (■) BKA and (▼) unlabeled compound 24.

Figure 7 shows competition of $^{125}$I-compound 24 binding to beef heart mitochondria by compound 23 (▼), compound 28 (♦) and ATR (■).

Figure 8 shows competition of $^{125}$I-compound 24 binding to beef heart mitochondria by compound 5 (♦) and ATR (■).

Figure 9 shows western blots of isolated mitochondria from bovine cardiac tissue and SH-SY5Y cells probed with Pan ANT antibody (left panel) and VDAC antibody (right panel).

Figure 10 shows binding curves for carboxyactractyloside (C-ATR) for isolated mitochondria from SH-SY5Y cells (■) and from bovine cardiac tissue (▼).

Figure 11 shows the actractyloside binding capacity of bovine cardiac tissue and SH-SY5Y cells.

Figure 12 shows western immunoblot detection, using pan-isoform specific rabbit anti-ANT antibodies, of an expressed recombinant His-tagged human ANT-1 fusion protein in Tn/ANT1 cells and in a mitochondrial fraction isolated from these cells; also shown is detection of human ANT (hANT) in SH-SY5Y cells.

Figure 13 shows displacement, by various concentrations of BKA, of $^{125}$I-labeled compound 24 binding to isolated Tn/ANT1 mitochondria.

## DETAILED DESCRIPTION OF THE INVENTION

The present invention is directed generally toward adenine nucleotide translocator (ANT) polypeptides, which as provided herein may refer to any ANT isoform; to expression constructs containing nucleic acids encoding ANT and to natural and synthetic small molecules that interact with ANT, including ANT binding ligands. The present invention relates in part to the unexpected findings that bacterial, insect, yeast or mammalian expression systems can be designed for.reliable production of recombinant human ANT polypeptides in significant quantities. In certain aspects the invention provides compositions and methods for producing recombinant ANT polypeptides that employ regulated promoters, and in certain of these and other aspects the invention provides compositions and methods for producing recombinant ANT polypeptides that are ANT fusion proteins. In certain preferred embodiments, the design of such expression systems includes the use of a host cell that lacks endogenous ANT or in which endogenous ANT gene expression is substantially impaired, as provided herein.

The present invention thus also pertains in part to methods for producing and isolating recombinant ANT polypeptides, including human ANT polypeptides and in preferred embodiments human ANT1 or ANT2 polypeptides, that may then be used in various binding assays and screening assays and the like. In view of the surprising observation that expression of recombinant human ANT polypeptides can be achieved at levels enabling such uses of these ANT polypeptide products, the present invention provides assays (including high throughput assays) for identifying agents that bind to recombinant human ANT. Accordingly, the present invention further relates in part to novel human ANT ligands, the synthesis, selection and characterization of which would heretofore have not been possible given the need for expressed recombinant ANT polypeptides to use in binding assays. The invention also pertains to agents that interact with ANT, including agents that enhance or impair any ANT functions known to the art, including but not limited to those described herein, and to incorporation of such agents into pharmaceutical compositions and their use in therapeutic methods.

As discussed above, the present invention relates in part to the unexpected finding that recombinant adenine nucleotide translocator (ANT) polypeptides, which includes full length ANT proteins and polypeptides, fragments and variants thereof, and further includes ANT fusion proteins as provided herein, can be produced in useful amounts by using a recombinant expression vector having a regulatory nucleic acid operably linked to a nucleic acid encoding ANT. In particular, the invention provides compositions and methods for producing recombinant ANT polypeptides through the use of a regulated promoter; the invention also provides recombinant ANT polypeptides that are ANT fusion proteins.

The invention also pertains to compositions and methods to identify the presence of ANT polypeptides and to isolate recombinant ANT, and in addition to screening assays for compounds that interact with ANT. Accordingly, the invention provides certain advantages with regard to regulation of mitochondrial function, and in particular regulation of the mitochondrial permeability "pore".

By way of background, four of the five multisubunit protein complexes (Complexes I, III, IV and V) that mediate ETC activity are localized to the inner mitochondrial membrane, which is the most protein rich of biological membranes in cells (75% by weight); the remaining ETC complex (Complex II) is situated in the matrix. ANT represents the most abundant of the inner mitochondrial membrane proteins. In at least three distinct chemical reactions known to take place

within the ETC, positively-charged protons are moved from the mitochondrial matrix, across the inner membrane, to the intermembrane space. This disequilibrium of charged species creates an electrochemical potential of approximately 220 mV referred to as the "protonmotive force" (PMF), which is often represented by the notation $\Delta\psi$ or $\Delta\psi m$ and represents the sum of the electric potential and the pH differential across the inner mitochondrial membrane (*see, e.g.,* Ernster et al., 1981 J. Cell Biol. 91:227s and references cited therein).

This membrane potential drives ANT-mediated stoichiometric exchange of adenosine triphosphate (ATP) and adenosine diphosphate (ADP) across the inner mitochondrial membrane, and provides the energy contributed to the phosphate bond created when ADP is phosphorylated to yield ATP by ETC Complex V, a process that is "coupled" stoichiometrically with transport of a proton into the matrix. Mitochondrial membrane potential, $\Delta\psi m$, is also the driving force for the influx of cytosolic $Ca^{2+}$ into the mitochondrion. Under normal metabolic conditions, the inner membrane is impermeable to proton movement from the intermembrane space into the matrix, leaving ETC Complex V as the sole means whereby protons can return to the matrix. When, however, the integrity of the inner mitochondrial membrane is compromised, as occurs during MPT that may accompany a disease associated with altered mitochondrial function, protons are able to bypass the conduit of Complex V without generating ATP, thereby "uncoupling" respiration because electron transfer and associated proton pumping yields no ATP. Thus, mitochondrial permeability transition involves the opening of a mitochondrial membrane "pore", a process by which, *inter alia,* the ETC and, ATP synthesis are uncoupled, $\Delta\psi m$ collapses and mitochondrial membranes lose the ability to selectively regulate permeability to solutes both small (*e.g.,* ionic $Ca^{2+}$, $Na^+$, $K^+$, $H^+$) and large (*e.g.*, proteins).

Without wishing to be bound by theory, it is unresolved whether this pore is a physically discrete conduit that is formed in mitochondrial membranes, for example by assembly or aggregation of particular mitochondrial and/or cytosolic proteins and possibly other molecular species, or whether the opening of the "pore" may simply represent a general increase in the porosity of the mitochondrial membrane.

MPT may also be induced by compounds that bind one or more mitochondrial molecular components. Such compounds include, but are not limited to, atractyloside and bongkrekic acid, which are known to bind to ANT. Methods of determining appropriate amounts of such compounds to induce MPT are known in the art (*see, e.g.,* Beutner et al., 1998 Biochim. Biophys. Acta 1368:7*;* Obatomi and Bach, 1996 Toxicol. Lett. 89:155*;* Green and Reed, 1998 Science 281:1309; Kroemer et al., 1998 Annu. Rev. Physiol. 60:619; and references cited therein). Thus certain mitochondrial molecular components, such as ANT, may contribute to the MPT mechanism. As noted above, it is believed that adenine nucleotide translocator (ANT) mediates ATP/proton exchange across the inner mitochondrial membrane, and that ANT inhibitors such as atractyloside or bongkrekic acid induce MPT under certain conditions. Hence, it is desirable to obtain specific ANT isoforms in sufficient quantities for structural and functional assays that provide, for example, ANT ligands and other agents that interact with ANT, which will be useful for therapeutic management of mitochondrial pore activity. *See also* U.S. 09/161,172, entitled "Compositions and Methods for Identifying Agents that Alter Mitochondrial Permeability Transition Pores", which is hereby incorporated by reference.

ANTISOFORMS

ANT proteins are present in many organisms from a variety of species. Full length amino acid sequences of at least 29 ANT proteins have been reported to date from a variety of animal and plant species, with most of these deduced from nucleic acid sequences (Fiore et al., 1998 Biochimie 80:137-150).

In a given species, two or more isoforms may be present. For example, two ANT isoforms (muANT1 and muANT2) have been identified in mice *(Mus musculus),* and other mammalian ANT homologues have been described, as have three human ANT isoforms (huANT1, huANT2 and huANT3). See, *e.g.*, Wallace et al., 1998 in Mitochondria & Free Radicals in Neurodegenerative Diseases, Beal, Howell and Bodis-Wollner, Eds., Wiley-Liss, New York, pp. 283-307, and references cited therein. ANT sequences among mammalian species are highly conserved; for example, at the amino acid level, murine ANT1 and ANT2 exhibit 98% sequence identity with human ANT2.

Moreover, within an organism of a particular species, a variety of ANT isoforms may be present in different amounts in different tissues of a particular organism (Doerner et al., 1999 Biochim. Biophys. Acta. 141:16-24; Doerner et al., 1997 FEBS Lett. 414:258). Usually one isoform is predominant, and one or more of the other isoforms is expressed to a lesser degree. The differential expression of ANT isoforms in varying tissues may be a causative factor in some of the physiological differences among the tissues (e.g., Drummler et al., 1996 Biochem. J. 317:913-918).

If each ANT isoform is biochemically and/or is functionally different form other ANT isoforms present within the same organism, the ability of a particular isoform to bind ANT ligands such as actractyloside (ATR) and/or bongkrekic acids (BKA.). Indeed, reduced sensitivity to ATR and BKA is reduced in isolated hepatoma cells as compared to control cells, suggesting that the relative amounts of different ANT isoforms differ in these cells, and/or that the absolute amount of ANT proteins varies in different cell types (Woldegiorgis et al., 1985 J. Biol. Chem. 260:7585). The present invention provides compositions and methods for determining the relative or absolute amounts of specific ANT isoform proteins in different tissues or organisms. The present invention also provides compositions and methods for determining the

biochemical, bioenergetic and physiological differences among ANT isoforms.

Human ANT2 is clearly expressed differently from the other two isoforms and is upregulated in tissues with high glycolytic rates. It is also the predominant isoform in highly glycolytic cultured cell lines such as rho$^0$ and tumoral cells (Giraud et al., 1998 J. Mol. Biol. 281:409). Experiments by the same group have shown that when cells not normally expressing huANT2 are put under anaerobic conditions, the huANT2 gene is transcribed. If these cells are then supplied with glutamine to promote oxidative metabolism, huANT2 expression is once again suppressed. One interesting finding regarding ANT2 expression is that it is not just cell lines or benign proliferative cell types that have high levels of ANT2 message. Increased expression of ANT2 has also been measured in oncocytoma and malignant urothelial renal tumors and compared to control tissue (Faure-Vigny et al., 1996 Molec. Carcinogen. 16:165-172). Without wishing to be bound by any particular theory, it is possible that ANT2 may play a direct role in tumorigenesis and/or may be a compensatory mechanism for the production of cytosolic ATP brought on by increased glycolysis in tumor cells.

Because the ANT isoforms have peptide sequences that are fairly homolgous to each other, attempts to use biochemical protein purification techniques to purify individual isoforms of ANT have not been successful. The present invention overcomes this limitation by providing compositions and methods for the separate production of each individual ANT isoform. Using the human ANT isoforms as an example, non-mammalian host cells comprising an expression construct for a particular huANT isoform are used to overexpress the gene encoding that huANT isoform, resulting in the production of a particular huANT isoform in a host cell that lacks any endogenous huANT.

A particular ANT isoform that has been produced in this manner can be isolated or partially isolated from the proteins (and other biomolecules) of the host cell, thereby producing a composition of matter that (i) comprises a specific isoform of an ANT protein from an organism and (ii) does not comprise any of the other isoforms of ANT from that organism. For brevity's sake, such compositions are referred to herein as being "ANT-x specific," where "x" is a term that distinguishes the ANT of the composition from other ANT isoforms. For example, an ANT-1 specific composition of matter comprises ANT-1 but lacks ANT-2 and ANT-3.

Unlike previous preparations of ANT proteins, ANT-x specific compositions can be examined and analyzed for biochemical and other properties of a particular ANT isoform (*i.e.*, ANT-x). Non-limiting examples include, for example, determining the biochemical kinetics of one or more ANT isoforms; examining the ability of a specific ANT isoform to form ANT multimers (e.g., dimers, trimers, tetramers, and the like) with itself or with other, separately introduced, ANT isoforms; and determining the ability of a given ANT isoform to alter the ATP/ADP concentration in the mitochondrial matrix and/or the cytoplasm or organelles. Altering the ATP/ADP concentration in the matrix can lead to secondary alterations in various mitochondrial activities, including but not limited to oxidative phosphorylation, rates of TCA cycle flux and/or flux of metabolic intermediates out of or into mitochondria. The present invention provides compositions and methods for determining the biochemical characteristics of specific ANT isoforms and the ability of each isoform to influence mitochondrial functions.

The compositions and methods of the present invention can be adapted to any prokaryotic or eukaryotic ANT, including plant and animal ANTs, which may further include, for example, yeast, vertebrate, mammalian, rodent, primate and human ANTs, for which amino acid sequences and/or encoding nucleic acids will be known to those familiar with the art. Three human ANT isoforms have been described that differ in their tissue expression patterns. (Stepien et al., 1992 J. Biol. Chem. 267:14592; Graham et al., 1997 Nat. Genet. 16:226; *see also* Wallace et al., 1998 in Mitochondria & Free Radicals in Neurodegenerative Diseases, Beal, Howell and Bodis-Wollner, Eds., Wiley-Liss, New York, pp. 283-307, and references cited therein.) Nucleic acid sequences for cDNAs encoding these three human ANT isoforms have been reported (Figure 1; *See* Neckelmann et al., Proc. Nat'l. Acad. Sci. U.S.A. 84:7580-7.584 (1987) for huANT1 cDNA [SEQ ID NO:1]; Battini et al., *J. Biol. Chem. 262*:4355-4359 (1987) for huANT2 cDNA [SEQ ID NO:2], and Cozens et al., J. Mol. Biol. 206:261-280 (1989) for huANT3 cDNA [SEQ ID NO:3]; see Figure 2 for amino acid sequences of huANT1 [SEQ ID NO:31] huANT2 [SEQ ID NO:32] and huANT3 [SEQ ID NO:33].), and ANT gene sequences have been determined for a number of species (See, *e.g.*, Li et al., 1989 J. Biol. Chem. 264:13998 for huANT1 genomic DNA, see also, *e.g.,* Li et al. 1990 J. Biol. Chem. 265:20585; Liew et al. GenBank Acc. #N86710 for huANT2; Shinohara et al., 1993 Biochim. Biophys. Acta 1152:192 for rat ANT gene; for others see also, *e.g.,* Ku et al., 1990 J. Biol. Chem. 265:16060; Adams et al., 1991 Science 252:1651; and WO 98/19714.). The present invention further relates to nucleic acids which hybridize to ANT encoding polynucleotide sequences as provided herein, as incorporated by reference or as will be readily apparent to those familiar with the art, if there is at least 70%, preferably at least 90%, and more preferably at least 95% identity between the sequences. The present invention particularly relates to nucleic acids which hybridize under stringent conditions to the ANT encoding nucleic acids referred to herein. As used herein, the term "stringent conditions" means hybridization will occur only if there is at least 95% and preferably at least 97% identity between the sequences. The nucleic acids which hybridize to ANT encoding nucleic acids referred to herein, in preferred embodiments, encode polypeptides which either retain substantially the same biological function or activity as the ANT polypeptides encoded by the cDNAs of Figure 1 [SEQ ID NOS:1, 2 and 3], or the deposited expression constructs.

As used herein, to "hybridize" under conditions of a specified stringency is used to describe the stability of hybrids formed between two single-stranded nucleic acid molecules. Stringency of hybridization is typically expressed

in conditions of ionic strength and temperature at which such hybrids are annealed and washed. Typically "high", "medium" and "low" stringency encompass the following conditions or equivalent conditions thereto: high stringency: 0.1 x SSPE or SSC, 0.1% SDS, 65˚C; medium stringency: 0.2 x SSPE or SSC, 0.1% SDS, 50˚C; and low stringency: 1.0 x SSPE or SSC, 0.1% SDS, 50˚C.

The deposits referred to herein will be maintained under the terms of the Budapest Treaty on the International Recognition of the Deposit of Micro-organisms for purposes of Patent Procedure. These deposits are provided merely as convenience to those of skill in the art and are not an admission that a deposit is required under 35 U.S.C. § 112. The sequences of the nucleic acids contained in the deposited materials, as well as the amino acid sequences of the polypeptides encoded thereby, are incorporated herein by reference and are controlling in the event of any conflict with any description of sequences herein. A licensee may be required to make, use or sell the deposited materials, and no such license is hereby granted.

NUCLEIC ACIDS

The nucleic acids of the present invention may be in the form of RNA or in the form of DNA, which DNA includes cDNA, genomic DNA, and synthetic DNA. The DNA may be double-stranded or single-stranded, and if single stranded may be the coding strand or non-coding (anti-sense) strand. A coding sequence which encodes an ANT polypeptide for use according to the invention may be identical to the coding sequence known in the art for any given ANT, as described above and, for example, as shown for human ANT1 [SEQ ID NO:1], human ANT2 [SEQ ID NO:2] and human ANT3 [SEQ ID NO:3] in Figure 1, or to that of any deposited clone, or may be a different coding sequence, which, as a result of the redundancy or degeneracy of the genetic code, encodes the same ANT polypeptide as, for example, the cDNAs of Figure 1 or the deposited expression constructs.

The nucleic acids which encode ANT polypeptides, for example the human ANT polypeptides having the amino acid sequences of Figure 2 [SEQ ID NOS:31-33] or any other ANT polypeptides for use according to the invention, or for the ANT polypeptides encoded by the deposited constructs may include, but are not limited to: only the coding sequence for the ANT polypeptide; the coding sequence for the ANT polypeptide and additional coding sequence; the coding sequence for the ANT polypeptide (and optionally additional coding sequence) and non-coding sequence, such as introns or non-coding sequences 5' and/or 3' of the coding sequence for the ANT polypeptide, which for example may further include but need not be limited to one or more regulatory nucleic acid sequences that may be a regulated or regulatable promoter, enhancer, other transcription regulatory sequence, repressor binding sequence, translation regulatory sequence or any other regulatory nucleic acid sequence. Thus, the term "nucleic acid encoding an ANT polypeptide" encompasses a nucleic acid which includes only coding sequence for the polypeptide as well as a nucleic acid which includes additional coding and/or non-coding sequence(s).

The present invention further relates to variants of the herein described nucleic acids which encode for fragments, analogs and derivatives of an ANT polypeptide, for example the human ANT1, ANT2 and ANT3 polypeptides having the deduced amino acid sequences of Figure 2 [SEQ ID NOS:31-33] or any ANT polypeptide, including ANT polypeptides encoded by the cDNAs of the deposited expression constructs. The variants of the nucleic acids encoding ANTs may be naturally occurring allelic variants of the nucleic acids or non-naturally occurring variants. As is known in the art, an allelic variant is an alternate form of a nucleic acid sequence which may have at least one of a substitution, a deletion or an addition of one or more nucleotides, any of which does not substantially alter the function of the encoded ANT polypeptide. Thus, for example, the present invention includes nucleic acids encoding the same ANT polypeptides as shown in Figure 2 [SEQ ID NOS:31-33], or the same ANT polypeptides encoded by the cDNAs of the deposited expression constructs, as well as variants of such nucleic acids, which variants encode a fragment, derivative or analog of any of the polypeptides of Figure 2 (SEQ ID NO:2) or the polypeptides encoded by the cDNAs of the deposited expression constructs.

Variants and derivatives of ANT may be obtained by mutations of nucleotide sequences encoding ANT polypeptides. Alterations of the native amino acid sequence may be accomplished by any of a number of conventional methods. Mutations can be introduced at particular loci by synthesizing oligonucleotides containing a mutant sequence, flanked by restriction sites enabling ligation to fragments of the native sequence. Following ligation, the resulting reconstructed sequence encodes an analog having the desired amino acid insertion, substitution, or deletion.

Alternatively, oligonucleotide-directed site-specific mutagenesis procedures can be employed to provide an altered gene wherein predetermined codons can be altered by substitution, deletion or insertion. Exemplary methods of making such alterations are disclosed by Walder et al. (Gene 42:133, 1986); Bauer et al. (Gene 37:73, 1985); Craik (BioTechniques, January 1985, 12-19); Smith et al. (Genetic Engineering: Principles and Methods, Plenum Press, 1981); Kunkel (Proc. Natl. Acad. Sci. USA 82:488, 1985); Kunkel et al. (Methods in Enzymol. 154:367, 1987); and U.S. Patent Nos. 4,518,584 and 4,737,462.

Equivalent DNA constructs that encode various additions or substitutions of amino acid residues or sequences, or deletions of terminal or internal residues or sequences not needed for biological activity are also encompassed by

the invention. For example, sequences encoding Cys residues that are not essential for biological activity can be altered to cause the Cys residues to be deleted or replaced with other amino acids, preventing formation of incorrect intramolecular disulfide bridges upon renaturation. Other equivalents can be prepared by modification of adjacent dibasic amino acid residues to enhance expression in yeast systems in which KEX2 protease activity is present. EP 212,914 discloses the use of site-specific mutagenesis to inactivate KEX2 protease processing sites in a protein. KEX2 protease processing sites are inactivated by deleting, adding or substituting residues to alter Arg-Arg, Arg-Lys, and Lys-Arg pairs to eliminate the occurrence of these adjacent basic residues. Lys-Lys pairings are considerably less susceptible to KEX2 cleavage, and conversion of Arg-Lys or Lys-Arg to Lys-Lys represents a conservative and preferred approach to inactivating KEX2 sites.

POLYPEPTIDES AND FUSION PROTEINS

The present invention further relates to ANT polypeptides, and in particular to methods for producing recombinant ANT polypeptides by culturing host cells containing ANT expression constructs, and to isolated recombinant human ANT polypeptides, including, for example, the human ANT1, ANT2 and ANT3 polypeptides which have the deduced amino acid sequence of Figure 2 [SEQ ID NOS:31-33] or which have the amino acid sequence encoded by the deposited recombinant expression constructs, as well as fragments, analogs and derivatives of such polypeptides. The polypeptides and nucleic acids of the present invention are preferably provided in an isolated form, and in certain preferred embodiments are purified to homogeneity.

The terms "fragment," "derivative" and "analog" when referring to ANT polypeptides or fusion proteins, or to ANT polypeptides or fusion proteins encoded by the deposited recombinant expression constructs, refers to any ANT polypeptide or fusion protein that retains essentially the same biological function or activity as such polypeptide. Thus, an analog includes a proprotein which can be activated by cleavage of the proprotein portion to produce an active ANT polypeptide. The polypeptide of the present invention may be a recombinant polypeptide or a synthetic polypeptide, and is preferably a recombinant polypeptide.

A fragment, derivative or analog of an ANT polypeptide or fusion protein, including ANT polypeptides or fusion proteins encoded by the cDNAs of the deposited constructs, may be (i) one in which one or more of the amino acid residues are substituted with a conserved or non-conserved amino acid residue (preferably a conserved amino acid residue) and such substituted amino acid residue may or may not be one encoded by the genetic code, or (ii) one in which one or more of the amino acid residues includes a substituent group, or (iii) one in which the ANT polypeptide is fused with another compound, such as a compound to increase the half-life of the polypeptide (for example, polyethylene glycol), or (iv) one in which additional amino acids are fused to the ANT polypeptide, including amino acids that are employed for purification of the ANT polypeptide or a proprotein sequence. Such fragments, derivatives and analogs are deemed to be within the scope of those skilled in the art from the teachings herein.

The polypeptides of the present invention include ANT polypeptides and fusion proteins having amino acid sequences that are identical or similar to sequences known in the art. For example by way of illustration and not limitation, the human ANT ("huANT") polypeptides of Figure 2 [SEQ ID NOS:31-33] are contemplated for use according to the instant invention, as are polypeptides having at least 70% similarity (preferably a 70% identity) to the polypeptides of Figure 2 [SEQ ID NOS:31-33] and more preferably 90% similarity (more preferably a 90% identity) to the polypeptides of Figure 2 [SEQ ID NOS: 31-33] and still more preferably a 95% similarity (still more preferably a 95% identity) to the polypeptides of Figure 2 [SEQ ID NOS:31-33] and to portions of such polypeptides, wherein such portions of an ANT polypeptide generally contain at least 30 amino acids and more preferably at least 50 amino acids.

As known in the art "similarity" between two polypeptides is determined by comparing the amino acid sequence and conserved amino acid substitutes thereto of the polypeptide to the sequence of a second polypeptide. Fragments or portions of the polypeptides of the present invention may be employed for producing the corresponding full-length polypeptide by peptide synthesis; therefore, the fragments may be employed as intermediates for producing the full-length polypeptides. Fragments or portions of the nucleic acids of the present invention may be used to synthesize full-length nucleic acids of the present invention.

The term "isolated" means that the material is removed from its original environment (e.g., the natural environment if it is naturally occurring). For example, a naturally occurring nucleic acid or polypeptide present in a living animal is not isolated, but the same nucleic acid or polypeptide, separated from some or all of the co-existing materials in the natural system, is isolated. Such nucleic acids could be part of a vector and/or such nucleic acids or polypeptides could be part of a composition, and still be isolated in that such vector or composition is not part of its natural environment.

The term "gene" means the segment of DNA involved in producing a polypeptide chain; it includes regions preceding and following the coding region "leader and trailer" as well as intervening sequences (introns) between individual coding segments (exons).

As described herein, the invention provides ANT fusion proteins encoded by nucleic acids that have the ANT coding sequence fused in frame to an additional coding sequence to provide for expression of an ANT polypeptide

sequence fused to an additional functional or non-functional polypeptide sequence that permits, for example by way of illustration and not limitation, detection, isolation and/or purification of the ANT fusion protein. Such ANT fusion proteins may permit detection, isolation and/or purification of the ANT fusion protein by protein-protein affinity, metal affinity or charge affinity-based polypeptide purification, or by specific protease cleavage of a fusion protein containing a fusion sequence that is cleavable by a protease such that the ANT polypeptide is separable from the fusion protein.

Thus ANT fusion proteins may comprise polypeptide sequences added to ANT to facilitate detection and isolation of ANT. Such peptides include, for example, poly-His or the antigenic identification peptides described in U.S. Patent No. 5,011,912 and in Hopp et al., (1988 Bio/Technology 6:1204), or the XPRESS™ epitope tag (Invitrogen, Carlsbad, CA). The affinity sequence may be a hexa-histidine tag as supplied, for example, by a pBAD/His (Invitrogen) or a pQE-9 vector to provide for purification of the mature polypeptide fused to the marker in the case of a bacterial host, or, for example, the affinity sequence may be a hemagglutinin (HA) tag when a mammalian host, *e.g.,* COS-7 cells, is used. The HA tag corresponds to an antibody defined epitope derived from the influenza hemagglutinin protein (Wilson et al., Cell 37:767 (1984)).

ANT fusion proteins may further comprise immunoglobulin constant region polypeptides added to ANT to facilitate detection, isolation and/or localization of ANT. The immunoglobulin constant region polypeptide preferably is fused to the C-terminus of an ANT polypeptide. General preparation of fusion proteins comprising heterologous polypeptides fused to various portions of antibody-derived polypeptides (including the Fc domain) has been described, *e.g.*, by Ashkenazi et al. (PNAS USA 88:10535, 1991) and Byrn et al. (Nature 344:677, 1990). A gene fusion encoding the ANT: Fc fusion protein is inserted into an appropriate expression vector. In certain embodiments of the invention, ANT:Fc fusion proteins may be allowed to assemble much like antibody molecules, whereupon interchain disulfide bonds form between Fc polypeptides, yielding dimeric ANT fusion proteins.

ANT fusion proteins having specific binding affinities for pre-selected antigens by virtue of fusion polypeptides comprising immunoglobulin V-region domains encoded by DNA sequences linked in-frame to sequences encoding ANT are also within the scope of the invention, including variants and fragments thereof as provided herein. General strategies for the construction of fusion proteins having immunoglobulin V-region fusion polypeptides are disclosed, for example, in EP 0318554; U.S. 5,132,405; U.S. 5,091,513; and U.S. 5,476,786.

The nucleic acid of the present invention may also encode a fusion protein comprising an ANT polypeptide fused to other polypeptides having desirable affinity properties, for example an enzyme such as glutathione-S-transferase. As another example, ANT fusion proteins may also comprise an ANT polypeptide fused to *a Staphylococcus aureus* protein A polypeptide; protein A encoding nucleic acids and their use in constructing fusion proteins having affinity for immunoglobulin constant regions are disclosed generally, for example, in U.S. Patent 5,100,788. Other useful affinity polypetides for construction of ANT fusion proteins may include streptavidin fusion proteins, as disclosed, for example, in WO 89/03422; U.S. 5,489,528; U.S. 5,672,691; WO 93/24631; U.S. 5,168,049; U.S. 5,272,254 and elsewhere, and avidin fusion proteins (see, *e.g.*, EP 511,747). As provided herein and in the cited references, ANT polypeptide seqences may be fused to fusion polypeptide sequences that may be full length fusion polypeptides and that may alternatively be variants or fragments thereof.

The present invention also provides a method of targeting a polypeptide of interest to a membrane, and in particular embodiments to a cellular membrane, and in further embodiments to a mitochondrial membrane. This aspect of the invention is based on the unexpected observation that certain recombinant expression constructs as provided herein, which constructs include a nucleic acid encoding a first polypeptide that is an ANT polypeptide, and that is expressed as a fusion protein with a second polypeptide sequence, provide recombinant ANT fusion proteins capable of preferentially localizing to cell membranes. In certain embodiments the cell membrane is a prokaryotic cell membrane such as a bacterial cell membrane, for example an E. *coli* membrane. In other embodiments the cell membrane is a eukaryotic cell membrane such as a yeast or a mammalian cell membrane, for example a membrane of any eukaryotic cell contemplated herein.

A cell membrane as used herein may be any cellular membrane, and typically refers to membranes that are in contact with cytosolic components, including intracellular membrane bounded compartments such as mitochondrial inner and outer membranes as described above, and also intracellular vesicles, ER-Golgi constituents, other organelles and the like, as well as the plasma membrane. In preferred embodiments, an ANT fusion protein may be targeted to a mitochondrial membrane. In other preferred embodiments, for example, recombinant expression constructs according to the invention may encode ANT fusion proteins that contain polypeptide sequences that direct the fusion protein to be retained in the cytosol, to reside in the lumen of the endoplasmic reticulum (ER), to be secreted from a cell via the classical ER-Golgi secretory pathway, to be incorporated into the plasma membrane, to associate with a specific cytoplasmic component including the cytoplasmic domain of a transmembrane cell surface receptor or to be directed to a particular subcellular location by any of a variety of known intracellular protein sorting mechanisms with which those skilled in the art will be familiar. Accordingly, these and related embodiments are encompassed by the instant compositions and methods directed to targeting a polypeptide of interest to a predefined intracellular, membrane or extracellular localization.

VECTORS

The present invention also relates to vectors and to constructs that include nucleic acids of the present invention, and in particular to "recombinant expression constructs" that include any nucleic acids encoding ANT polypeptides according to the invention as provided above; to host cells which are genetically engineered with vectors and/or constructs of the invention and to the production of ANT polypeptides and fusion proteins of the invention, or fragments or variants thereof, by recombinant techniques. ANT proteins can be expressed in mammalian cells, yeast, bacteria, or other cells under the control of appropriate promoters. Cell-free translation systems can also be employed to produce such proteins using RNAs derived from the DNA constructs of the present invention. Appropriate cloning and expression vectors for use with prokaryotic and eukaryotic hosts are described by Sambrook, et al., Molecular Cloning: A Laboratory Manual, Second Edition, Cold Spring Harbor, NY, (1989).

Generally, recombinant expression vectors will include origins of replication and selectable markers permitting transformation of the host cell, *e.g.*, the ampicillin resistance gene of *E. coli* and *S. cerevisiae* TRP1 gene, and a promoter derived from a highly-expressed gene to direct transcription of a downstream structural sequence. Such promoters can be derived from operons encoding glycolytic enzymes such as 3-phosphoglycerate kinase (PGK), α-factor, acid phosphatase, or heat shock proteins, among others. The heterologous structural sequence is assembled in appropriate phase with translation initiation and termination sequences. Optionally, the heterologous sequence can encode a fusion protein including an N-terminal identification peptide imparting desired characteristics, *e.g.*, stabilization or simplified purification of expressed recombinant product.

Useful expression constructs for bacterial use are constructed by inserting into an expression vector a structural DNA sequence encoding a desired protein together with suitable translation initiation and termination signals in operable reading phase with a functional promoter. The construct may comprise one or more phenotypic selectable marker and an origin of replication to ensure maintenance of the vector construct and, if desirable, to provide amplification within the host. Suitable prokaryotic hosts for transformation include *E. coli, Bacillus subtilis, Salmonella typhimurium* and various species within the genera Pseudomonas, Streptomyces, and Staphylococcus, although others may also be employed as a matter of choice. Any other plasmid or vector may be used as long as they are replicable and viable in the host.

As a representative but nonlimiting example, useful expression vectors for bacterial use can comprise a selectable marker and bacterial origin of replication derived from commercially available plasmids comprising genetic elements of the well known cloning vector pBR322 (ATCC 37017). Such commercial vectors include, for example, pKK223-3 (Pharmacia Fine Chemicals, Uppsala, Sweden) and GEM1 (Promega Biotec, Madison, WI, USA). These pBR322 "backbone" sections are combined with an appropriate promoter and the structural sequence to be expressed.

Following transformation of a suitable host strain and growth of the host strain to an appropriate cell density, the selected promoter, if it is a regulated promoter as provided herein, is induced by appropriate means (e.g., temperature shift or chemical induction) and cells are cultured for an additional period. Cells are typically harvested by centrifugation, disrupted by physical or chemical means, and the resulting crude extract retained for further purification. Microbial cells employed in expression of proteins can be disrupted by any convenient method, including freeze-thaw cycling, sonication, mechanical disruption, or use of cell lysing agents; such methods are well know to those skilled in the art.

Thus, for example, the nucleic acids of the invention as provided herein may be included in any one of a variety of expression vector constructs as a recombinant expression construct for expressing an ANT polypeptide. Such vectors and constructs include chromosomal, nonchromosomal and synthetic DNA sequences, *e.g.*, derivatives of SV40; bacterial plasmids; phage DNA; baculovirus; yeast plasmids; vectors derived from combinations of plasmids and phage DNA, viral DNA, such as vaccinia, adenovirus, fowl pox virus, and pseudorabies. However, any other vector may be used for preparation of a recombinant expression construct as long as it is replicable and viable in the host.

The appropriate DNA sequence(s) may be inserted into the vector by a variety of procedures. In general, the DNA sequence is inserted into an appropriate restriction endonuclease site(s) by procedures known in the art. Standard techniques for cloning, DNA isolation, amplification and purification, for enzymatic reactions involving DNA ligase, DNA polymerase, restriction endonucleases and the like, and various separation techniques are those known and commonly employed by those skilled in the art. A number of standard techniques are described, for example, in Ausubel et al. (1993 Current Protocols in Molecular Biology, Greene Publ. Assoc. Inc. & John Wiley & Sons, Inc., Boston, MA); Sambrook et al. (1989 Molecular Cloning, Second Ed., Cold Spring Harbor Laboratory, Plainview, NY); Maniatis et al. (1982 Molecular Cloning, Cold Spring Harbor Laboratory, Plainview, NY); and elsewhere.

The DNA sequence in the expression vector is operatively linked to at least one appropriate expression control sequences (e.g., a promoter or a regulated promoter) to direct mRNA synthesis. Representative examples of such expression control sequences include LTR or SV40 promoter, the *E. coli lac* or *trp,* the phage lambda $P_L$ promoter and other promoters known to control expression of genes in prokaryotic or eukaryotic cells or their viruses. Promoter regions can be selected from any desired gene using CAT (chloramphenicol transferase) vectors or other vectors with selectable markers. Two appropriate vectors are pKK232-8 and pCM7. Particular named bacterial promoters include lacI, lacZ,

T3, T7, gpt, lambda $P_R$, $P_L$ and trp. Eukaryotic promoters include CMV immediate early, HSV thymidine kinase, early and late SV40, LTRs from retrovirus, and mouse metallothionein-I. Selection of the appropriate vector and promoter is well within the level of ordinary skill in the art, and preparation of certain particularly preferred recombinant expression constructs comprising at least one promoter or regulated promoter operably linked to a nucleic acid encoding an ANT polypeptide is described herein.

In certain preferred embodiments the expression control sequence is a "regulated promoter", which may be a promoter as provided herein and may also be a repressor binding site, an activator binding site or any other regulatory sequence that controls expression of a nucleic acid sequence as provided herein. In certain particularly preferred embodiments the regulated promoter is a tightly regulated promoter that is specifically inducible and that permits little or no transcription of nucleic acid sequences under its control in the absence of an induction signal, as is known to those familiar with the art and described, for example, in Guzman et al. (1995 J. Bacteriol. 177:4121), Carra et al. (1993 EMBO J. 12:35), Mayer (1995 Gene 163:41*),* Haldimann et al. (1998 J. Bacteriol. 180:1277), Lutz et al. (1997 Nuc. Ac. Res. 25:1203), Allgood et al. (1997 Curr. Opin. Biotechnol. 8:474) and Makrides (1996 Microbiol. Rev. 60:512), all of which are hereby incorporated by reference. In other preferred embodiments of the invention a regulated promoter is present that is inducible but that may not be tightly regulated. In certain other preferred embodiments a promoter is present in the recombinant expression construct of the invention that is not a regulated promoter; such a promoter may include, for example, a constitutive promoter such as an insect polyhedrin promoter as described in the Examples or a yeast phosphoglycerate kinase promoter (see, *e.g.,* Giraud et al., 1998 J. Mol. Biol. 281:409). The expression construct also contains a ribosome binding site for translation initiation and a transcription terminator. The vector may also include appropriate sequences for amplifying expression.

Transcription of the DNA encoding the polypeptides of the present invention by higher eukaryotes may be increased by inserting an enhancer sequence into the vector. Enhancers are *cis*-acting elements of DNA, usually about from 10 to 300 bp that act on a promoter to increase its transcription. Examples including the SV40 enhancer on the late side of the replication origin bp 100 to 270, a cytomegalovirus early promoter enhancer, the polyoma enhancer on the late side of the replication origin, and adenovirus enhancers.

As noted above, in certain embodiments the vector may be a viral vector such as a retroviral vector. For example, retroviruses from which the retroviral plasmid vectors may be derived include, but are not limited to, Moloney Murine Leukemia Virus, spleen necrosis virus, retroviruses such as Rous Sarcoma Virus, Harvey Sarcoma virus, avian leukosis virus, gibbon ape leukemia virus, human immunodeficiency virus, adenovirus, Myeloproliferative Sarcoma Virus, and mammary tumor virus.

The viral vector includes one or more promoters. Suitable promoters which may be employed include, but are not limited to, the retroviral LTR; the SV40 promoter; and the human cytomegalovirus (CMV) promoter described in Miller, et al., Biotechniques 7:980-990 (1989), or any other promoter (e.g., cellular promoters such as eukaryotic cellular promoters including, but not limited to, the histone, pol III, and β-actin promoters). Other viral promoters which may be employed include, but are not limited to, adenovirus promoters, thymidine kinase (TK) promoters, and B19 parvovirus promoters. The selection of a suitable promoter will be apparent to those skilled in the art from the teachings contained herein, and may be from among either regulated promoters or promoters as described above.

The retroviral plasmid vector is employed to transduce packaging cell lines to form producer cell lines. Examples of packaging cells which may be transfected include, but are not limited to, the PE501, PA317, ψ-2, ψ-AM, PA12, T19-14X, VT-19-17-H2, ψCRE, ψCRIP, GP+E-86, GP+envAm12, and DAN cell lines as described in Miller, *Human Gene Therapy, 1*:5-14 (1990), which is incorporated herein by reference in its entirety. The vector may transduce the packaging cells through any means known in the art. Such means include, but are not limited to, electroporation, the use of liposomes, and $CaPO_4$ precipitation. In one alternative, the retroviral plasmid vector may be encapsulated into a liposome, or coupled to a lipid, and then administered to a host.

The producer cell line generates infectious retroviral vector particles which include the nucleic acid sequence (s) encoding the ANT polypeptides or fusion proteins. Such retroviral vector particles then may be employed, to transduce eukaryotic cells, either *in vitro* or *in vivo.* The transduced eukaryotic cells will express the nucleic acid sequence(s) encoding the ANT polypeptide or fusion protein. Eukaryotic cells which may be transduced include, but are not limited to, embryonic stem cells, embryonic carcinoma cells, as well as hematopoietic stem cells, hepatocytes, fibroblasts, myoblasts, keratinocytes, endothelial cells, and bronchial epithelial cells.

As another example of an embodiment of the invention in which a viral vector is used to prepare the recombinant ANT expression construct, in one preferred embodiment, host cells transduced by a recombinant viral construct directing the expression of ANT polypeptides or fusion proteins may produce viral particles containing expressed ANT polypeptides or fusion proteins that are derived from portions of a host cell membrane incorporated by the viral particles during viral budding. In another preferred embodiment, ANT encoding nucleic acid sequences are cloned into a baculovirus shuttle vector, which is then recombined with a baculovirus to generate a recombinant baculovirus expression construct that is used to infect, for example, Sf9 or *Trichoplusia ni* (PharMingen, Inc., San Diego, CA) host cells, as described in Baculovirus Expression Protocols, Methods in Molecular Biology Vol. 39, Christopher D. Richardson, Editor, Human Press, Totowa,

NJ, 1995; Piwnica-Worms, "Expression of Proteins in Insect Cells Using Baculoviral Vectors," Section II in Chapter 16 in: Short Protocols in Molecular Biology, 2nd Ed., Ausubel et al., eds., John Wiley & Sons, New York, New York, 1992, pages 16-32 to 16-48.

HOST CELLS

In another aspect, the present invention relates to host cells containing the above described recombinant ANT expression constructs. Host cells are genetically engineered (transduced, transformed or transfected) with the vectors and/or expression constructs of this invention which may be, for example, a cloning vector, a shuttle vector or an expression construct. The vector or construct may be, for example, in the form of a plasmid, a viral particle, a phage, etc. The engineered host cells can be cultured in conventional nutrient media modified as appropriate for activating promoters, selecting transformants or amplifying particular genes such as genes encoding ANT polypeptides or ANT fusion proteins. The culture conditions for particular host cells selected for expression, such as temperature, pH and the like, will be readily apparent to the ordinarily skilled artisan.

The host cell can be a higher eukaryotic cell, such as a mammalian cell, or a lower eukaryotic cell, such as a yeast cell, or the host cell can be a prokaryotic cell, such as a bacterial cell. Representative examples of appropriate host cells according to the present invention include, but need not be limited to, bacterial cells, such as *E. coli, Streptomyces, Salmonella tvphimurium;* fungal cells, such as yeast; insect cells, such as *Drosophila S2, Trichoplusia ni* (PharMingen, San Diego, CA) and *Spodoptera Sf9;* animal cells, such as CHO, COS or 293 cells; adenoviruses; plant cells, or any suitable cell already adapted to *in vitro* propagation or so established *de novo.* The selection of an appropriate host is deemed to be within the scope of those skilled in the art from the teachings herein.

Various mammalian cell culture systems can also be employed to express recombinant protein. Examples of mammalian expression systems include the COS-7 lines of monkey kidney fibroblasts, described by Gluzman, Cell 23: 175 (1981), and other cell lines capable of expressing a compatible vector, for example, the C127, 3T3, CHO, HeLa and BHK cell lines. Mammalian expression vectors will comprise an origin of replication, a suitable promoter and enhancer, and also any necessary ribosome binding sites, polyadenylation site, splice donor and acceptor sites, transcriptional termination sequences, and 5' flanking nontranscribed sequences, for example as described herein regarding the preparation of ANT expression constructs. DNA sequences derived from the SV40 splice, and polyadenylation sites may be used to provide the required nontranscribed genetic elements. Introduction of the construct into the host cell can be effected by a variety of methods with which those skilled in the art will be familiar, including but not limited to, for example, calcium phosphate transfection, DEAE-Dextran mediated transfection, or electroporation (Davis et al., 1986 Basic Methods in Molecular Biology*).*

As will be aprreciated by those of ordinary skill in the art, in certain situations it may be desirable to prepare the compositions of the invention and to practice the methods of the invention under conditions where endogenous ANT expression by a host cell is compromised, in order to provide advantages associated with the expression of a desired ANT encoding construct. For example, detection of particular ANT encoding nucleic acid sequences or ANT polypeptides that are highly similar to those encoded by the host cell genome may be facilitated by inhibiting host cell ANT gene expression. As another example, where functional activity of an exogenously introduced recombinant ANT polypeptide is to be determined in a host cell or in a biological sample derived therefrom, it may also be advantageous to inhibit endogenous host cell ANT gene expression.

Thus, in certain preferred embodiments of the invention, host cells may lack at least one isoform of an endogenous ANT, and in certain preferred embodiments the host cells may lack all endogenous ANT isoforms. For example, in the yeast system described by Giraud et al. (1998 J. Mol. Biol. 281:409) a *S. cerevisiae* triple null mutant is described that lacks all three yeast ANT isoforms and is unable to grow under anaerobic conditions. In other preferred embodiments, expression in host cells of at least one gene encoding an endogenous ANT isoform is substantially impaired. Substantial impairment of endogenous ANT isoform expression may be achieved by any of a variety of methods that are well known in the art for blocking specific gene expression, including site-specific or site-directed mutagenesis as described above, antisense inhibition of gene expression, ribozyme mediated inhibition of gene expression and generation of mitochondrial DNA depleted ($\rho^0$) cells.

Identification of oligonucleotides and ribozymes for use as antisense agents and DNA encoding genes for targeted delivery for genetic therapy involve methods well known in the art. For example, the desirable properties, lengths and other characteristics of such oligonucleotides are well known. Antisense oligonucleotides are typically designed to resist degradation by endogenous nucleolytic enzymes by using such linkages as: phosphorothioate, methylphosphonate, sulfone, sulfate, ketyl, phosphorodithioate, phosphoramidate, phosphate esters, and other such linkages *(see, e.g.,* Agrwal et al., Tetrehedron Lett. 28:3539-3542 (1987); Miller et al., J. Am. Chem. Soc. 93:6657-6665 (1971); Stec et al., Tetrehedron Lett. 26:2191-2194 (1985); Moody et al., Nucl. Acids Res. 12:4769-4782 (1989); Uznanski et al., Nucl. Acids Res. (1989); Letsinger et al., Tetrahedron 40:137-143 (1984); Eckstein, Annu. Rev. Biochem. 54:367-402 (1985); Eckstein, Trends Biol. Sci. 14:97-100 (1989); Stein In: Oligodeoxynucleotides. Antisense Inhibitors of Gene

Expression, Cohen, Ed, Macmillan Press, London, pp. 97-117 (1989); Jager et al., Biochemistry 27:7237-7246 (1988)).

Antisense nucleotides are oligonucleotides that bind in a sequence-specific manner to nucleic acids, such as mRNA or DNA. When bound to mRNA that has complementary sequences, antisense prevents translation of the mRNA (*see, e.g.,* U.S. Patent No. 5,168,053 to Altman et al.; U.S. Patent No. 5,190,931 to Inouye, U.S. Patent No. 5,135,917 to Burch; U.S. Patent No. 5,087,617 to Smith and Clusel et al. (1993) Nucl. Acids Res. 21:3405-3411, which describes dumbbell antisense oligonucleotides). Triplex molecules refer to single DNA strands that bind duplex DNA forming a colinear triplex molecule, thereby preventing transcription (*see, e.g.,* U.S. Patent No. 5,176,996 to Hogan et al., which describes methods for making synthetic oligonucleotides that bind to target sites on duplex DNA).

According to this embodiment of the invention, particularly useful antisense nucleotides and triplex molecules are molecules that are complementary to or bind the sense strand of DNA or mRNA that encodes an ANT polypeptide or a protein mediating any other process related to expression of endogenous ANT genes, such that inhibition of translation of mRNA encoding the ANT polypeptide is effected.

A ribozyme is an RNA molecule that specifically cleaves RNA substrates, such as mRNA, resulting in specific inhibition or interference with cellular gene expression. There are at least five known classes of ribozymes involved in the cleavage and/or ligation of RNA chains. Ribozymes can be targeted to any RNA transcript and can catalytically cleave such transcripts (*see, e.g.,* U.S. Patent No. 5,272,262; U.S. Patent No. 5,144,019; and U.S. Patent Nos. 5,168,053, 5,180,818, 5,116,742 and 5,093,246 to Cech et al.). According to certain embodiments of the invention, any such ANT mRNA-specific ribozyme, or a nucleic acid encoding such a ribozyme, may be delivered to a host cell to effect inhibition of ANT gene expression. Ribozymes, and the like may therefore be delivered to the host cells by DNA encoding the ribozyme linked to a eukaryotic promoter, such as a eukaryotic viral promoter, such that upon introduction into the nucleus, the ribozyme will be directly transcribed.

As used herein, expression of a gene encoding an endogenous adenine nucleotide translocator isoform is substantially impaired by any of the above methods for inhibiting when cells are substantially but not necessarily completely depleted of functional DNA or functional mRNA encoding the endogenous ANT isoform, or of the relevant ANT polypeptide. ANT isoform expression is substantially impaired when cells are preferably at least 50% depleted of DNA or mRNA encoding the endogenous ANT (as measured using high stringency hybridization as described above) or depleted of ANT polypeptide (as measured by Western immunoblot as described herein, see also, *e.g.*, Giraud et al. 1998 J. Mol Biol. 281:409); and more preferably at least 75% depleted of endogenous ANT DNA, mRNA or polypeptide. Most preferably, ANT isoform expression is substantially impaired when cells are depleted of >90% of their endogenous ANT DNA, mRNA, or polypeptide.

Alternatively, expression of a gene encoding an endogenous adenine nucleotide translocator isoform may be substantially impaired through the use of mitochondrial DNA depleted $\rho^0$ cells, which are incapable of mitochondrial replication and so may not continue to express functional ANT polypeptides. Methods for producing $\rho^0$ cells are known and can be found, for example in PCT/US95/04063, which is hereby incorporated by reference.

PROTEIN PRODUCTION

The expressed recombinant ANT polypeptides or fusion proteins may be useful in intact host cells; in intact organelles such as mitochondria, cell membranes, intracellular vesicles other cellular organelles; or in disrupted cell preparations including but not limited to cell homogenates or lysates, submitochondrial particles, uni- and multilamellar membrane vesicles or other preparations. Alternatively, expressed recombinant ANT polypeptides or fusion proteins can be recovered and purified from recombinant cell cultures by methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Protein refolding steps can be used, as necessary, in completing configuration of the mature protein. Finally, high performance liquid chromatography (HPLC) can be employed for final purification steps.

The polypeptides of the present invention may be a naturally purified product, or a product of chemical synthetic procedures, or produced by recombinant techniques from a prokaryotic or eukaryotic host (for example, by bacterial, yeast, higher plant, insect and mammalian cells in culture). Depending upon the host employed in a recombinant production procedure, the polypeptides of the present invention may be glycosylated or may be non-glycosylated. Polypeptides of the invention may also include an initial methionine amino acid residue.

SAMPLES

A "biological sample containing mitochondria" may comprise any tissue or cell preparation in which intact mitochondria capable of maintaining a membrane potential when supplied with one or more oxidizable substrates such as glucose, malate or galactose are or are thought to be present. Mitochondrial membrane potential may be determined according to methods with which those skilled in the art will be readily familiar, including but not limited to detection

and/or measurement of detectable compounds such as fluorescent indicators, optical probes and/or sensitive pH and ion-selective electrodes (*See, e.g.,* Ernster et al., 1981 J. Cell Biol. 91:227s and references cited therein; *see also* Haugland, 1996 Handbook of Fluorescent Probes and Research Chemicals- Sixth Ed., Molecular Probes, Eugene, OR, pp. 266-274 and 589-594.). By "capable of maintaining a potential" it is meant that such mitochondria have a membrane potential that is sufficient to permit the accumulation of a detectable compound (e.g., DASPMI [2-,4-dimethylaminostyryl-N-methylpyridinium], TMRM [tetramethylrhodamine methyl ester], *etc.*) used in the particular instance. A biological sample containing mitochondria may, for example, be derived from a normal (*i.e.,* healthy) individual or from an individual having a disease associated with altered mitochondrial function. Biological samples containing mitochondria may be provided by obtaining a blood sample, biopsy specimen, tissue explant, organ culture or any other tissue or cell preparation from a subject or a biological source. The subject or biological source may be a human or non-human animal, a primary cell culture or culture adapted cell line including but not limited to genetically engineered cell lines that may contain chromosomally integrated or episomal recombinant nucleic acid sequences, immortalized or immortalizable cell lines, somatic cell hybrid or cytoplasmic hybrid "cybrid" cell lines, differentiated or differentiatable cell lines, transformed cell lines and the like.

A "biological sample" may comprise any tissue or cell preparation as just described for a biological sample containing mitochondria, but does not require the presence of intact mitochondria. Thus a "biological sample" may comprise any tissue or cell preparation and a "biological sample containing at least one recombinant ANT polypeptide" comprises any tissue or cell preparation in which an expressed recombinant ANT polypeptide or fusion protein as provided herein is thought to be present. A biological sample may, for example, be derived from a recombinant cell line or from a transgenic animal. Biological samples containing recombinant ANT may be provided by obtaining a blood sample, biopsy specimen, tissue explant, organ culture or any other tissue or cell preparation from a subject or a biological source. The subject or biological source may be a human or non-human animal, a primary cell culture or culture adapted cell line including but not limited to genetically engineered cell lines that may contain chromosomally integrated or episomal recombinant nucleic acid sequences, immortalized or immortalizable cell lines, somatic cell hybrid or cytoplasmic hybrid "cybrid" cell lines, differentiated or differentiatable cell lines, transformed cell lines and the like.

PROTEINS

As described herein, isolation of a mitochondrial pore component or a mitochondrial molecular species with which an agent identified according to the methods of the invention interacts refers to physical separation of such a complex from its biological source, and may be accomplished by any of a number of well known techniques including but not limited to those described herein, and in the cited references. Without wishing to be bound by theory, a compound that "binds a mitochondrial component" can be any discrete molecule, agent compound, composition of matter or the like that may, but need not, directly bind to a mitochondrial molecular component, and may in the alternative bind indirectly to a mitochondrial molecular component by interacting with one or more additional components that bind to a mitochondrial molecular component. These or other mechanisms by which a compound may bind to and/or associate with a mitochondrial molecular component are within the scope of the claimed methods, so long as isolating a mitochondrial pore component also results in isolation of the mitochondrial molecular species that directly or indirectly binds to the identified agent. Thus, for example, as provided herein, any ANT polypeptide including recombinant ANT polypeptides and fusion proteins may be a mitochondrial molecular component and/or a mitochondrial pore component, and any ANT ligand or agent that binds to an ANT polypeptide may be a compound that binds a mitochondrial component and/or an agent that affects mitochondrial pore activity.

As described herein, the mitochondrial permeability transition "pore" may not be a discrete assembly or multisubunit complex, and the term thus refers instead to any mitochondrial molecular component (including, *e.g.*, a mitochondrial membrane *per se)* that regulates the inner membrane selective permeability where such regulated function is impaired during MPT. As used herein, mitochondria are comprised of "mitochondrial molecular components", which may be any protein, polypeptide, peptide, amino acid, or derivative thereof; any lipid, fatty acid or the like, or derivative thereof; any carbohydrate, saccharide or the like or derivative thereof, any nucleic acid, nucleotide, nucleoside, purine, pyrimidine or related molecule, or derivative thereof, or the like; or any other biological molecule that is a constituent of a mitochondrion. "Mitochondrial molecular components" includes but is not limited to "mitochondrial pore components". A "mitochondrial pore component" is any mitochondrial molecular component that regulates the selective permeability characteristic of mitochondrial membranes as described above, including those responsible for establishing $\Delta\Psi m$ and those that are functionally altered during MPT.

Isolation and, optionally, identification and/or characterization of the mitochondrial pore component or components with which an agent that affects mitochondrial pore activity interacts may also be desirable and are within the scope of the invention. Once an agent is shown to alter MPT according to the methods provided herein and in U.S. 09/161,172, those having ordinary skill in the art will be familiar with a variety of approaches that may be routinely employed to isolate the molecular species specifically recognized by such an agent and involved in regulation of MPT,

where to "isolate" as used herein refers to separation of such molecular species from the natural biological environment. Thus, for example, once an ANT ligand is prepared according to the methods provided herein, such approaches may be routinely employed to isolate the ANT polypeptide. Techniques for isolating a mitochondrial pore component such as an ANT polypeptide or fusion protein may include any biological and/or biochemical methods useful for separating the complex from its biological source, and subsequent characterization may be performed according to standard biochemical and molecular biology procedures. Those familiar with the art will be able to select an appropriate method depending on the biological starting material and other factors. Such methods may include, but need not be limited to, radiolabeling or otherwise detectably labeling cellular and mitochondrial components in a biological sample, cell fractionation, density sedimentation, differential extraction, salt precipitation, ultrafiltration, gel filtration, ion-exchange chromatography, partition chromatography, hydrophobic chromatography, electrophoresis, affinity techniques or any other suitable separation method that can be adapted for use with the agent with which the mitochondrial pore component interacts. Antibodies to partially purified components may be developed according to methods known in the art and may be used to detect and/or to isolate such components.

Affinity techniques may be particularly useful in the context of the present invention, and may include any method that exploits a specific binding interaction between a mitochondrial pore component and an agent identified according to the invention that interacts with the pore component. For example, because ANT ligands as provided herein and other agents that influence MPT can be immobilized on solid phase matrices, an affinity binding technique for isolation of the pore component may be particularly useful. Alternatively, affinity labeling methods for biological molecules, in which a known MPT-active agent or a novel ANT ligand as provided herein may be modified with a reactive moiety, are well known and can be readily adapted to the interaction between the agent and a pore component, for purposes of introducing into the pore component a detectable and/or recoverable labeling moiety. (*See, e.g.,* Pierce Catalog and Handbook, 1994 Pierce Chemical Company, Rockford, IL; Scopes, R.K., Protein Purification: Principles and Practice, 1987, Springer-Verlag, New York; and Hermanson, G.T. et al., Immobilized Affinity Ligand Techniques, 1992, Academic Press, Inc., California; for details regarding techniques for isolating and characterizing biological molecules, including affinity techniques.

Characterization of mitochondrial pore component molecular species, isolated by MPT-active agent affinity techniques described above or by other biochemical methods, may be accomplished using physicochemical properties of the pore component such as spectrometric absorbance, molecular size and/or charge, solubility, peptide mapping, sequence analysis and the like. *(See, e.g.,* Scopes, *supra.)* Additional separation steps for biomolecules may be optionally employed to further separate and identify molecular species that co-purify with mitochondrial pore components. These are well known in the art and may include any separation methodology for the isolation of proteins, lipids, nucleic acids or carbohydrates, typically based on physicochemical properties of the newly identified components of the complex. Examples of such methods include RP-HPLC, ion exchange chromatography, hydrophobic interaction chromatography, hydroxyapatite chromatography, native and/or denaturing one- and two-dimensional electrophoresis, ultrafiltration, capillary electrophoresis, substrate affinity chromatography, immunoaffinity chromatography, partition chromatography or any other useful separation method. Preferably extracts of cultured cells, and in particularly preferred embodiments extracts of biological tissues or organs may be sources of mitochondrial molecular components, including ANT polypeptides. Preferred sources may include blood, brain, fibroblasts, myoblasts, liver cells or other cell types.

## ANT LIGANDS

As noted above, the binding of the adenine nucleotide translocator (ANT) is responsible for mediating transport of ADP and ATP across the mitochondrial inner membrane. ANT has also been implicated as the critical component of the mitochondrial permeability transition pore, a $Ca^{2+}$ regulated inner membrane channel that plays an important modulating role in apoptotic processes. Additionally, ANT activity appears to be related to changes in ANT polypeptide conformation within the mitochondrial membrane, as evidenced by studies using agents that are capable of binding to ANT. (Block et al., 1986 Meths. Enzymol. 125:658) Accordingly, it is another aspect of the present invention to provide compositions and methods for producing and identifying agents that bind to ANT, which agents are also referred to herein as ANT ligands.

Binding interactions between ANT and a variety of small molecules are known to those familiar with the art. For example, these interactions include binding to ANT by atractyloside, carboxyatractyloside, palmitoyl-CoA, bongkrekic acid, thyroxin, eosin Y and erythrosin B. (See, *e.g.*, Stubbs, 1979 Pharm. Ther. 7:329; Klingenberg et al., 1978 Biochim. Biophys. Acta 503:193; Sterling, 1986 Endocrinol. 119:292; Majima et al., 1998 Biochem. 37:424; Block et al. 1986 Meths. Enzymol. 125:658; for erythrosin B and additional ANT inhibitors, see Beavis et al. 1993 J. Biol. Chem. 268:997; Powers et al. 1994 J. Biol. Chem. 269:10614.)

The ANT ligands of the present invention represent novel atractyloside derivatives. Atractyloside (ATR) and its known derivatives, including carboxyatractyloside (CATR), naphthoyl-ATR, MANT-ATR and other ATR derivatives (see, *e.g.,* Boulay et al., Analytical Biochemistry 128:323-330,1983; Roux et al., Analytical Biochemistry 234:31-37,1996;

Lauquin et al., FEBS Letters 67:306-311,1976; and Gottikh et al., Tetrahedron 26:4419-4433, 1970; for other known ATR derivatives see, *e.g.,* Block et al., 1986 Meths. Enzymol. 125:658) have proven invaluable in the elucidation of the structure and the mechanism of action of the adenine nucleotide translocator. According to the ANT ligands of the invention, the binding mode of ATR to ANT allows for modifications of the ATR 6'-hydroxyl functionality without significantly altering ATR binding affinity for ANT. Thus, ANT ligands as provided herein may be ATR derivatives modified by chemical substitution at the 6' hydroxyl position. In particular, the novel ANT ligands as provided herein further include long linker moieties at the 6' position, which linkers may include a 6'-amine linker, thereby permitting additional chemical modification to the ANT ligand as will be appreciated by those skilled in the art and as illustrated in the non-limiting Examples. Also, as shown in Examples 6-11, such linkers as provided herein may have carbon chain backbones of 2-20 carbon atoms, and in preferred embodiments 2-6 carbon atoms.

The invention therefore provides ANT ligands that may be intermediates for conjugation to a variety of additional chemical moieties to yield further ATR derivatives that are ANT ligands within the scope of the invention. These include ANT ligands to which $^{125}I$ may be covalently attached under mild reaction conditions; the invention also includes ANT ligands to which reactive amine groups may be covalently linked. ANT ligands which are such amine-containing ATR derivatives may then be reacted with a variety of fluorophores and haptens bearing, for example, reactive isothiocyanate, N-hydroxysuccinimide ester, anhydride and other useful functionalities to yield stable ATR derivatives including, for example, derivatives that have thiourea, amide or other linkages.

Thus, ANT ligands as provided herein also include ATR derivatives that are detectable by virtue of substituents introduced at the 6' position. Accordingly, detectable ATR derivatives as herein provided include ATR derivatives having a 6' hydroxyl substitution that includes a radiolabeled substituent, for example $^{125}I$, $^{131}I$, $^{3}H$, $^{14}C$ or $^{35}5$. Other ANT ligands that are detectable ATR derivatives may comprise fluorescent substituents, including those appropriately tagged with reporter molecules such as fluorophores and haptens having utility in high throughput screening assays for identifying agents that bind to ANT. More specifically, in preferred embodiments, an ANT ligand according to the present invention that includes fluorescent substituents has an extinction coefficient $\geq$10,000 $M^{-1}$ (see Table 1); further, this property provides an advantage for using such ANT ligands according to the methods provided herein, and in particular for use in high throughput screening assays. Additionally, the ANT ligands of the invention exhibit high affinities for ANT, and in preferred embodiments have binding constants in the nanomolar range.

In certain embodiments of the invention, ANT ligands may be ATR derivatives such as ATR-lanthanide chelating agents, which have utility in time-resolved fluorescence detection, for example detection of these compounds complexed to a lanthanide ion such as $Eu^{3+}$, $Tb^{3+}$, $Sm^{3+}$ and $Dy^{3+}$. In addition, ANT ligands may comprise ATR conjugated to readily detectable substituents such as highly fluorescent moieties, for example by way of illustration and not limitation, cyanine and coumarin derivatives. These and other highly fluorescent substituents permit the synthesis, according to the methods of the invention, of ANT ligands that are detectable with extremely high sensitivities. Those familiar with the art are aware of additional fluorescent substituents that may be used, for example, those disclosed in Haugland, 1996 Handbook of Fluorescent Probes and Research Chemicals- Sixth Ed., Molecular Probes, Eugene, OR. In other embodiments, the invention provides detectable ANT ligands produced by coupling of biotin-NHS ester with the ATR derivatives as disclosed herein; these and other ANT ligands similarly generated according to the instant methods can be detected with commercially available enzyme-avidin conjugates using, for example, colorimetric, fluorescent or chemiluminescent techniques.

In one embodiment, the ANT ligands of this invention have the following structure(I):

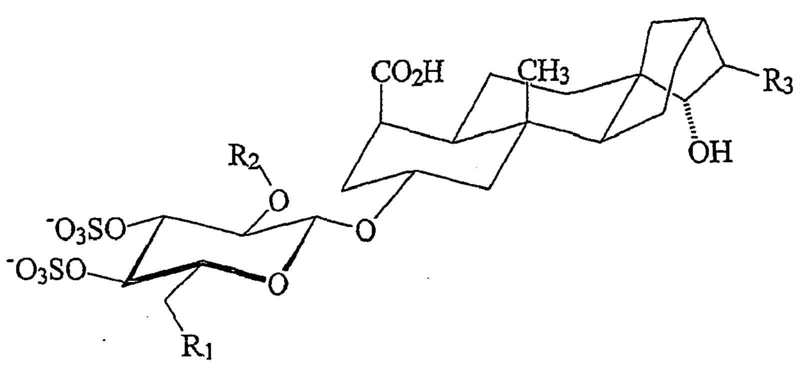

including stereoisomers and pharmaceutically acceptable salts thereof,
wherein

$R_1$ is hydroxyl, halogen, -OC(=O)$R_4$ or -NH$R_4$;
$R_2$ is hydrogen or -C(=O)$R_5$;
$R_3$ is -$CH_3$ or =$CH_2$;
$R_4$ is -X-aryl, -X-substituted aryl, -X-arylalkyl, -X-substituted arylalkyl, X-heteroaryl, or -X-heteroarylallcyl, wherein X is an optional amido or alkylamido linker moiety; and
$R_5$ is alkyl.

As used herein, the above terms have the meanings set forth below.

"Amido" means -NHC(=O)- or -C(=O)NH-.

"Alkylamido" means -(alkyl)-NHC(=O)- or -(alkyl)-C(=O)NH-, such as - $CH_2$NHC(=O)-, -$CH_2CH_2$NHC(=O)-, -$CH_2$C(=O)NH-, -$CH_2CH_2$C(=O)NH-, and the like.

"Alkyl" means a straight chain or branched, noncyclic or cyclic, unsaturated or saturated aliphatic hydrocarbon containing from 1 to 8 carbon atoms. Representative saturated straight chain alkyls include methyl, ethyl, n-propyl, n-butyl, n-pentyl, n-hexyl, and the like; while saturated branched alkyls include isopropyl, *sec*-butyl, isobutyl, *tert-butyl,* isopentyl, and the like. Representative saturated cyclic alkyls include cyclopropyl, cyclobutyl, cyclopentyl, cyclohexyl, and the like; while unsaturated cyclic alkyls include cyclopentenyl and cyclohexenyl, and the like. Unsaturated alkyls contain at least one double or triple bond between adjacent carbon atoms (referred to as an "alkenyl" or "alkynyl", respectively). Representative straight chain and branched alkenyls include ethylenyl, propylenyl, 1-butenyl, 2-butenyl, isobutylenyl, 1-pentenyl, 2-pentenyl, 3-methyl-1-butenyl, 2-methyl-2-butenyl, 2,3-dimethyl-2-butenyl, and the like; while representative straight chain and branched alkynyls include acetylenyl, propynyl, 1-butynyl, 2-butynyl, 1-pentynyl, 2-pentynyl, 3-methyl-1 butynyl, and the like.

"Aryl" means an aromatic carbocyclic moiety such as phenyl or naphthyl (i.e., 1- or 2-naphthyl).

"Arylalkyl" means an alkyl having at least one alkyl hydrogen atoms replaced with an aryl moiety, such as benzyl, -$(CH_2)_2$phenyl, -$(CH_2)_3$phenyl, and the like.

"Heteroaryl" means an aromatic heterocycle ring of 5- to 10 members and having at least one heteroatom selected from nitrogen, oxygen and sulfur, and containing at least 1 carbon atom, including both mono- and bicyclic ring systems. Representative heteroaryls are pyridyl, furyl, benzofuranyl, thiophenyl, benzothiophenyl, quinolinyl, pyrrolyl, indolyl, oxazolyl, benzoxazolyl, imidazolyl, benzimidazolyl, thiazolyl, benzothiazolyl, isoxazolyl, pyrazolyl, isothiazolyl, pyridazinyl, pyrimidinyl, pyrazinyl, triazinyl, cinnolinyl, phthalazinyl, and quinazolinyl.

"Heteroarylalkyl" means an alkyl having at least one alkyl hydrogen atom replaced with a heteroaryl moiety, such as -$CH_2$pyridinyl, -$CH_2$pyrimidinyl, and the like.

"Heterocycle" means a 5- to 7-membered monocyclic, or 7- to 10- membered bicyclic, heterocyclic ring which is either saturated, unsaturated, or aromatic, and which contains from 1 to 4 heteroatoms independently selected from nitrogen, oxygen and sulfur, and wherein the nitrogen and sulfur heteroatoms may be optionally oxidized, and the nitrogen heteroatom may be optionally quaternized, including bicyclic rings in which any of the above heterocycles are fused to a benzene ring. The heterocycle may be attached via any heteroatom or carbon atom. Heterocycles include heteroaryls as defined above. Thus, in addition to the heteroaryls listed above, heterocycles also include morpholinyl, pyrrolidinonyl, pyrrolidinyl, piperidinyl, hydantoinyl, valerolactamyl, oxiranyl, oxetanyl, tetrahydrofuranyl, tetrahydropyranyl, tetrahydropyridinyl, tetrahydroprimidinyl, tetrahydrothiophenyl, tetrahydrothiopyranyl, tetrahydropyrimidinyl, tetrahydrothiophenyl, tetrahydrothiopyranyl, and the like.

"Heterocyclealkyl" means an alkyl having at least one alkyl hydrogen atom replaced with a heterocycle, such as -$CH_2$morpholinyl, and the like.

The term "substituted" as used herein means any of the above groups (*i.e.,* alkyl, aryl, arylalkyl, heteroaryl, heteroarylalkyl, heterocycle and heterocyclealkyl) wherein at least one hydrogen atom is replaced with a substituent. In the case of a keto substituent ("C(=O)") two hydrogen atoms are replaced. Substituents include halogen, hydroxy, alkyl, haloalkyl, aryl, substituted aryl, arylalkyl, substituted arylalkyl, heterocycle, substitued heterocycle, heterocyclealkyl or substituted heterocyclealkyl.

"Halogen" means fluoro, chloro, bromo and iodo.

"Haloalkyl" means an alkyl having at least one hydrogen atom replaced with halogen, such as trifluoromethyl and the like.

"Alkoxy" means an alkyl moiety attached through an oxygen bridge (i. e., -O-alkyl) such as methoxy, ethoxy, and the like.

In one embodiment, $R_2$ is -C(=O)$CH_2CH(CH_3)_2$, $R_3$ is =$CH_2$, and the ANT ligand is an atractyloside derivative having the following structure (II):

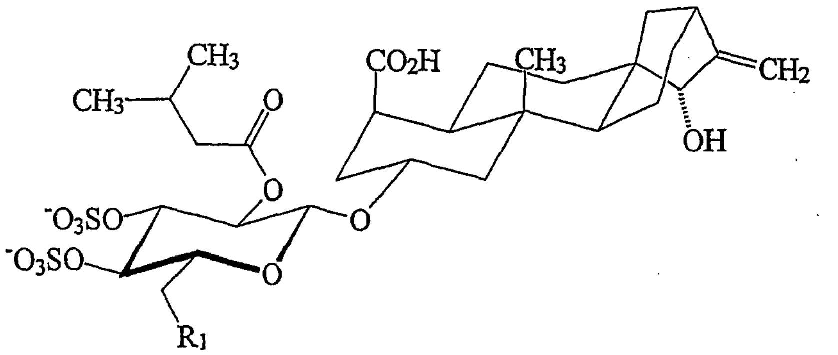

(II)

wherein $R_1$ is as defined above.

In another embodiment, $R_2$ is $-C(=O)CH_2CH(CH_3)_2$, $R_3$ is $-CH_3$, and the ANT ligand is a dihydro-atractyloside derivative having the following structure (III):

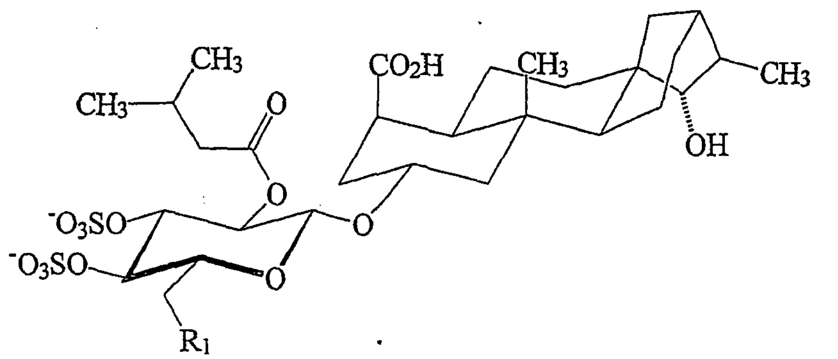

(III)

wherein $R_1$ is as defined above.

In still a further embodiment, $R_2$ is -OH, $R_3$ is $=CH_2$, and the ANT ligand is an apoatractyloside derivative having the following structure (IV):

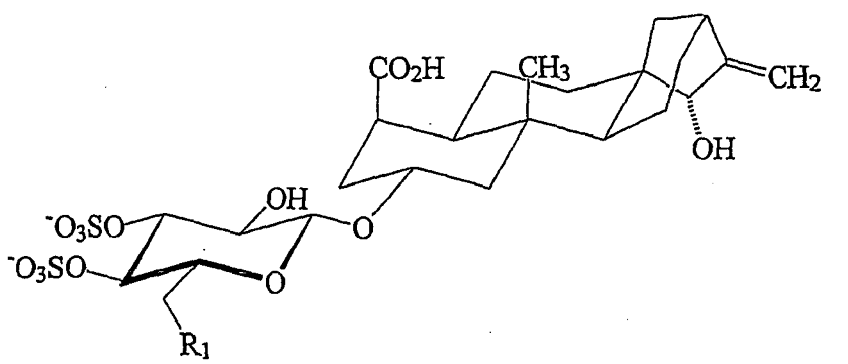

(IV)

wherein $R_1$ is as defined above.

In more specific embodiments of structures (II), (III) and (IV), $R_1$ is -OC(=O)(aryl), -OC(=O)(substituted aryl), -OC(=O)(arylalkyl), -OC(=O)(substituted arylalkyl), $-NH(CH_2)_2NHC(=O)$(arylalkyl), $-NH(CH_2)_2NHC(=O)$(substituted ar-

ylalkyl). Representative $R_1$ moieties in this regard include -OC(=O)(phenyl), -OC(=O)(1-naphthyl), -OC(=O)(substituted phenyl), -OC(=O)(substituted 1-naphthyl), - OC(=O)$(CH_2)_{1-3}$(phenyl), -OC(=O)$(CH_2)_{1-3}$(substituted phenyl), - NH $(CH_2)_2$NHC(=O)$(CH_2)_{1-3}$(phenyl), -NH$(CH_2)_2$NHC(=O)$(CH_2)_{1-3}$(substituted phenyl). In this context, representative substituted phenyl moieties include (but are not limited to) 4-hydroxyphenyl, 3-iodo-4-hydroxyphenyl, 3,5-iodo-4-hydroxyphenyl, 4-(4-hydoxyphenyl)phenyl, 4-(3-iodo-4-hyroxyphenyl)phenyl, 3-methyl-4-hyroxyphenyl, and 3-methyl-4-hydroxy-5-iodophenyl.

The ANT ligands of structure (I) may readily be made by one skilled in the art of organic chemisty and, more particularly, by the techniques disclosed in Examples 6-11.

The compounds of the present invention may generally be utilized as the free base. Alternatively, the compounds of this invention may be used in the form of acid addition salts. Acid addition salts of the free amino compounds of the present invention may be prepared by methods well known in the art, and may be formed from organic and inorganic acids. Suitable organic acids include maleic, fumaric, benzoic, ascorbic, succinic, methanesulfonic, acetic, oxalic, propionic, tartaric, salicylic, citric, gluconic, lactic, mandelic, cinnamic, aspartic, stearic, palmitic, glycolic, glutamic, and benzenesulfonic acids. Suitable inorganic acids include hydrochloric, hydrobromic, sulfuric, phosphoric, and nitric acids. Thus, the term "pharmaceutically acceptable salt" of structure (I) is intended to encompass any and all acceptable salt forms.

With regard to stereoisomers, the compounds of structure (I) may have chiral centers and may occur as recemates, reacemic mixtures and as individual enantiomers or diastereomers. All such isomeric forms are included within the present invention, including mixtures thereof. Furthermore, some of the crystalline forms of the compounds of structure (I) may exist as polymorphs, which are included in the present invention. In addition, some of the compounds of structure (I) may also form solvates with water or other organic solvents. Such solvates are similarly included within the scope of this invention.

Activities of ANT ligands are typically calculated from the $IC_{50}$ as the concentration of a compound necessary to displace 50% of the detectable (*i.e.*, detectably labeled, for example, radiolabeled) ligand from ANT molecules, which may be present as isolated or purified polypeptides or as components of preparations containing isolated mitochondria or submitochondrial particles (SMP) using established ligand binding assays or modifications thereof. For example, ANT ligands may be tested for their ability to compete with radiolabeled ATR, or with a radiolabeled ATR derivative such as compound 24 as provided herein, for binding to isolated ANT polypeptides or to ANT present in isolated mitochondria or SMP. Techniques for isolating mitochondria and for preparing SMP are well known to the person having ordinary skill in the art and may include certain minor modifications as appropriate for the particular conditions selected *(e.g.,* Smith, A.L., Meths. Enzymol. 10:81-86; Darley-Usman et al., (eds.), Mitochondria: A Practical Approach, IRL Press, Oxford, UK; Storrie et al., 1990 Meths. Enzymol. 182:203-255). Cell or tissue lysates, homogenates, extracts, suspensions, fractions or the like, or other preparations containing isolated mitochondrial molecular components such as mitochondrial proteins (e.g., naturally occurring or recombinantly expressed ANT) may also be useful in these and related embodiments. According to certain other related embodiments, one or more isolated mitochondrial molecular components such as an isolated ANT polypeptide may be present in membrane vesicles such as uni- or multilamellar membrane vesicles, or reconstituted into naturally derived or synthetic liposomes or proteoliposomes or similar membrane-bounded compartments, or the like, according to generally accepted methodologies (e.g., Jezek et al., 1990 J. Biol. Chem. 265: 10522-10526).

As another example, the relative affinities for ANT of various ANT ligands as provided herein may be determined by a fluorescence assay that exploits the flourescent properties of compound 22 (Example 11), a naphthoyl-ATR derivative that is an ANT ligand having a fluorescence excitation peak at 312 nm and an emission peak at 400 nm. When compound 22 is bound to ANT, the fluorescence is quenched. When, however, compound 22 is displaced from ANT by a known concentration of ATR or an ATR derivative that is an ANT ligand, fluorescence dequenching that results from displacement of the fluorophore can be measured in real time.

Briefly, a mitochondrial preparation (see, *e.g.*, Example 13) is washed and resuspended in a suitable buffer in the presence of compound 22 (*e.g.*, 10 mM Tris-120 mM KC1 containing 3.6 nmoles of compound 22 per mg mitochondrial protein, 10 min at room temperature), washed to remove unbound fluorophore and placed in a fluorometer equipped with a light source and filter set appropriate for the fluorophore. Fluorescence intensity is monitored as a function of time, and a candidate ANT ligand is then added to determine its ability to compete with compound 22 for binding to ANT, as evidenced by a change in detectable relative fluorescence intensity units. After the fluorescence signal has stabilized, any additional compound 22 that remains bound to ANT is displaced by adding an excess (*e.g.*, $\mu$M quantities) of ATR as a competitive inhibitor, to determine maximal signal intensity and therefrom calculate the proportion of compound 22 displaced by the candidate ANT ligand. Those having familiarity with the art will appreciate that variations and modifications may be made to ANT-binding assays such as those illustrated above and described in the Examples for determing $IC_{50}$ values of candidate ANT ligands, and which are not intended to be limiting.

Activity of each ANT ligand is reported as a "$K_i$" value calculated by the following equation:

$$K_i = \frac{IC_{50}}{1 + L / K_D}$$

where L = radioligand and $K_D$ = affinity of radioligand for receptor (Cheng and Prusoff, Biochem. Pharmacol. 22:3099, 1973). ANT ligands of this invention have a $K_i$ of 100 $\mu$M or less. In a preferred embodiment of this invention, the ANT ligands have a $K_i$ of less than 10 $\mu$M, and more preferably less than 1 $\mu$M. To this end, ANT ligands of this invention having a $K_i$ of less than 100 $\mu$M include compound 5 (Example 7), compound 6 (Example 8), and compounds 22, 23, 24, 26, 29, 33, 35, 37 and 38 (Example 11). Preferred ANT ligands having a $K_i$ of less than 10 $\mu$M include compounds 6, 22, 23, 24, 29, 33, 35, and 38 and more preferred ANT ligands having a $K_i$ of less than 1 $\mu$M include compounds 6, 24, 33, and 38, as well as ATR.

ASSAYS

It is another aspect of the invention to provide compositions and methods for the determination of the presence of ANT polypeptides and for the identification of agents that bind to, or that interact with, ANT polypeptides. Such compositions and methods will be useful for diagnostic and prognostic purposes, for example in the determination of the existence of altered mitochondrial function which, as described above, may accompany both normal and disease states. These compositions and methods will also be useful for the identification of agents that alter or regulate mitochondrial function based on ANT roles in mitochondrial activities, for example by way of illustration and not limitation, maintenance of mitochondrial membrane potential, ATP biosynthesis, induction of apoptosis, MPT and other mitochondrial function. In certain preferred embodiments these compositions and methods are useful as high throughput screening assays.

In certain aspects the invention provides a method for determining the presence of an ANT polypeptide in a biological sample, comprising contacting a sample suspected of containing an ANT polypeptide with an ANT ligand under conditions and for a time sufficieint to allow binding of the ANT ligand to an ANT polypeptide, and detecting such binding. "ANT ligands" according to these aspects of the invention may include any novel ANT ligands as provided herein. The use of human ANT1, ANT2 and ANT3 according to these methods represent particularly preferred embodiments. Other preferred embodiments include the use of any ANT polypeptide or ANT fusion protein as provided herein. Accordingly, the instant method for determining the presence of ANT polypeptide in a sample will be useful for monitoring expression of ANT encoding constructs provided herein. In some preferred embodiments an ANT fusion protein is used that is a GST fusion protein, and in other preferred embodiments the ANT fusion protein is a His-tagged fusion protein. As provided herein, the biological sample may be a cell, a mitochondrion, submitochondrial particles, a cell membrane (including any cellular membrane as described herein), a cell extract, cell conditioned medium, a tissue homogenate or an isolated ANT.

In other aspects, the invention provides a method for identifying an agent that binds to an ANT polypeptide, comprising contacting a candidate agent with a host cell expressing at least one recombinant ANT polypeptide under conditions and for a time sufficient to permit binding of the agent to the ANT polypeptide and detecting such binding. In various preferred embodiments the host cell may be a prokaryotic cell or a eukaryotic cell. In certain other preferred embodiments the host cell may lack at least one isoform of an endogenous ANT, for example, due to a mutation in one or more endogenous ANT encoding genes. In certain other embodiments host cell expression of at least one gene encoding an endogenous ANT isoform is substantially impaired, for example, through the use of ANT nucleic acid-specific ribozyme or antisense constructs as provided herein, or through the use of $\rho^0$ cells, as also provided herein. According to other embodiments of this aspect of the invention, it may be preferred to use intact cells or, alternatively, to use permeabilized cells. Those having ordinary skill in the art are familiar with methods for permeabilizing cells, for example by way of illustration and not limitation, through the use of surfactants, detergents, phospholipids, phospholipid binding proteins, enzymes, viral membrane fusion proteins and the like; through the use of osmotically active agents; by using chemical crosslinking agents; by physicochemical methods including electroporation and the like, or by other permeabilizing methodologies.

In other aspects, the invention provides a method for identifying an agent that binds to an ANT polypeptide comprising contacting a candidate agent with a biological sample containing at least one recombinant ANT polypeptide under conditions and for a time sufficient to permit binding of the agent to the ANT polypeptide, and detecting such binding. The use of human ANT1, ANT2 and ANT3 according to these methods represent particularly preferred embodiments. Other preferred embodiments include the use of any ANT polypeptide or ANT fusion protein as provided herein. In some preferred embodiments an ANT fusion protein is used that is a GST fusion protein, and in other preferred embodiments the ANT fusion protein is a His-tagged fusion protein. As provided herein, the biological sample may be

a cell, a mitochondrion, submitochondrial particles, a cell membrane (including any cellular membrane as described herein), a cell extract, cell conditioned medium, a recombinant viral particle, a tissue homogenate or an isolated ANT. Detection of binding may be by any of a variety of methods and will depend on the nature of the candidate agent being screened. For example, certain candidate agents are inherently detectable as a consequence of their physicochemical properties, such as will be apparent to those skilled in the art and including spectrophotometric, colorimetric, fluorimetric, solubility, hydrophobic, hydrophilic, electrostatic charge, molecular mass or other physicochemical properties: As another example, certain candidate agents may be radioactively labeled with a readily detectable radionuclide, as is well known in the art. Certain candidate agents may also be directly or indirectly detectable by ANT protein affinity methodologies, for example by their ability to interfere with binding of an ANT- specific antibody to an ANT; or by their being removable from an assay solution using a protein affinity reagent that binds to a fusion polypeptide present as a portion of an ANT fusion protein. A candidate agent bound to an ANT polypeptide may be detected by any method known for the detection, identification or characterization of relevant molecules, including spectrophotometric, mass spectrometric, chromatographic, electrophoretic, calorimetric or any other suitable analytical technique.

In another aspect the invention provides a method for identifying an agent that interacts with an ANT polypeptide comprising contacting a biological sample containing recombinant ANT with a detectable ANT ligand (or a known detectable molecule capable of binding to ANT) in the presence of a candidate agent, and comparing binding of the detectable ANT ligand (or known detectable ANT binding molecule) to recombinant ANT in the absence of the agent to binding of the detectable ANT ligand (or known detectable ANT binding molecule) to recombinant ANT in the presence of the agent, and therefrom identifying an agent that interacts with an ANT polypeptide. It will be appreciated that in certain preferred embodiments this aspect provides competitive binding assays wherein novel ANT ligands as provided hereinabove are useful. However, this aspect of the invention need not be so limited and may be modified to employ known detectable ANT binding molecules, in which case it should be pointed out that the selection of biological sample and/or of recombinant ANT as provided by the present invention offer unexpected advantages heretofore unknown in the art. Examples of known detectable ANT-binding molecules include suitably labeled ATP, ADP, ATR, CATR, palmitoyl-CoA, bongkrekic acid, thyroxin, eosin Y and erythrosin B or other ANT-binding molecules known in the art. (See, *e.g.,* Block et al., 1986 Meths. Enzymol. 125:658.) The use of human ANT1, ANT2 and ANT3 according to these methods represent particularly preferred embodiments. Other preferred embodiments include the use of any ANT polypeptide or ANT fusion protein as provided herein. In some preferred embodiments an ANT fusion protein is used that is a GST fusion protein, and in other preferred embodiments the ANT fusion protein is a His-tagged fusion protein. As provided herein, the biological sample may be a cell, a mitochondrion, submitochondrial particles, a cell membrane (including any cellular membrane as described herein), a cell extract, cell conditioned medium, a recombinant viral particle, a tissue homogenate or an isolated ANT.

The ANT ligands compounds are preferably part of a pharmaceutical composition when used in the methods of the present invention. The pharmaceutical composition will include at least one of a pharmaceutically acceptable carrier, diluent or excipient, in addition to one or more ANT ligands and, optionally, other components.

"Pharmaceutically acceptable carriers" for therapeutic use are well known in the pharmaceutical art, and are described, for example, in Remingtons Pharmaceutical Sciences, Mack Publishing Co. (A.R Gennaro edit. 1985). For example, sterile saline and phosphate-buffered saline at physiological pH may be used. Preservatives, stabilizers, dyes and even flavoring agents may be provided in the pharmaceutical composition. For example, sodium benzoate, sorbic acid and esters of p-hydroxybenzoic acid may be added as preservatives. Id. at 1449. In addition, antioxidants and suspending agents may be used. Id.

"Pharmaceutically acceptable salt" refers to salts of the compounds of the present invention derived from the combination of such compounds and an organic or inorganic acid (acid addition salts) or an organic or inorganic base (base addition salts). The compounds of the present invention may be used in either the free base or salt forms, with both forms being considered as being within the scope of the present invention.

The pharmaceutical compositions that contain one or more ANT substrates/ ligands compounds may be in any form which allows for the composition to be administered to a patient. For example, the composition may be in the form of a solid, liquid or' gas (aerosol). Typical routes of administration include, without limitation, oral, topical, parenteral (e.g., sublingually or buccally), sublingual, rectal, vaginal, and intranasal. The term parenteral as used herein includes subcutaneous injections, intravenous, intramuscular, intrastemal, intracavernous, intrameatal, intraurethral injection or infusion techniques. The pharmaceutical composition is formulated so as to allow the active ingredients contained therein to be bioavailable upon administration of the composition to a patient. Compositions that will be administered to a patient take the form of one or more dosage units, where for example, a tablet may be a single dosage unit, and a container of one or more compounds of the invention in aerosol form may hold a plurality of dosage units.

For oral administration, an excipient and/or binder may be present. Examples are sucrose, kaolin, glycerin, starch dextrins, sodium alginate, carboxymethylcellulose and ethyl cellulose. Coloring and/or flavoring agents may be present. A coating shell may be employed.

The composition may be in the form of a liquid, *e.g.*, an elixir, syrup, solution, emulsion or suspension. The

liquid may be for oral administration or for delivery by injection, as two examples. When intended for oral administration, preferred composition contain, in addition to one or more ANT substrates/ ligands compounds, one or more of a sweetening agent, preservatives, dye/colorant and flavor enhancer. In a composition intended to be administered by injection, one or more of a surfactant, preservative, wetting agent, dispersing agent, suspending agent, buffer, stabilizer and isotonic agent may be included.

A liquid pharmaceutical composition as used herein, whether in the form of a solution, suspension or other like form, may include one or more of the following adjuvants: sterile diluents such as water for injection, saline solution, preferably physiological saline, Ringer's solution, isotonic sodium chloride, fixed oils such as synthetic mono or digylcerides which may serve as the solvent or suspending medium, polyethylene glycols, glycerin, propylene glycol or other solvents; antibacterial agents such as benzyl alcohol or methyl paraben; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. The parenteral preparation can be enclosed in ampoules, disposable syringes or multiple dose vials made of glass or plastic. Physiological saline is a preferred adjuvant. An injectable pharmaceutical composition is preferably sterile.

A liquid composition intended for either parenteral or oral administration should contain an amount of ANT substrates/ ligands compound such that a suitable dosage will be obtained. Typically, this amount is at least 0.01 wt% of an ANT substrates/ ligands compound in the composition. When intended for oral administration, this amount may be varied to be between 0.1 and about 70% of the weight of the composition. Preferred oral compositions contain between about 4% and about 50% of ANT substrates/ ligands compound(s). Preferred compositions and preparations are prepared so that a parenteral dosage unit contains between 0.01 to 1% by weight of active compound.

The pharmaceutical composition may be intended for topical administration, in which case the carrier may suitably comprise a solution, emulsion, ointment or gel base. The base, for example, may comprise one or more of the following: petrolatum, lanolin, polyethylene glycols, beeswax, mineral oil, diluents such as water and alcohol, and emulsifiers and stabilizers. Thickening agents may be present in a pharmaceutical composition for topical administration. If intended for transdermal administration, the composition may include a transdermal patch or iontophoresis device. Topical formulations may contain a concentration of the ANT substrates/ ligands compound of from about 0.1 to about 10% w/v (weight per unit volume).

The composition may be intended for rectal administration, in the form, *e.g.*, of a suppository which will melt in the rectum and release the drug. The composition for rectal administration may contain an oleaginous base as a suitable nonirritating excipient. Such bases include, without limitation, lanolin, cocoa butter and polyethylene glycol.

In the methods of the invention, the ANT substrates/ ligands compound(s) may be administered through use of insert(s), bead(s), timed-release formulation(s), patch(es) or fast-release formulation(s).

It will be evident to those of ordinary skill in the art that the optimal dosage of the ANT substrates/ ligands compound(s) may depend on the weight and physical condition of the patient; on the severity and longevity of the physical condition being treated; on the particular form of the active ingredient, the manner of administration and the composition employed. It is to be understood that use of an ANT substrates/ ligands compound in a chemotherapy can involve such a compound being bound to an agent, for example, a monoclonal or polyclonal antibody, a protein or a liposome, which assist the delivery of said compound.

## EXAMPLES

The following Examples are offered by way of illustration and not by way of limitation.

### EXAMPLE 1

### CLONING AND EXPRESSION OF HIS-TAGGED HUMAN AND RAT ANT1 AND ANT2 PROTEINS IN BACTERIA

#### A. PCR Amplification of ANT cDNAs

Total cellular RNA prepared from whole human brain was obtained from a commercial source (Clontech, Palo Alto, CA). The RNA was purified by treatment with RNase-free DNase I (Roche Molecular Biochemicals, formerly Boehringer Mannheim Biochemicals, Indianapolis, IN) using 1 ul of DNase I (10 u/ul) in a buffer containing 40 mM Trsi-HCl, pH 7.0, 6 mM magnesium chloride and 2 mM calcium chloride for 30 minutes at 37˚C. This treatment was followed by two phenol/chloroform extractions, one chloroform extraction and an ethanol precipitation in the presence of sodium acetate. The RNA pellet was collected by centrifugation, washed with 70% ethanol, air dried, and resuspended in RNase-free sterile water. The RNA was reverse transcribed to generate cDNA using RNase H-deficient Reverse Transcriptase (SUPERSCRIPT™; Life Technologies, Rockville, MD).

ANT cDNAs were amplified by polymerase chain reactions (PCR) in a thermal cycler using the following primers,

AMPLITAQ™ DNA Polymerase (Perkin-Elmer), and reagents and buffers supplied in a GENEAMP™ PCR Reagent Kit (Perkin-Elmer), according to the manufacturer's instructions. In the following representations of the PCR primers, underlined nucleotides indicate sequences complementary to the 5'-ends and 3'-ends of the ANT cDNAs and double-underlined nucleotides indicate recognition sequences for the restriction enzymes *Xho*I (recognition sequence: 5'-CTCGAG) and *Asp*718 (recognition sequence: 5'-GGTACC).

For human ANT1 (huANT1; SEQ ID NO: 1), the following primers were used:
Forw

ard (sense):
5'-TTATATCTCGAGTATGGGTGATCACGCTTGGAGCTTCCTAAAG SEQ ID NO:4

and Reverse (antisense):
5'-TATATAGGTACCTTAGACATATTTTTGATCTCATCATACAAC SEQ ID NO:5.

For human ANT2 (huANT2; SEQ ID NO:2), the following primers were used:

Forward (sense):
5'- TTATATCTCGAGTATGACAGATGCCGCTGTGTCCTTCGCCAAG SEQ ID NO:6

and Reverse (antisense):
5'-TATATAGGTACCTTATGTGTACTTCTTGATTTCATCATACAAG SEQ ID NO:7.

DNAs encoding rat ANT isoforms have been described (GenBank Accession No. 398592). Procedures essentially identical to those described above were used to prepare rat ANT1 and ANT2 PCR products, with the exceptions that (1) a library of rat nucleic acids (Clontech, Palo Alto, CA) was used instead of a human library (a rat heart library was used for rat ANT1, and a rat liver library was used for rat ANT2), and (2) primers having sequences specific for the nucleic acid sequences of rat ANT1 or ANT2 were used.

For rat ANT1 (rANT1), the following primers were used:

Forward (sense):
CTCGAGATGGGGGATCAGGCTTTGAGCT SEQ ID NO:34
Reverse (antisense):
GGTACCTTACACATATTTTTGATCTCATCATA SEQ ID NO:35

For rat ANT2 (rANT2), the following primers were used:

Forward (sense):
CTCGAGATGACAGATGCCGCTGTGTCCT SEQ ID NO:36
Reverse (antisense):
GGTACCTTATGTGTACTTCTTGATTTCATCA SEQ ID NO:37

B. Generation of ANT Expression Constructs

PCR products were digested with the restriction endonucleases *Xho*I and *Asp*718 (both enzymes from Roche Molecular Biochemicals) according to the manufacturer's recommendations using manufacturer-supplied reaction buffers. Restricted DNAs were purified by horizontal agarose gel electrophoresis and band extraction using the UltraClean GelSpin kit (Mo Bio Laboratories, Inc., Solana Beach, CA).

The expression vector pBAD/His ("B" derivative; Invitrogen, Carlsbad, CA) was used. This vector contains the following elements operably linked in a 5' to 3' orientation: the inducible, but tightly regulatable, *araBAD* promoter; optimized *E. coli* translation initiation signals; an amino terminal polyhistidine(6xHis)-encoding sequence (also referred to as a "His-Tag"); an XPRESS™ epitope-encoding sequence; an enterokinase cleavage site which can be used to remove the preceding N-terminal amino acids following protein purification, if so desired; a multiple cloning site; and an in-frame termination codon.

Plasmid pBAD/His DNA was prepared by digestion with the restriction endonucleases *Xho*I and *Asp*718 according to the manufacturer's instructions and subjected to horizontal agarose gel electrophoresis and band extraction using the UltraClean GelSpin kit (Mo Bio Laboratories). Restricted ANT cDNAs were ligated into the linearized plasmid with restricted expression vector DNA using T4 DNA ligase (New England Biolabs, Beverly, MA) using the manufacturer's reaction buffer and following the manufacturer's instructions. Competent *rec*A1 *hsd*R *end*A1 *E. coli* cells (strain TOP10F';

Invitrogen, Catalog #C3030-03) were transformed with ligation mixtures containing the prokaryotic vector construct according to the manufacturer's instructions. Single colonies were selected and grown in 3-5 ml of LB broth (Sambrook, J., Fritsch, E.F., and Maniatis, T., Molecular Cloning: A Laboratory Manual, Cold Spring Harbor Laboratory Press, Cold Spring Harbor, NY, 1989) containing 50 μg/ml ampicillin (Roche Molecular Biochemicals). Plasmid DNA was isolated from the bacterial cultures using the WIZARD™ *Plus* Series 9600 Miniprep Reagents System (Promega, Madison, WI).

The recombinant huANT nucleotide sequences present in the expression constructs were determined and their authenticity confirmed relative to the published ANT sequences (Figure 1; *See* Neckelmann et al., Proc. Nat'l. Acad. Sci. U.S.A. 84:7580-7584 (1987) for huANT1 and Battini et al., J. Biol. Chem. 262:4355-4359 (1987) for huANT2 by sequencing using the PRISM™ Ready BIG DYE™ Terminator Cycle Sequencing Kit (The Perkin-Elmer Corp., Norwalk, CT) and the following sequencing primers 5'- TATGCCATAGCATTTTTATCC (SEQ ID NO:10) and 5'-CGCCAAAACAGCCAAGCT (SEQ ID NO:11). For each human ANT sequence, both primers are located inside the vector sequence adjacent to the DNA insertion. Sequence data was analyzed using the SEQUENCE NAVIGATOR™ analysis software package (Perkin-Elmer).

The expression plasmids encoding His-tagged human ANT1 and ANT2 are referred to herein as follows: For human ANT1, "pMK1 (His-tagged huANT1)" or simply "pMK1"; and, for human ANT2, "pMK2 (His-tagged huANT2)" or simply "pMK2"." Plasmids pMK1 and pMK2 were deposited at the American Type Culture Collection (ATCC; Manassas, VA) on November 3, 1998, and given the accession numbers ATCC 98969 and ATCC 98970, respectively.

The expression constructs derived from the pBAD/His expression expression vector and encoding His-tagged rat ANT1 and ANT2 are referred to herein as follows: "pMKr1" for the expression construct encoding 6xHis/XPRESS™ rat ANT1; and "pMKr2" for the expression construct encoding 6xHis/XPRESS™ rat ANT2.

EXAMPLE 2

PURIFICATION OF ANT PROTEINS

ANT proteins, and ANT fusion proteins, produced by the expression systems described herein are purified using a variety of methods. The purification of ANT proteins, particularly mammalian ANT proteins, is described in this Example. However, those skilled in the art may readily adapt and apply the methods exemplified in Example 1, and the following procedures, to cloned ANT proteins from any species of organism.

Regardless of which of the following protein purification methods is used, or others that can be derived from the present disclosure, it is important to add sufficient amounts of DNase and RNase to eliminate the viscosity associated with some bacterial lysates (typically 10 μg/mL of each enzyme; both from Roche Biochemicals) when the bacterial cells are lysed (or immediately thereafter). An alternative or additional means by which viscosity has been minimized and ANT solubility has been optimized is vigorous sonication, as opposed to standard sonication, of the lysates. The term "vigorous sonication" refers to, for example, sonication with a Branson Sonifier (Model 450) 2x (30 seconds each time) at 50% duty cycle and 80% output using a tapered, flat-tipped probe (as opposed to sonication with a cup and horn apparatus). Although either type of sonication will suffice, better yields have typically been observed when vigorous sonication has been used.

Furthermore, in various ANT purification methods, it is often desirable to make the lysate at least 1% Triton-X, in order to solubilize the maximum possible amount of ANT protein, after which insoluble material is removed by a high-speed (i.e., about 100,000 g) spin. Typically, protease inhibitors such as, for example, pepstatin, leupeptin, phenylmethylsulfonyl fluoride (PMSF) and/or aprotinin (all from Sigma) are added up to an effective level (typically 10 μg/mL) during the preparation. Depending on the particular ANT protein or ANT fusion protein being isolated, all four protease inhibitors or any effective combination thereof are used.

One method incorporates novel methods with several techniques previously used only for purifying ANT proteins from non-human mammals, i.e., bovine cardiac tissue and rats (Aquila et al., 1982, Hoppe-Seyler's Z. Physiol. Chem. 363:345-349; and Sterling, 1986, Endocrinology 119:292-295). In brief, bacterial cells expressing an ANT fusion protein are lysed by lysozyme treatment, and $^{14}C$-palmityl-CoA (Sigma) is added at a concentration of 50 nmol per gram of E. *coli.* Because it associates with ANT proteins, $^{14}C$-palmityl-CoA acts as a radiolabeled tracer that can be used to follow the ANT protein in subsequent purification steps. The lysates were then sonicated and made 6% Triton X-100 (Sigma) and incubated at 4°C for 1 hr to solubilize material. A high-speed spin was used to remove insoluble material, and the resulting solute was applied either (1) for small scale preparations, to hydroxyapatite beads (Bio-Rad Laboratories, Hercules, CA), or (2) in the case of larger preparations (i.e., ≥1 liter of bacterial culture), to a hydroxyapatite column (Bio-Rad) essentially according to the manufacturer's instructions. Unlike other intramembrane mitochondrial proteins, ANT has a low affinity for hydroxyapatite (Klingenberg et al., 1978, Biochim. et Biophys. Acta 503:193-210). The hydroxyapatite column was eluted with Column Buffer A (10mM MOPS, pH 7.2, 100Mm NaCl, 9.5% Triton X100) and washed with Column Buffer B (10 mM MOPS, pH 7.2, 100mM NaCl, 400mM sodium phosphate). Non-recombinant ANT proteins from the non-human host cells are eluted in the void volume with Column Buffer A, and the ANT fusion protein

is expected to be present in the void volume as well; Column Buffer B is used to wash the column in the event that ANT fusion protein behaves differently. Samples were collected in such a manner as to have a final concentration of 30 mM octyl glucoside (Calbiochem), a nonionic detergent that helps solubulize ANT proteins with minimal effect on activity (Sterling, 1986, Endrocrinol. 119: 292-295). The bead-extracted supernatant or column eluent was collected, and Triton X-100 was removed therefrom using the EXTRACTI-GEL™ affinity matrix (Pierce) essentially according to the manufacturer's instructions (see also Berman et al., 1985, Biochemistry 24:7140-7147).

Varying amounts of ANT isoforms, or fusion proteins thereof, prepared in the above manner were subject to PAGE and the gel was stained using a colloidal blue protein stain (Novex, San Diego, CA). The stained gel displayed a single band having a molecular weight corresponding to that predicted for the fusion protein. Based on the intensity of bands from samples of varying volumes, and the known volume of the preparation and minimal sensitivity of the stain, the yield from 100 mL of bacterial culture was estimated to be about 50 $\mu$g. In one of the lanes of the gel, approximately 500 ng of protein was loaded, and no contaminating bands were detected; this indicates that the His-tagged ANT fusion protein was from at least about 90% pure to at least about 95% pure.

ANT fusion proteins (see the Detailed Description of the Invention and Example 1) have been purified by this method, and other ANT fusion proteins, including His-tagged ANT1 and ANT2, and other His-tagged or otherwise-tagged ANT proteins, are purified in like fashion.

His-tagged ANT fusion proteins are additionally or alternatively further purified via Nickel-coated resins (such as, *e.g.*, PROBOND™ $Ni^{2+}$ charged agarose resin; Invitrogen) essentially according to the manufacturer's instructions. In brief, a solution comprising His-tagged ANT fusion proteins is contacted with the Nickel-coated resin, and the resin is washed to release undesirable contaminants. Next, the [resin:His-tagged ANT] complexes are treated with an appropriate enzyme, i.e., one that separates the ANT polypeptide from the remainder of the fusion protein. In the case of the His-tagged ANT fusion proteins described herein, enterokinase (Sigma, or EKMAX™ from Invitrogen may be used) cleaves the fusion protein in such a manner so as to produce two polypeptides: a first polypeptide comprising the His-tag and XPRESS™ epitope moieties, which remains bound to the resin, and a second polypeptide which corresponds to the ANT encoded by the particular expression construct used.

The ANT protein is released into the liquid phase, which is then collected to generate a solution comprising the ANT protein and some amount of the liberating enzyme. The amount of liberating enzyme needed is minimal because the treatment is catalytic in nature; nevertheless, some enzyme remains in the preparation. If desired, enzyme molecules may be removed from the preparation using any of a variety of means known in the art. For example, an enzyme may be removed from a solution by contacting the solution with a resin conjugated to a ligand having a high affinity for the enzyme. In the case of enterokinases, one such resin is the EK-AWAY™ resin (Invitrogen) which comprises the soybean trypsin inhibitor having a high affinity for enterokinases. In general, however, any suitable means for separating the liberating enzyme from any given ANT protein may be used.

ANT proteins produced by this expression system, and others described herein, are alternatively or additionally purified using known methods for purifying ANT proteins from humans and other mammals. See for example, Klingenberg et al., 1978 Biochim. Biophys. Acta 503:193-210; Aquila et al., 1982 Hoppe-Seyler's Z. Physiol. Chem. 363:345-349; and Sterling, 1986 Endocrinol. 119:292-295.

## EXAMPLE 3

## ANT ISOFORM-SPECIFIC ANTIBODIES

### 1. Pan Antibodies for ANT Isoforms

A monospecific (antipeptide) antibody that recognizes ANT1, ANT2 and ANT3 (hereinafter, a "Pan ANT antibody") was prepared as follows. A synthetic polypeptide corresponding to a portion of huANT3 located near the carboxy terminus and predicted to have high antigenicity according to the Jameson-Wolf Index (Wolf et al., Comput. Appl. Biosci. 4:187-191 (1988)) was synthesized using known means by Alpha Diagnostic International (San Antonio, TX) and determined to be at least about 70% pure, preferably at least about 90% pure, by HPLC and MS analyses. The sequence of the synthetic polypeptide (SEQ ID NO:30) is:

Cys-Trp-Arg-Lys-Ile-Phe-Arg-Asp-Glu-Gly-Gly-Lys-Ala-Phe-Phe

The synthetic polypeptide was conjugated to a carrier molecule, keyhole limpet hemocyanin (KLH), using MSB (*m*-maleimidobenzoyl-*N*-hydroxysuccinimide ester; Pierce Chemical Co., Rockford, Illinois), and the conjugated material was used to immunize several rabbits, according to known means (Collawn and Paterson, Units 11.14 and 11.15 in Chapter 11 in: Short Protocols in Molecular Biology, 2nd Ed., Asubel et al., eds., John Wiley & Sons, New York, New York (1992) 11:37-41. The rabbits were bled at 0 (preimmune, 2 mL), 7, 9, 11, 13 (15 mL for each bleed) or 15 weeks (50 mL) post-inoculation. Sodium azide (0.1%) was added to the bleeds as preservative.

2. ANT3-Specific Antibodies

A monospecific (antipeptide) antibody specific to ANT3 was prepared essentially according to the above-described procedure, with the exception that the synthetic peptide used was derived from a portion of the huANT3 polypeptide sequence, i.e., the following sequence (SEQ ID NO:38:
$Cys^+$-Ser-Gly-Thr-Glu-Arg-Glu-Phe-Arg-Gly-Leu-Gly-Asp-Cys-Leu-Val-Lys-Ile-Thr
The "$Cys^+$" residue in this and the following immunogenic synthetic polypeptides refers to cysteine residues not present in the natural huANT3 polypeptide; these "extra" Cys residues were introduced to facilitate the linking of the peptide to KHL hemocyanin. Rabbits were inoculated with the KHL-peptide conjugate and bled essentially according to the above-described procedure.

3. ANT2-specific Antibodies

A monospecific (antipeptide) antibody specific to ANT2 was prepared essentially according to the above-described procedure, with the exception that the synthetic peptide used had the following sequence (SEQ ID NO: 39) derived from huANT2:
Met-Thr-Asp-Ala-Ala-Val-Ser-Phe-Ala-Lys-Asp-Phe-Leu-Ala-Gly-$Cys^+$

3. ANT1-Specific Antibodies

A monospecific (antipeptide) antibody specific to ANT1 was prepared essentially according to the above-described procedure, with the exception that the synthetic peptide used had the following sequence (SEQ ID NO:40) derived from huANT1:
Met-Gly-Asp-His-Ala-Trp-Ser-Phe-Leu-Lys-Asp-Leu-Leu-Ala-Gly-$Cys^+$

4. Specificity of ANT Antibodies

The desired isoform-specific binding of the ANT1-, ANT2- and ANT3- specific antibodies was confirmed in the following manner. Extracts of bacterial host cells comprising expression vectors encoding His-Tagged ANT fusion proteins of huANT1, huANT2 and huANT3 (see the preceding Examples) were prepared as follows. Separate cultures of E. *coli* cells containing pMK1 (huANT1), pMK2 (huANT2) or pMK3 (huANT3) were grown in LB media containing 50 μg/ml ampicillin to mid-log phase ($OD_{600}$ ~ 0.5) and induced for 3-4 hours by the addition of arabinose to a final concentration of 0.02%). One ml of each culture was centrifuged at 5,000 x g for 10 minutes at 4°C to pellet the cells. Cell pellets were resuspended and lysed by adding 100 ul of Phosphate Buffered Saline (PBS; pH 7.4) containing 1% cholate, 1% n-dodecyl maltoside, and 0.1% 2-mercaptoethanol (in the preceding text, and throughout the specification, unless specified otherwise, all chemicals are from Sigma, St. Louis, MO). Total protein content in the lysates was determined using the BCA (bicinchoninic acid; Smith et al,, 1985, Anal. Biochem. 150:76-85) Protein Assay kit (Pierce Chemical Co., Rockford, IL). Ten μg of total protein was loaded per lane onto an SDS polyacrylamide gel, electrophoresed and transferred to a nitrocellulose membrane (HYBOND™ ECL Nitrocellulose Membrane, Amersham Pharmacia Biotech, formerely Amersham Life Sciences, Piscataway, NJ).

As shown in Figure 3 (left panel), the His-tagged ANT fusion proteins corresponding to huANT1, huANT2 and huANT3 were detected in the western blot using ANTI-XPRESS™ Antibody (Invitrogen) and horseradish peroxidase-conjugated anti-mouse secondary antibody (Amersham Pharmacia Biotech) according to the manufacturers' instructions. The central panel in Figure 3 shows the results of a western analysis of the same three ANT-expressing bacterial extracts but probed with the ANT1-specific antibody described above, and demonstrates that this antibody is specific for ANT1. Similarly, the right panel in Figure 3 shows the results of a western analysis of the same three ANT-expressing bacterial extracts but probed with the ANT2-specific antibody described above, and demonstrates that this antibody is specific for ANT2.

EXAMPLE 4

EXPRESSION OF ANT ISOFORMS IN INSECT CELLS

1. Generation of Baculovirus Expression Constructs

The ANT-encoding PCR products described in Example 1 were digested with the restriction endonucleases *Xho*I and *Kpn*I (both from New England Biolabs) according to the manufacturer's recommendations. Subsequent purification was carried out by horizontal agarose gel electrophoresis and band extraction using the UltraClean GelSpin kit

(Mo Bio Laboratories, Inc.).

The Baculovirus transfer vector pBlueBacHis2 (B version, Invitrogen) comprises, in 5' to 3' orientation, a constitutive polyhedrin promotor operably linked to nucleotide sequences encoding (1) a translation initiation sequence, (2) an N-terminal polyhistidine sequence, (3) an XPRESS™ epitope tag for detection and purification of the recombinant protein and (4) an enterokinas cleavage site, followed by a multiple cloning site wherein cDNAs can be inserted.

The transfer vector pBlueBacHis2 was prepared by digestion with the restriction endonucleases *Xho*I and *Kpn*I (an isoschizomer of *Asp718,* both of which have the recognition sequence: 5'-GGTACC) according to the manufacturer's instructions; after which the restricted DNA was subject to horizontal agarose gel electrophoresis and band extraction using the UltraClean™ GelSpin kit (Mo Bio Laboratories, Inc.). The restricted PCR products were ligated with the restricted expression vector DNA as in the preceding Examples.

Competent E. *coli* TOP10F' cells (Invitrogen) were transformed with the ligation reaction following the manufacturer's instructions. Single colonies were selected for growth in 3-5 ml of LB broth containing 50 μg/ml ampicillin. Plasmid DNA was isolated from the bacterial cultures using the WIZARD™*Plus* Series 9600 Miniprep Reagents System (Promega).

The recombinant ANT gene sequences were determined and their authenticities confirmed (SEQ ID NOS:1 and 2, corresponding to human ANTs 1 and 2, respectively; as well as the rat ANT1 and ANT2 sequences, Genbank accession nos. 398592 and D12771) by DNA sequencing using the Prism Ready Dye Terminator Cycle Sequencing Kit (Perkin-Elmer, Catalog #402080. Sequence data were analysed using the SEQUENCE NAVIGATOR™ analysis software package (Perkin-Elmer). Plasmids having the correct sequences were isolated and named pMK-11 (huANT1), pMK-12 (huANT2), pMK-13 (rANT1) and pMK-14 (rANT2).

Baculovirus Expression

In order to insert sequences encoding a particular ANT protein (and assoicated regulatory sequences) into the baculovirus genome, insect cells (MAXBAC™ *Spodoptera frugiperda* Sf9 cells, Invitrogen, Carlsbad, CA; or *Trichoplusia ni* {abbreviated as *T. ni.*} cells, PharMingen, San Diego, CA) were cotransfected with the baculoviral transfer constructs described above and linear baculoviral *(Autographa californica* nuclear polyhedrosis virus, AcMNPV) DNA engineered to contain a promoterless 3' fragment of the *lacZ* gene (BAC-N-BLUE™, Invitrogen) using the BAC-N-BLUE™ Transfection Kit (Invitrogen) following the manufacturer's instructions. Recombinant baculovirus plaques express functional beta-galactosidase and were identified as blue plaques in the presence of X-gal (5-bromo-4-chloro-3-indoyl-beta-D-glactosidase). These recombinant viruses are expression constructs that express the ANT polypeptide of choice in insect cells, as shown by the following experiments.

High titer viral stock was produced, and recombinant protein is expressed in infected Sf9 (Invitrogen, Carlsbad, CA) or *T*. ni (PharMingen, San Diego, CA) cells according to the manufacturer's instructions (see also Piwnica-Worms, Expression of Proteins in Insect Cells Using Baculovirus Vectors, Section II of Chapter 16 in: Short Protocols in Molecular Biology, 2nd Ed., Asubel et al., eds., John Wiley & Sons, New York, New York, 1992, pages 16-32 to 16-48; Kitts, Chapter 7 in: Baculovirus Expression Protocols, Methods in Molecular Biology, Vol. 39, C.R. Richardson, Ed., Humana Press, Totawa, NJ, 1995, pages 129-142).

*T*. ni cells were prepared by a subcontractor (PharMingen, San Diego, CA) as portions of about 250 mg of cells per tube. Each portion was suspended in 1 ml of MSB with protease inhibitors (leupeptin, final concentration 10 μg/ml; pepstatin, final concentration 10 μg/ml; aprotinin, final concentration, 2 μg/ml; phenylmethylsulfonyl fluoride, [PMSF], final concentration, 100 μM; all from Sigma Chemical Co., St. Louis, MO). The resuspended cell suspensions were frozen and thawed twice, then homogenized using a rotating teflon-coated probe and a close-fitting glass container (10 passes). The cellular homogenate was centrifuged (3,700 rpm, approximately 1,500 x g) at 4˚C for 5 minutes; this supernatant from the first spin was saved. The pellet was washed with about 500 μl of MSB with protease inhibitors, centrifuged (3,800 rpm, approximately 1,600 x g) at 4˚C for 5 minutes, and supernatant from this spin was combined with the supernatant from the first spin. The combined supernatant was centrifuged (14,000 rpm, approximately 20,800 x *g*) at 4˚C for 15 minutes, and the pellet was resuspended in 300 μl of a 1:1 solution of (a) 20 mM MOPS and (b) MSB, wherein both (a) and (b) contain the previously described protease inhibitors. The resultant suspension was frozen and thawed three times.

High titer viral stock was produced, and recombinant protein is expressed in infected Sf9 (Invitrogen, Carlsbad, CA) or *T. ni* (PharMingen, San Diego, CA) cells according to the manufacturer's instructions (see also Piwnica-Worms, Expression of Proteins in Insect Cells Using Baculovirus Vectors, Section II of Chapter 16 in: Short Protocols in Molecular Biology, 2nd Ed., Asubel et al., eds., John Wiley & Sons, New York, New York, 1992, pages 16-32 to 16-48; Kitts, Chapter 7 in: Baculovirus Expression Protocols, Methods in Molecular Biology, Vol. 39, C.R. Richardson, Ed., Humana Press, Totawa, NJ, 1995, pages 129-142).

To confirm expression of the ANT of choice in the insect expression system, lysates of transfected Sf9 cells are pelleted by centrifugation and lysed by adding 100 μl of MSB buffer (210 mM mannitol (Sigma), 70 mM sucrose

(Fluka), 50 mM Tris-HCl, pH 7.4, 10 mM EDTA) and performing three freeze-thaw cycles. The total protein concentration of each lysate is determined, and identical amounts of protein are electrophoresed and transferred to nitrocellulose membranes, as described in the preceding Examples. The transferred proteins are probed in these western experiments with ANTI-XPRESS™ Antibody (Invitrogen), as the ANT fusion proteins expressed in this system include the XPRESS™ epitope, as described in the previous Examples.

EXAMPLE 5

EXPRESSION OF ANT IN MAMMALIAN CELLS

The preceding Examples describe a variety of means by which ANT and ANT fusion proteins can be recombinantly produced in various systems. Although such ANT proteins can be used in a variety of assays (see *infra),* it may be desirable to express and/or isolate large amounts of the native ANT protein from mammalian cells. One use of mammalian expression systems is the generation and isolation of human mitochondria in which a particular ANT isoform is over-represented in order to determine the specific biological role(s) of such isoforms. For example, ANT3 is apparently ubiquitously expressed in human tissues in a manner believed to reflect the relative aerobic activity of a particular cell type, whereas ANT1 may be primarily expressed in brain, heart and skeletal muscle, and ANT-2 is widely expressed but exhibits higher expression levels in proliferative cells (Stepien et al., 1992, J. Biol. Chem. 267:14592-14597; Fiore et al., 1998 Biochimie 80:137; Li et al., 1989 J. Biol. Chem. 264:13998; Graham et al., 1997 Nat. Genet. 16:226; Giraud et al., 1998 J. Mol. Biol. 281:409). Directed overexpression of huANT1 in cultured heart or muscle cells is expected to result in mitochondria that contain mostly the ANT1 isoform. Such "ANT1-enriched" mitochondria can be isolated and tested for various mitochondrial functions related to ANT activities as mediated by a particular ANT isoform.

A. Shuttle Vectors

One type of mammalian ANT expression constructs is derived from commercially or otherwise available "shuttle" (i.e., capable of replicaton in both *E. coli* and mammalian cells) vectors that comprise promoters that function in mammalian cells and can be operably linked to an ANT-encoding sequence. Such shuttle vectors include, but are not limited to, SV40 late promoter expression vectors (e.g., pSVL, Pharmacia), glucocorticoid-inducible promoter expression vectors (e.g., pMSG, Pharmacia), Rous sarcoma enhancer-promoter expression vectors (e.g., pRc/RSV, Invitrogen) and CMV early promoter expression vectors, including deriavtives thereof having selectable markers to agents such as Neomycin, Hygromycin or ZEOCIN™ (e.g., pRc/CMV2, pCDM8, pcDNA1.1, pcDNA1.1/Amp, pcDNA3.1, pcDNA3.1/Zeo and pcDNA3.1/Hygro, Invitrogen) In general, preferred shuttle vectors for ANT genes are those having selectable markers (for ease of isolation and maintenance of transformed cells) and inducible, and thus regulatable, promoters (as overexpression of ANT genes may have toxic effects).

Methods for transfecting mammalian cells are known in the art (see, Kingston et al., "Transfection of DNA into Eukaryotic Cells," Section I of Chapter 9 in: Short Protocols in Molecular Biology, 2nd Ed., Asubel et al., eds., John Wiley & Sons, New York, New York, 1992, pages 9-3 to 9-16). A control plasmid, such as pCH110 (Pharmacia), may be cotransfected with the ANT expression construct being examined so that levels of ANT can be normalized to a gene product expressed from the control plasmid.

In the present Example, the development of mammalian expression constructs for huANT1 and huANT2 is described. The shuttle plasmid pcDNA3.1(-) (Invitrogen) was digested with the restriction enzymes *Xho*I and *Kpn*I as described in the preceding Examples. *Xho*I- and *Kpn*I-digested PCR products comprising coding sequences for huANT1 or huANT2 were ligated to the digested pcDNA3.1(-) DNA as in the previous Examples. The resultant expression constructs operably link the ANT gene of choice to a CMV (cytomegalovirus) promoter that drives transcription of the ANT gene in mammalian cells. In order to confrim expression of ANT protein from a mammalian expression construct, western analyses of lysates of cells comprising ANT expression systems are performed essentially as described in the preceding Examples, with the exception that different methods are used to prepare protein preparations from mammalian cells as opposed to bacterial or insect cells.

B. Viral expression

Nucleic acids, preferably DNA, comprising preferred expression cassettes are isolated from the shuttle vector derived expression constructs in which they were prepared, characterized and optimized (see preceding section). A preferred method of isolating such expression cassettes is by amplification by PCR, although other methods (e.g., digestion with appropriate restriction enzymes) can be used. Preferred expression cassettes are introduced into viral expression vectors, preferably retroviral expression vectors, in the following manner.

A DNA molecule comprising a preferred expression cassette is introduced into a retroviral transfer vector by

ligation (see preceding Examples). Two types of retroviral transfer vectors are known in the art: replication-incompetent and replication-competent. Replication-incompetent vectors lack viral genes necessary to produce infectious particles but retain cis-acting viral sequences necessary for viral transmission. Such cis-acting sequences include the Ψ packaging sequence, signals for reverse transcription and integration, and viral promoter, enhancer, polyadenylation and other regulatory sequences. Replication-competent vectors retain all these elements as well as genes encoding virion structural proteins (typically, those encoded by genes designated *gag, pol* and *env*) and can thus form infectious particles in a variety of cell lines. In contrast, these functions are supplied in *trans* to replication-incompetent vectors in a packaging cell line, i.e, a cell line that produces mRNAs encoding *gag, pol* and *env* genes but lacking the Ψ packaging sequence. See, generally, Cepko, Unit 9.10 of Chapter 9 in: Short Protocols in Molecular Biology, 2nd Ed., Asubel et al., eds., John Wiley & Sons, New York, New York, 1992, pages 9-30 to 9-35.

A retroviral construct comprising an ANT expression cassette produces RNA molecules comprising the cassette sequences and the Ψ packaging sequence. These RNA molecules correspond to viral genomes that are encapsidated by viral structural proteins in an appropriate cell line (by "appropriate" it is meant that, for example, a packaging cell line must be used for constructs based on replication-incompetent retroviral vectors). Infectious viral particles are then produced, and released into the culture supernatant, by budding from the cellular membrane. The infectious particles, which comprise a viral RNA genome that includes the ANT expression cassette, are prepared and concentrated according to known methods. It may be desirable to monitor undesirable helper virus, *i.e.,* viral particles which do not comprise an ANT expression cassette. See, generally, Cepko, Units 9.11, 9.12 and 9.13 of Chapter 9 in: Short Protocols in Molecular Biology, 2nd Ed., Asubel et al., eds., John Wiley & Sons, New York, New York, 1992, pages 9-36 to 9-45.

Viral particles comprising an ANT expression cassette are used to infect *in vitro* (e.g., cultured cells) or *in vivo* (e.g., cells of a rodent, or of an avian species, which are part of a whole animal). Tissue explants or cultured embryos may also be infected according to methods known in the art. See, generally, Cepko, Unit 9.14 of Chapter 9 in: Short Protocols in Molecular Biology, 2nd Ed., Asubel et al., eds., John Wiley & Sons, New York, New York, 1992, pages 9-45 to 9-48. Regardless of the type of cell used, production of ANT protein is directed by the recombinant viral genome.

In a preferred embodiment, recombinantly produced ANT proteins are inserted into the cell membrane of cultured cells. Because the retroviral expression construct produces viral particles by budding of the cell membrane, the resultant viral particles delivered to the culture supernatant have ANT protein incorporated into their capsules, preferably on the surface of the particles. Such ANT-displaying viral particles are expected to provide a stable format for ANT proteins and to thus be useful in assays using ANT proteins, either directly or as a source material from which ANT can be further purified. If it is desired to minimize the amount of ANT protein inserted into mitochondrial membranes, $\rho^0$ cells, which have been treated in such a manner as to be nearly or completely devoid of mitochondria, are used as host cells. As is described in more detail below, SH-SY5Y cells have very little endogenous ANT proteins and are thus also preferred host cells.

C. ANT Antisense Constructs

Antisense versions of the preceding transient and viral ANT expression constructs are prepared by exchanging the antisense (non-encoding) strand for a sense (ANT protein encoding) strand in a construct. Such ANT antisense constructs are useful as research reagents, i.e., to reduce levels of expression of one or more isoforms in a cell transformed or infected with such a construct in order to determine the effects of such treatment on cellular physiology. ANT antisense constructs are also useful as gene therapy agents that interfere with the translation of one or more isoforms of ANT.

EXAMPLE 6

SYNTHESIS AND PROPERTIES OF REPRESENTATIVE ATR DERIVATIVES

A number of atractyloside (ATR) derivatives were prepared for use as ligands for adenine nucleotide translocators (ANTs) in the context of high-throughput screening assays. These compounds bind with high affinity *(i.e.,* in the nM range) to ANT and are thus useful for screening libraries of chemical compounds for molecules having high specificity for ANT (regardless of isoform) The structure of ATR is set forth below as compound (1). Compounds (3) and (4) represent novel fluorescent derivatives of ATR, while compound (2) is an ATR derivative which permits introduction of the $^{125}I$ under mild conditions.

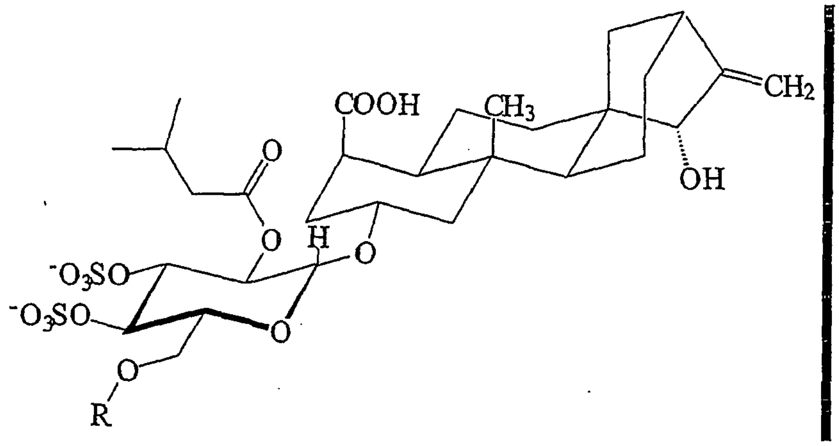

R = H

1

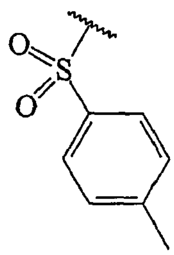

2

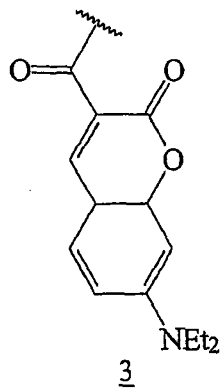

3

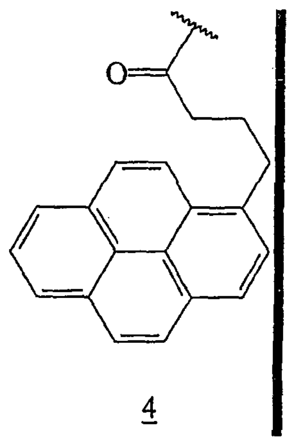

4

Purification

Compounds bland 4 were purified by silica gel chromatography using $CH_2Cl_2$/MeOH/AcOH (75:25:1) as the eluting solution. Detection was achieved by staining with a 0.5% solution of vanillin in $H_3PO_4$/$H_2O$ (1/1). Further purification was accomplished by reversed-phase HPLC using a Microsorb C8 column (250 x 10 mm). The column was eluted at a flow rate of 2.0 mL/min with a linear gradient of methanol/acetic acid/l M ammonium acetate 98:1:1 ("Solvent B") and $H_2O$/acetic acid/l M ammonium acetate aqueous solution 98:1:1 ("Solvent A"). The effluent was monitored for absorbance at 254 nm. Compound-containing fractions were pooled, evaporated, and repeatedly co-evaporated with added methanol (3x 5 mL).

Synthesis of Compound 2

Atractyloside 1 (0.10 mmol) was dried by repeated evaporation of added pyridine (3x 5 mL) and the resulting gummy residue dissolved in pyridine (5 mL). To the resulting solution, 0.20 mmol of toluenesulfonyl chloride was added. The reaction mixture was stirred at ambient temperature for 1.5 h. Then, another portion of toluenesulfonyl chloride (0.20 mmol) was added and the reaction left stirring an additional 1.5 h. 1 mL of methanol was added to the reaction mixture which was then stirred for 0.5 h, after which solvents were removed by evaporation. Residual pyridine was removed by evaporation of additional methanol (5x 10 mL). Silica gel chromatography followed by reversed-phase HPLC using a linear gradient of 50-80% of solvent B in solvent A for 30 min. resulted in the compound 2 eluting at 68% solvent B. Yield: 4.3 mg, 4.9%. ESI-MS (M-H) found:879, calc.:879.

Synthesis of Compounds 3 and 4

7-Diethylamino-2-oxo-2H-chromene-3-carboxylic acid or 0.20 mmol of 1-pyrenebutyric acid and 0.60 mmol of 1,1'-carbonyldiimidazole in 1 mL of dimethylformamide were allowed to react for 15 min. To the activated carboxylic acid was added a solution of atractyloside 1 in $H_2O$ (4 mL) and the resulting reaction mixture was stirred at ambient temperature for 16 h. Evaporation left a gummy residue which was purified by silica gel chromatography followed by reversed-phase HPLC. Using a linear gradient of 10-80% of solvent B in solvent A for 50 min (for compound 3) or 50-100% of solvent B in solvent A for 50 min (for compound 4) resulted in compound 3 eluting at 75% B and compound 4 eluting at 82% B. Yields: compound 3,3.1 mg, 8.0%; compound 4,1.3 mg, 3.6%. ESI-MS (M-H) compound 3 found: 968, calc.: 968; compound 4 found:995, calc.:995.

Properties of Representative ATR Derivatives

As summarized in Table 1 below, compounds 3 and 4 were found to be more advantageous in terms of fluorescence characteristics and sensitivity compared to the existing ATR derivatives Naphthoyl-ATR and MANT-ATR as reported by Boulay et al., Analytical Biochemistry 128:323-330,1983; Roux et al., Analytical Biochemistry 234: 31-37,1996; and Lauquin et al., FEBS Letters 67:306-311,1976.

Table 1

| ATR Derivative | Excitation (nm) | Emission (nm) | Extinction Coefficient ($M^{-1}$) (Predicted) |
|---|---|---|---|
| Naphthoyl-ATR | 300 | 405 | 6,200 |
| MANT-ATR | 350 | 460 | 5,800 |
| Compound 4 | 341 | 391 | 17,420 |
| Compound 3 | 417 | 470 | 46,400 |

EXAMPLE 7

SYNTHESIS OF REPRESENTATIVE ATR DERIVATIVE

The further representative ATR derivative, compound 5, was prepared by the procedure set forth below.

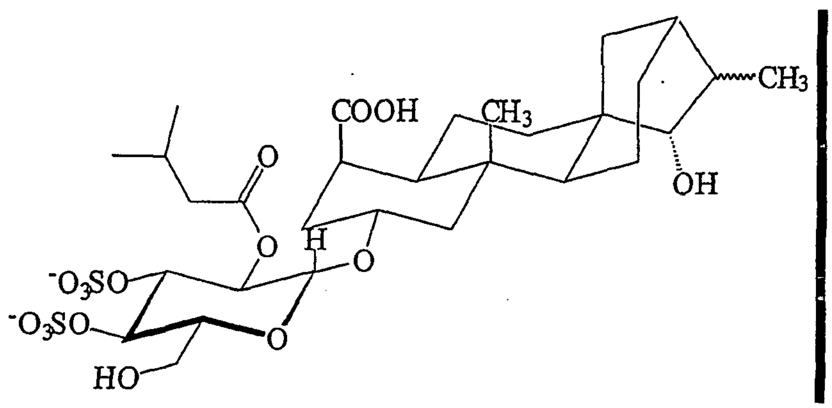

5

Synthesis of Compound 5

Dipotassium atractylate (0.10 mmol) was dissolved in 50% aq. ethanol (5 mL) and palladium on charcoal (10%, 17 mg) was added to the reaction mixture. After flushing the system with hydrogen, the reaction mixture was stirred under an atmosphere of hydrogen gas for 3 h. Removal of catalyst by filtration through Celite, washing with 50% aq. ethanol (10 mL), and evaporation of solvents afforded a white solid. Yield after thorough drying under high vacuum;

78.3 mg (97.3%). ESI-MS (M-2H+K) found:765, calc.:765. $^1$H-NMR analysis confirmed the absence of alkenic protons: DMSO-$d_6$) $\delta$ 0.88 (d, 3H), 0.89(d, 3H), 1.02(d, 3H).

EXAMPLE 8

SYNTHESIS OF REPRESENTATIVE IODINATED ATR DERIVATIVE

Compound 2 of Example 6 may be used as intermediate for conjugation of variety of chemical moieties to yield further ATR derivatives. In this example, compound 2 is employed to introduce $^{125}$I under mild conditions to yield the following compound 6.

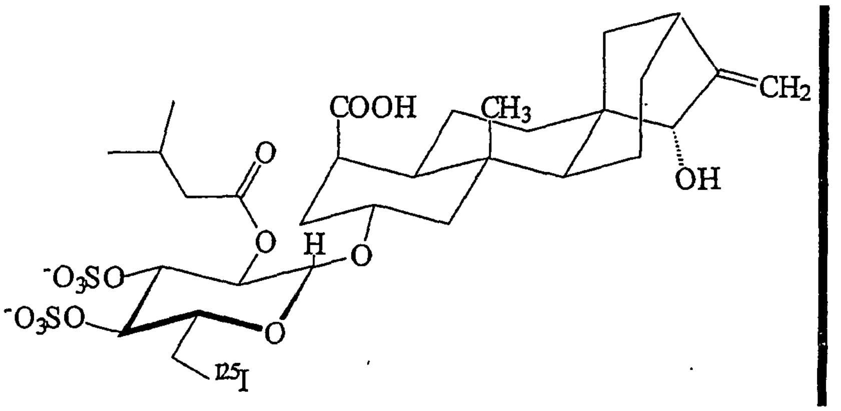

6

Synthesis of Compound 6

Five $\mu$l of 0.2 M sodium phosphate (pH 5) was combined with 21 ul of Na$^{125}$I (9.25 mCi) in its shipping container (specific activity, 2100 Ci/mmol; Amersham, Piscataway, New Jersey). Ten ul (200 $\mu$g, 212 nmol) of compound 2 of Example 1 was added to the mixture. The pH was checked with litmus paper to confirm that it did not rise above pH 5. The mixture was allowed to stand at ambient temperature overnight (17.5 hours) to yield radiolabelled compound 6. (Nonradioactive iodinated ATR derivative, for use as a "cold" competitor in binding studies, may be prepared in the same manner using unlabeled iodine). The iodinated derivative was purified over a C18 analytical column (4 x 6 x 250 mm) (Phenomenex, Torrance, California) using a 25%-55% acetonitrile gradient in running buffer (1% triethylammonium acetate, pH 4.5). A flow rate of 1 ml/min was used to run the gradient over 30 min. The desired product eluted at 25 min. ESI-MS: 835 (M-H), 707 (m-2H-I).

EXAMPLE 9

SYNTHESIS OF REPRESENTATIVE ATR DERIVATIVES

Activation of carboxylic acids with carbonyl diimidazole and their reaction with ATR has been the method of choice for synthesis of various 6'-O-acyl derivatives. The relatively low reactivity of the 6'-hydroxyl of ATR and the presence of an allylic secondary hydroxyl in the aglycon as well as the sulfated glucose moiety, are all factors that have a negative impact on the efficiencies of these acylation reactions. Hence, yields are generally poor and the approach requires a large excess of acylating reagents.

Two strategies for introduction of an amine functionality in the ATR system are described below that permit synthesis of a broader range of ATR derivatives. In the first strategy, as depicted by Scheme 1, displacement of the primary tosylate from compound 2 (Example 1) with azide followed by reduction yields the corresponding 6'-amine (compound 7). Alternatively, the amine group can be introduced as part of a spacer, which permits introduction of more sterically demanding functional moieties. Thus, reacting the 6'-O-succinoyl derivative (compound 8; *see* Brandolin et al., 1974 FEBS Lett. 46:149.) with a monoprotected diamine followed by deprotection affords compound 9 as illustrated by Reaction Scheme 2.

Reaction Scheme 1 Reaction Scheme 2

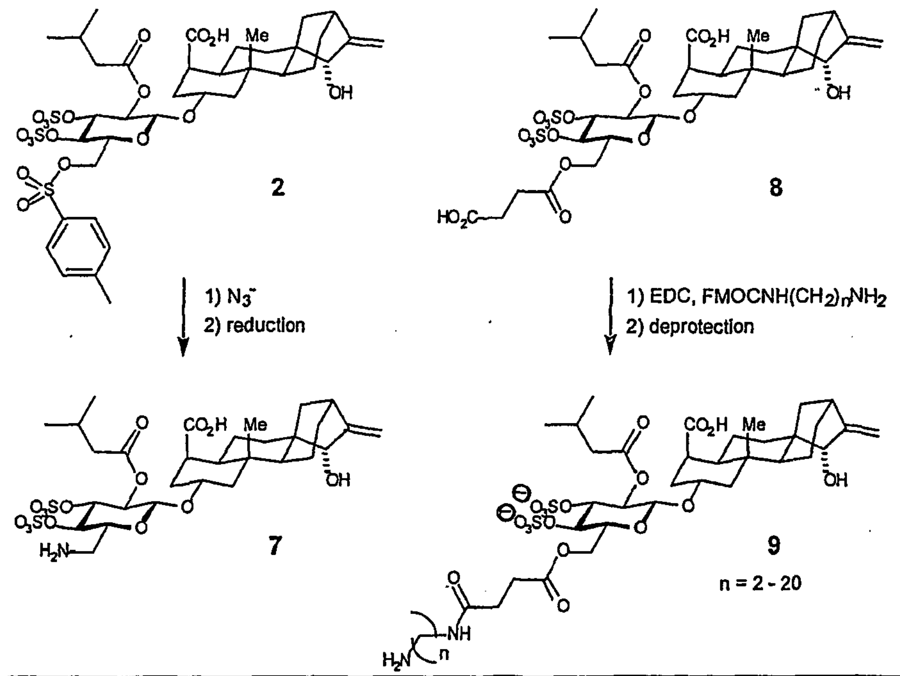


2 8 7 9 n = 2 - 20

The amine-containing ATR derivatives 7 and 9 may then be reacted with a variety of fluorophors and haptens bearing reactive isothiocyanate, N-hydroxysuccinimide ester and anhydride functionalities to yield stable ATR-derivatives having thiourea and amide linkages. Representative ATR derivatives that were prepared include ATR-lanthanide chelating agents (compounds 10, 11, 12, 13 20 and 21) that have utility for time-resolved fluorescence detection of these compounds complexed to $Eu^{3+}$. In addition, ATR was conjugated to cyanine (compounds 14 and 15) and fluorescein analogues (compounds 16 and 17) that are detectable by fluorescence with extremely high sensitivities. Coupling of biotin-NHS ester with the ATR derivatives of compounds 7 and 9 provided ATR-biotin conjugates (compounds 18 and 19) that can be detected with commercially available enzyme-avidin conjugates using colorimetric, fluorescent or chemiluminescent techniques.

More specifically, a solution of compound 2 in DMF was treated with azide ion for 8 hours at 80˚C to give the 6'-azido-ATR, that was purified by silica gel chromatography using a $CH_2Cl_2/CH_3OH$ solvent system supplemented with 1% acetic acid. Staudinger-reduction using 1.5 equivalents of triphenylphosphine in a $THF/H_2O$ mixture for 4 hours at RT afforded the amine of compound 7, that was isolated after silica gel chromatographic purification.

To accommodate more sterically demanding functional moieties, 6'-O-succinoyl-ATR may be condensed with commercially available monoprotected diamines (Calbiochem-Novabiochem Corp, San Diego, CA) to produce ATR-mono-protected amine derivatives. Thus, EDC-mediated coupling of 6'-O-succinayl-ATR in DMF with 1.1 equivalents of mono-protected FMOC diamines yield the amide that was deprotected using piperidine or 1,8-diazabicyclo[5.4.0] undee-7-ene (DBU) in acetonitrile to furnish the ATR derivative of compound 9. The amines were purified by silca gel chromatography as described above.

The ATR-amine derivatives of compounds 7 and 9 were coupled to a variety of fluorophors, chelates and haptens that contained amine-reactive functionalities, such as isothiocyanates, anhydrides and NHS esters in aqueous DMF to generate the ATR derivatives of compounds 10 through 19: These compounds were purified by a combination of silica gel chromatography and preparative reverse phase chromatography on a C-8 column using $CH_3OH/H_2O$ gradient containing 0.1-1% acetic acid.

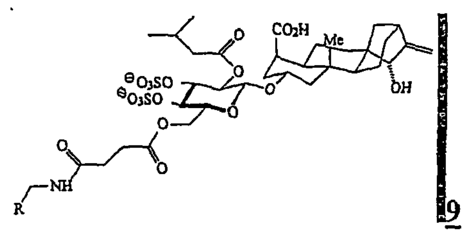

9

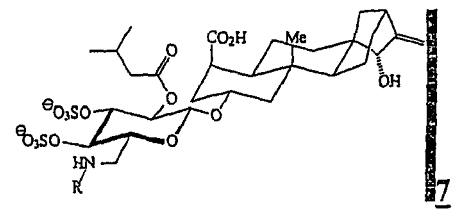

7

R=

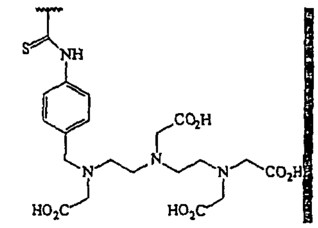

10

R =

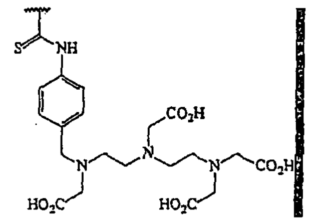

11

R=
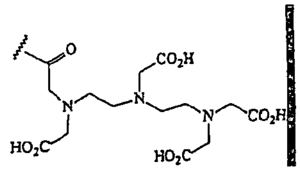
12

R =
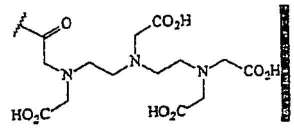
13

R=
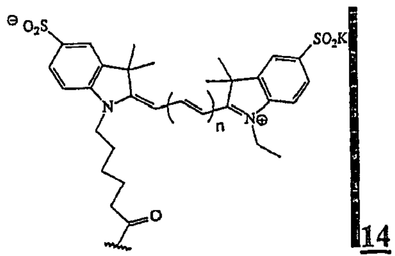
14

R =
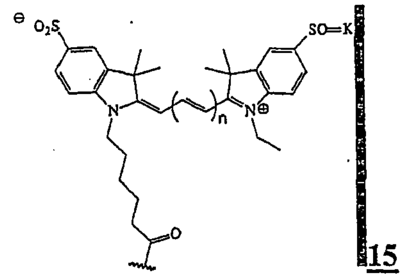
15

R =
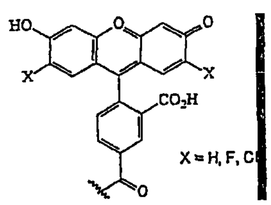
16

R =
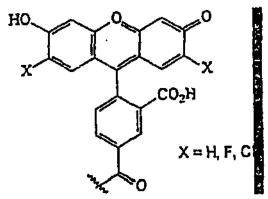
17

R =
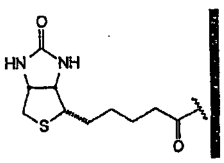
18

R =
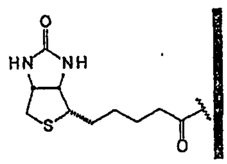
19

R =
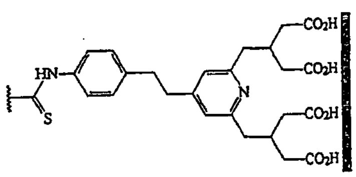
20

R =
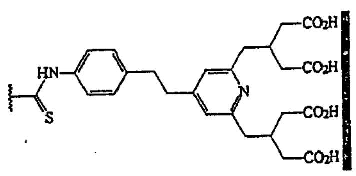
21

EXAMPLE 10

SYNTHESIS OF REPRESENTATIVE ATR DERIVATIVES AND INTERMEDIATES THEREFOR ,

General Procedure for Coupling Atractyloside or Dihydroaractyloside to Organic Acids

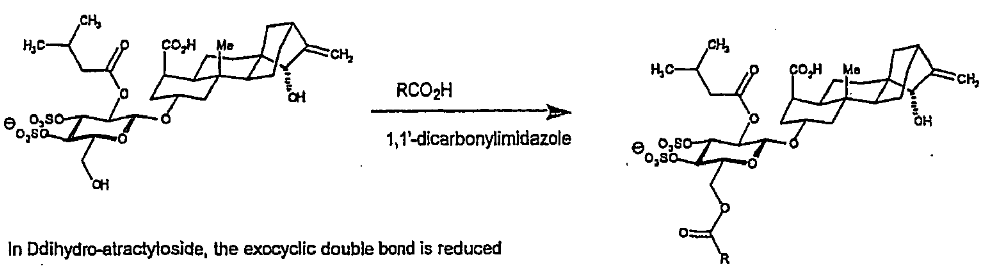


In Ddihydro-atractyloside, the exocyclic double bond is reduced

Carboxylic acid (200 μmol) and 1,1'-carbonyldiimidazole (700 μmol) were dissolved in DMF (2 mL) and stirred for 15 min. To the activated acid, a solution of atractyloside (ATR) (100 μmol, 85 mg) or dihydroatractyloside (100 μmol) in DMF:$H_2O$ (1:2, 6 mL) was added (in 1 mL portions over ca 30 sec). The reaction mixture was stirred at room temperature for 60 min, after which solvents were removed by rotary evaporation on a water bath in which the temperature was kept below 40°C. The residue was stripped of traces of DMF by repeated evaporation of added EtOH:$H_2O$ (1:1, 3 X 20 mL). The residue was then taken up in MeOH: $H_2O$ (1:1,10 mL), sonicated if necessary, and filtered through a 0.45 μm filter. Evaporation, re-dissolution in ~ 1.3 mL buffer, and purification by reverse phase HPLC furnished the desired 6'-O-acyl-ATR in yields ranging from 5-15%.

Reverse Phase HPLC Conditions for Purification of Atractyloside Derivatives

Purification by reverse phase HPLC (RP-HPLC) was performed in a 10 X 250 mm C-8 column, using a gradient of MeOH:AcOH:1 M $NH_4OAc$ (buffer B, 98:1:1) in $H_2O$:AcOH:1 M $NH_4OAc$ (buffer A, 98:1:1). Typically, a gradient of B in A from 50-80% over 30 min was employed. For more lipophilic derivatives, the gradient was from 60-90% or 70-100% B over the same time period.

Synthesis of Dihydroatractyloside

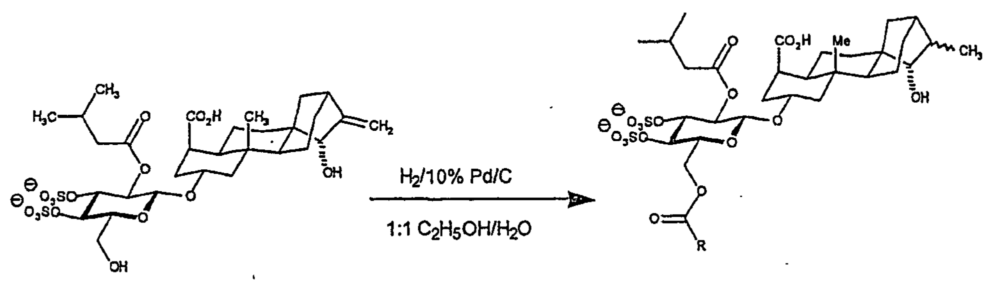


Atractyloside, dipotassium salt (254 mg, 0.3 mmole) in 15 ml of EtOH/$H_2O$ (1:1) was hydrogenated under atmospheric pressure for 3 hours using 51 mg of activated palladium/carbon (10%) as catalyst. The catalyst was removed by filtration through celite and the celite bed was washed with 15 ml of EtOH/ $H_2O$. The filtrates were concentrated by rotary evaporation, followed by drying under high vacuum overnight to provide the product as white solid (236 mg), that was pure by NMR.

6'-Tosyl-Atractyloside

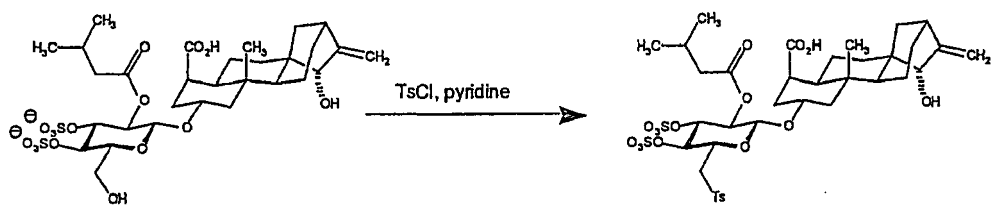


Atractyloside.3$H_2O$ (255 mg. 0.3 mmole)was dried by co-evaporation with dry pyridine (3 X 5 ml) and then was kept under high vacuum for 16 hrs. The dried atractyloside was dissolved in 15 ml of dry pyridine and 114 mg of tosyl chloride (0.6 mmole) was added. The reaction was stirred for 2 hrs at 23°C and then an additional 114 mg of tosyl chloride was added and the reaction was allowed to continue for an additional 1.5 hrs. Methanol (1 ml) was added to the reaction mixture to scavenge excess tosyl chloride and the mixture was stirred for several minutes. The mixture was evaporated to dryness, and residual pyridine was removed by evaporation of added methanol (2 X 30 ml). The crude

product was partially purified by silica gel flash chromatography ($CH_2Cl_2$/MeOH, 3:1 with 1% AcOH). The product containing fractions were dissolved in 3 ml of $H_2O$:MeOH:1M $NH_4OAc$:HOAc (49:49:1:1) and purified by RP-HPLC using a 50%-80% gradient of MeOH:AcOH:1 M NHaOAc (buffer B, 98:1:1) in $H_2O$:AcOH:1 M $NH_4OAc$ (buffer A, 98:1:1). Product containing fractions were pooled, evaporated, and subjected to additional co-evaporation of 3 X 10 ml ofMeOH. Tosyl-atractyloside was obtained as a glassy material weighing 60 mg.

General Procedure for Lactoperoxidase-Catalyzed Iodination of 4-Hydroxyphenyl Derivatives

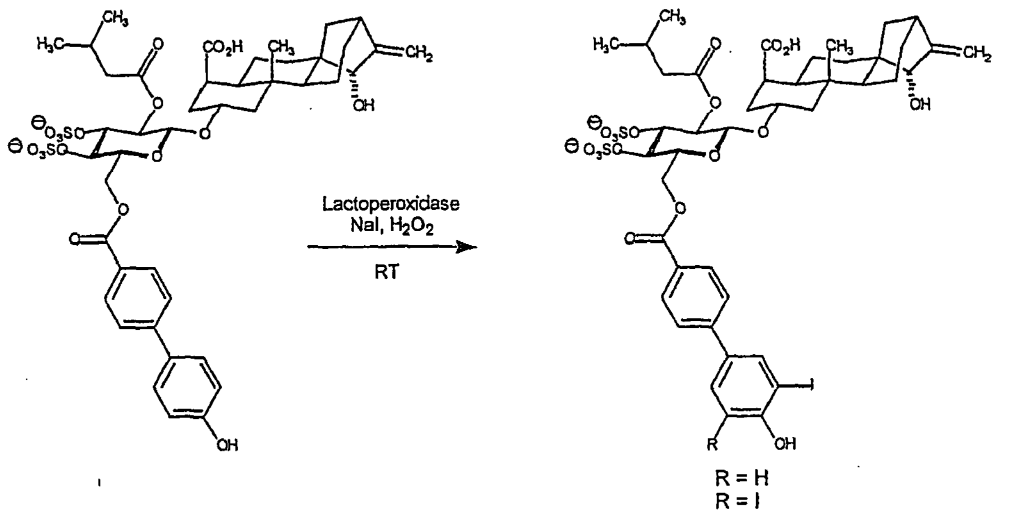


To a solution of 4'-hydroxy-biphenyl-4-carboxylic acid atractylosid-6'-yl ester (1.0 mg, 1.0 mmol) in $H_2O$ (120 μL) were added aqueous solutions of lactoperoxidase (50 μL, 200 IU/mL), NaI (10 μL, 100 mM) and $H_2O_2$ (20 μL, 100 mM). The reaction was left at room temperature for 1h after which it was frozen at -20 °C. The next day (~20 hours later) the reaction mixture was thawed and subjected to RP-HPLC. Three major peaks were eluted and electrospray ionization-mass spectrometic (ESI-MS) analysis confirmed their identity as unreacted starting material, and monoiodinated, and diiodinated atractyloside derivatives.

These conditions can be modified to drive the reaction completely to the mono and di-iodinated forms with additional aliquots of NaI and/or enzyme.

General for Synthesis of Iodophenols

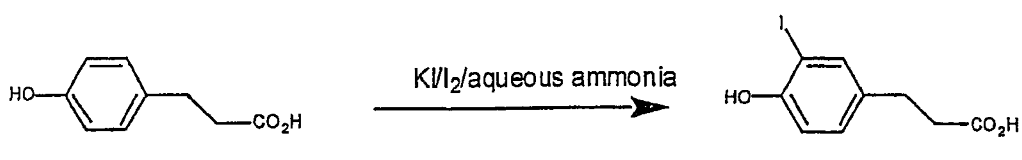


The iodination procedure described in Acta Chem, Scand. 12, 188 (1958) was used for mono-iodination of 3-(4-hydroxyphenyl)propionic acid, 4'-hydroxy-4-biphenylcarboxylic acid and 4-hydroxybenzoic acid. This method is also applicable for the mono-iodination of 3-(4-methoxyphenyl)-propionic acid and di-iodination of 3-(3-iodo-4-hydroxyphenyl)propionic acid.

Thus a solution of KI (1.99 gm, 12 mmole) and iodine (1.22 gm, 4.8 mmole) in 20 ml of $H_2O$ was added in a dropwise fashion to a solution of 3-(4-hydroxyphenyl)propionic acid (0.83 gm, 5 mmole) in 100 ml of concentrated aqueous ammonia solution over 20 min. The reaction mixture was stirred for an additional 40 min and then subjected

to vacuum to remove the ammonia. The mixture was dried further by rotary evaporation to afford an oily residue. The crude material was partitioned between 2M HCl (50 ml) and ether (2 X 50 ml) and the ether layers were combined and concentrated to give a yellowish solid residue. Flash silica gel chromatography using 95:5 $CH_2Cl_2$/MeOH as eluant, concentration of product containing fractions and recrystallization in 1:1 benzene hexane afforded 790 mg of 3-iodo-4-hydroxyphenylpropionic acid.

3-(3,5-Diiodo-4-hydroxyphenyl)propionic acid was prepared in similar fashion using 5.2 equivalents of KI and 2.1 equivalents of $I_2$. Following crystallization from toluene, the di-iodo derivative was obtained in 77% yield.

5-iodo-6-hydroxy-2-naphthoic acid

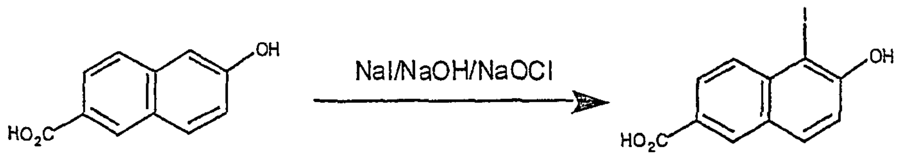


Mono-iodination of 6-hydroxy-2-naphthoic acid and 4-hydroxybenzoic acid was carried out following the procedure of Edgar and Falling (J. Org. Chem. 55, 5287, 1990). Thus, 0.75 gm of (4.34 mmol) was dissolved in 19 ml of MeOH and 1.04 gm of NaI (and 0.27 gm of NaOH was added. The solution was cooled to 0˚C and aqueous NaOCl (4% solution, 12.9 ml) was added dropwise over 75 min. The resulting mixture was stirred for 1 hr at 0˚C and then treated with 7 ml of 10% aqueous sodium metabisulfite. The mixture was adjusted to pH 7 using 5% HCl and extracted with 40 ml of ether. The organic layer was washed with brine and dried over $MgSO_4$. The solution was concentrated to an off-white solid, that was recrystallized from toluene/$CH_3OH$ to provide 0.42 gm of 5-iodo-6-hydroxy-2-naphthoic acid.

Reaction of 6'-Tostylatractyloside with 1,2-ethylenediamine

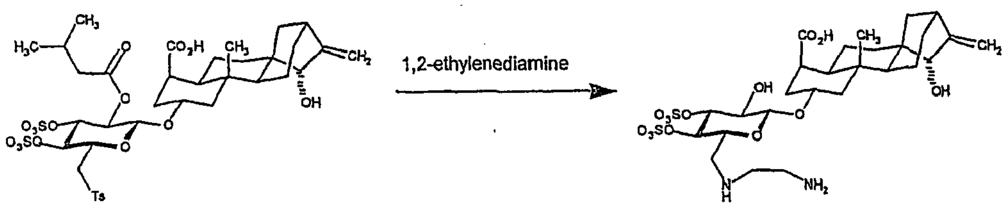


6'-Tosyl-Atractyloside (25 mg) was dissolved in 2 ml of 1,2-ethylenediamine and the mixture was stirred at 23˚C overnight. The 1,2-ethylenediamine was removed in vacuo, the residue was dissolved in MeOH/$H_2O$ (2:1) and 10.6 mg of the product was isolated in by RP-HPLC using the conditions described above. Proton nmr and mass spectra indicate the loss of the isovaleryl group.

Reaction of N-(6-Deoxy-apo-atractylosyl)-ethanediamine with Bolton Hunter Reagent

N-(6-Deoxy-apo-atractylosyl)-ethanediamine (10.6 mg) in 2.75 ml of DMF/DMSO (8:3) was reacted with 60 mg of 4-hydroxyphenylpropionyl-N-hydroxysuccinimidyl ester (Bolton Hunter reagent) at 23˚C for 16 hrs. The solution was diluted with water and purified by RP-HPLC to afford 9.1 mg of the desired compound.

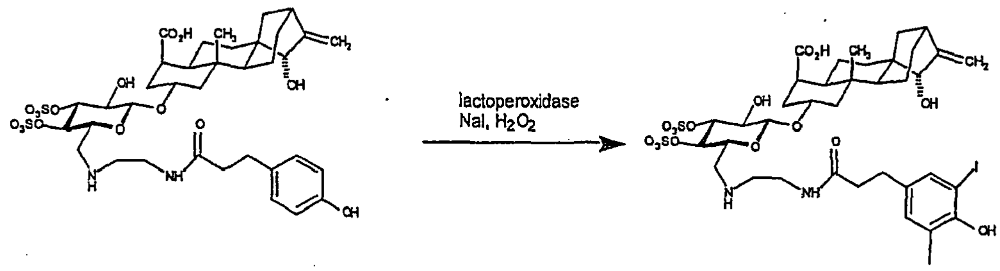


Lactoperoxidase catalyzed iodination of the compound leads to a quantitative conversion to the di-iodo-apo-atractyloside derivative.

Reaction of atractyloside with succinic anhydride

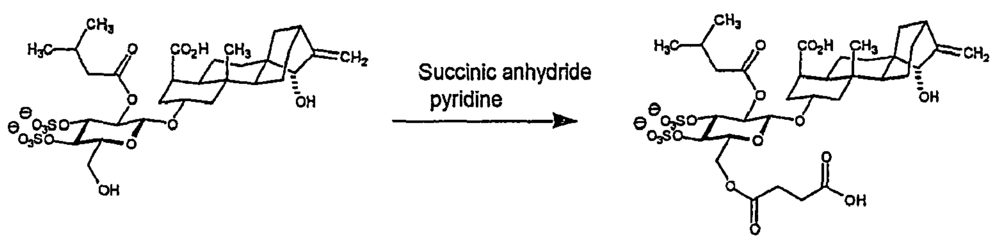


Atractyloside.3$H_2O$ (255 mg. 0.3 mmole)was dried by co-evaporation with dry pyridine (3 X 5 ml) and then was kept under high vacuum for 16 hrs. The dried atractyloside was dissolved in 6 ml of dry pyridine and 60 mg of succinic anhydride (0.6 mmole) was added. The mixture was kept at 80˚C for 30 min, another 60 mg (0.6 mmole) of succinic anhydride was added and the reaction mixture was stirred for an additional 3 hrs. The pyridine was removed in vacuo, and the residue was triturated with 10 ml of MeOH. The 6'-O-succinyl-ATR derivative was collected by filtration as a white solid, washed with MeOH and dried overnight over $P_2O_5$.

Reaction of 6'-O-succinylatractyloside with tyramine and iodination

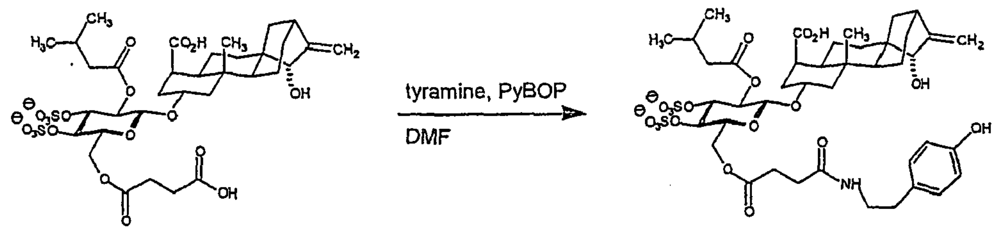


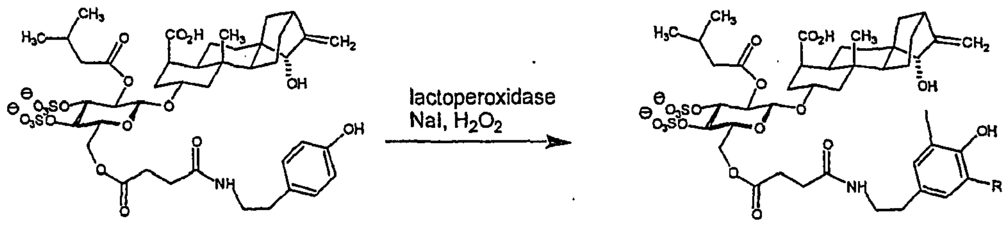


(a) R = H
(b) R = I

6'-O-Succinylatractyloside (85 mg) was dissolved in 2 ml of DMF and 28 mg of tyramine and 100 mg of PyBOP was added. The mixture was stirred at 23°C for 16 hrs. The crude mixture was subjected to RP-HPLC and the desired amide was isolated in 19.7 mg. Lactoperoxidase catalyzed iodination of the product using the standard conditions described above provides the mono and the di-iodinated products.

Synthesis of 4-(4-Hydroxy-3-Methyl)-Butyric Acid

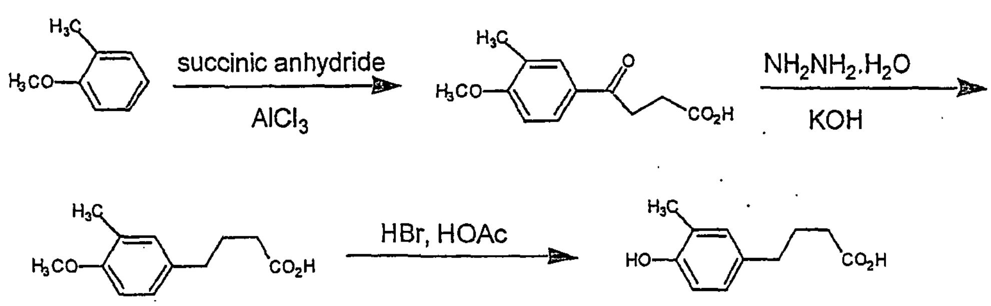


To 4 gm (30 mmol) of AlC13 in 50 ml of 1,2-dichloroethane at 0°C was added 2.7 gm (27 mmol) of succinic anhydride and the mixture was stirred for 20 min. 2-Methylanisole (3.1 ml, 25 mmol) was added, and the reaction mixture was warmed to 23°C and stirred for 12 hrs. The mixture was poured into 300 ml of ice-cold water and the precipitate was filtered off. The precipitate was washed with 2 X 300 ml of water to afford a white solid. The solid material was dried under vacuum to afford 3.51 gm of product that was used in the next reaction.

3-(4-Methoxy-3-methylbenzoyl)propionic acid (4.4 gm, 20 mmol) and 4.49 gm of KOH pellets (80 mmol) were dissolved in 30 ml of ethylene glycol and 3.88 ml of hydrazine hydrate (80 mmol) was added to the stirred solution in four portions. The resulting reaction mixture was heated at 155°C for 24 hrs in an oil bath. After cooling, the reaction mixture was taken up in 100 ml of benzene and washed with 10% aqueous citric acid. The organic layer was washed with another portion of citric acid, dried over anhydrous sodium sulfate, and concentrated in vacuo to afford an oily residue that crystallized upon standing. The material was triturated with hot hexane and the solvent was evaporated off to afford 3.55 gm of a crystalline solid that was homogeneous by silica gel tlc using hexane/ethyl acetate (8:2) as eluting solvent.

4-(4-Methoxy-3-methylphenyl)butyric acid (3.12 gm) was heated in 120 ml of a 1:1 mixture of 48% aqueous HBr/acetic acid at 155°C for 24 hrs. The reaction mixture was cooled to room temperature and was extracted with 200 ml of benzene/ether (1:1). The organic layer was dried over anhydrous magnesium sulfate and concentrated to afford

a light brown solid residue. The reaction products were separated by silica gel flash chromatography using hexane/ethyl acetate (3:1) as eluting solvent to provide 0.86 mg of 4-(4-hydroxy-3-methyl)-butyric acid as a light yellow solid.

Representative Synthesis of Atractyloside Derivatives

To a solution of 3-(4-hydroxyphenyl)propionic acid (HPP) (0.498 g, 3.0 mmole) in 10 ml of anhydrous DMF was added carbonyldiimidazole (0.486 g, 3.0 mmole). The mixture was stirred at room temperature for 30 minutes and added in portions (2 ml/hour) to a solution of atractyloside (ATR) (0.086 g, 0.1 mmole) in I ml of anhydrous DMF. The reaction mixture was stirred at room temperature overnight and quenched with 1 ml of water. The solvent was evaporated under vacuum and the residue was dissolved in ethyl acetate (75 ml) and water (50 ml).. The aqueous layer is separated, extracted with ethyl acetate (3 x 75 ml) and concentrated under vacuum. The residue was dissolved in 1.5 ml of methanol/water(1/1), filtered through a 0.2 mm filter and purified using HPLC with a preparative C-8 column (microsorb, 10 x 250 mm) using a linear gradient elution of 30%-60% solvent B with a flow rate of 2.0 ml/min (solvent A: H2O/HOAc/NH4Oac (1.0M, aq.): {1000/1/1}; solvent B: CH3OH/HOAc/NH4OAc(1.0 M, aq.): {1000/1/1}). The title compound (compound 36 of Example 11 below) was obtained as a white film (6..2 mg).

**EXAMPLE 11**

**FURTHER REPRESENTATIVE ATR DERIVATIVES**

Following the procedures set forth in Example 10, the following ATR derivatives were prepared.

Atractyloside Derivatives

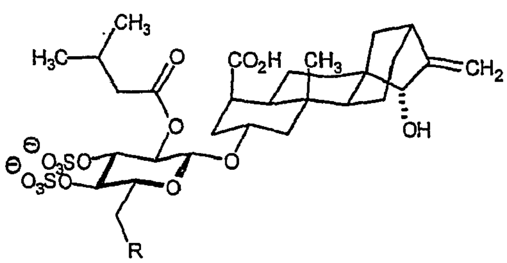

| Cpd | R | MS | NMR |
|---|---|---|---|
| 22 | 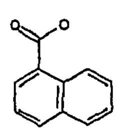 | 879.3 (M-H)$^-$<br>799.3 (M-$SO_3$-H)$^-$ | $^1$H NMR ($CD_3OD$) δ 0.63(s, 3H), 0.95(d, 3H), 0.96(d, 3H), 4.94(s, 1H), 5.09(s, 1H), 7.53(m, 2H), 7.60(m, 1H), 7.93(d, 1H), 8.07(d, 1H), 8.26 (d, 1H), 8.88(d, 1H) |
| 23 | 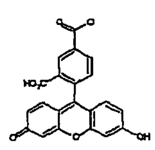 | 1083 (M-H)$^-$<br>1003 (M-$SO_3$-H)$^-$ | $^1$H NMR ($CD_3OD$) δ 0.65(s, 3H), 0.95(d, 3H), 0.96(d, 3H), 5.02(s, 1H), 5.13(s, 1H), 6.61(ddd, 2H), 6.69(d, 2H), 6.86(dd, 2H), 7.34(d, 1H), 8.33 (dd, 1H), 8.67(s, 1H) |
| 24 | 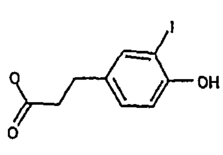 | 999 (M-H)$^-$ | $^1$H NMR ($CD_3OD$) δ 0.94(s, 3H), 0.95(d, 3H), 0.96(d, 3H), 5.05(s, 1H), 5.15(s, 1H), 6.75(d, 1H), 7.04(dd, 1H), 7.52(d,1H) |

(continued)

| Cpd | R | MS | NMR |
|---|---|---|---|
| 25 | 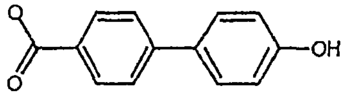 | 921 (M-H)$^-$<br>841 (M-$SO_3$-H)$^-$ | $^1$H NMR ($CD_3OD$) δ 0.75(s, 3H), 0.95(d, 3H), 0.96(d, 3H), 4.97(s, 1H), 5.08(s, 1H), 6.89(d, 2H), 7.52(d, 2H), 7.64(d, 2H), 8.07(d. 2H) |
| 26 | 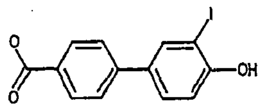 | 1047 (M-H)$^-$<br>967 (M-$SO_3$-H)$^-$ | $^1$H NMR ($CD_3OD$) δ 0.73(s, 3H), 0.95(d, 3H), 0.96(d, 3H), 4.98(s, 1H), 5.09(s, 1H), 6.93(d, 1H), 7.52(dd, 1H), 7.61 (d, 2H), 7.98(d. 1H), 8.08(d, 2H) |
| 27 | 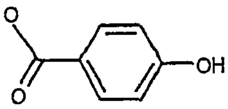 | --- | $^1$H NMR ($CD_3OD$) δ 0.81(s, 3H), 0.95(d, 3H), 0.96(d, 3H), 5.04(s, 1H), 5.15(s, 1H), 6.80(d, 2H), 7.90(d, 2H), 7.64(d, 2H) |
| 28 | 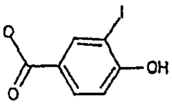 | 1097.1 (M-H)$^-$<br>1017.0 (M-$SO_3$-H)$^-$ | $^1$H NMR ($CD_3OD$) δ 0.83(s, 3H), 0.95(d, 3H), 0.96(d, 3H), 5.03(s, 1H), 5.15(s, 1H), 6.84(d, 1H), 7.88(dd, 1H), 8.33(d, 1H) |
| 29 | 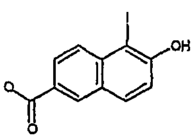 | 1043.2 (M-2H+Na)$^-$<br>1021.2 (M-H)$^-$ | $^1$H NMR ($CD_3OD$) δ 0.66(s, 3H), 0.95(d, 3H), 0.96(d, 3H), 4.93(s, 1H), 5.05(s, 1H), 7.22(d, 1H), 7.87(d, 1H), 8.04(dd, 1H), 8.08(d, 1H), 8.50 (d, 1H) |
| 30 | 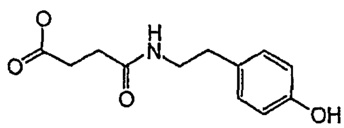 | 944 (M-H)$^-$<br>864 (M-$SO_3$-H)$^-$ | $^1$H NMR ($CD_3OD$) δ 0.97(s, 3H), 0.98 (d, 6H), 5.06(s, 1H), 5.17(s, 1H), 5.70(d, 2H), 7.03(d, 2H). |
| 31 | 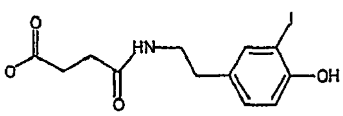 | 1070(M-H)$^-$<br>990(M-$SO_3$-H)$^-$ | - |
| 32 | 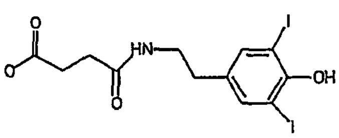 | 1116(M-$SO_3$-H)$^-$ | - |
| 33 | 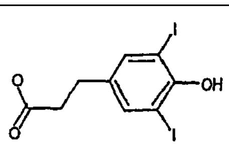 | 1125(M-H)$^-$<br>1147(M-2H+Na)$^-$ | $^1$H NMR ($CD_3OD$) δ 0.95(s, 3H), 0.96(s, 6H), 5.05(s, 1H),5.15(s, 1H), 7.58(s, 2H) |
| 34 | 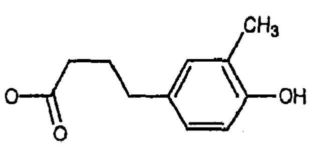 | 923.2 (M-2H+Na)$^-$<br>901.3 (M-H)$^-$ | - |

(continued)

| Cpd | R | MS | NMR |
|---|---|---|---|
| 35 | 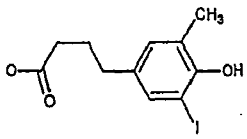 | 1049.3 (M-2H+Na)- | - |
| 36 | 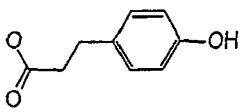 | 873(M-H)-<br>895 (M-2H+Na)- | $^1$H NMR ($CD_3OD$) δ 0.94(s, 3H), 0.96(d, 6H), 5.05(s, 1H), 5.16(s, 1H), |

Dihydroactractyloside Derivatives

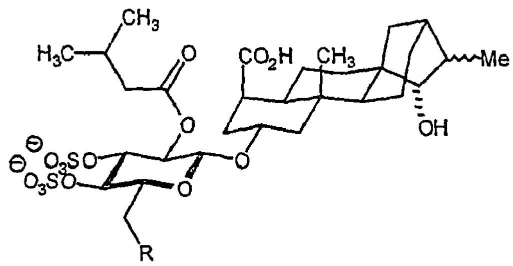

| Cpd | R | MS | NMR |
|---|---|---|---|
| 37 | 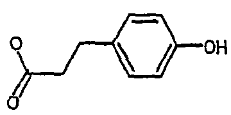 | 897 (M-2H+Na)-<br>875 (M-H)- | $^1$H NMR ($CD_3OD$) δ 0.93(s, 3H), 0.95(d, 3H), 0.96(d, 3H), 1.08(d, 3H), 6.69(d, 2H), 7.02(d, 2H), resonances from alkenic protons absent |
| 38 | 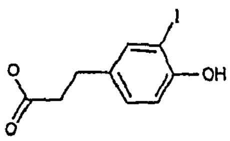 | 1105 (M-2H+Na)-<br>1083 (M-H)- | $^1$H NMR ($CD_3OD$) δ 0.93(s, 3H), 0.95(d, 3H), 0,96(d, 3H), 1.08(d, 3H), 6.74(d, 1H), 7.05(dd, 1H), 7.53(d, 1H), resonances from alkenic protons absent |

Apoactractyloside Derivatives

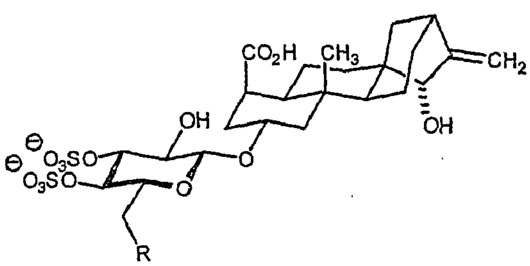

| Cpd | R | MS | NMR |
|---|---|---|---|
| 39 | 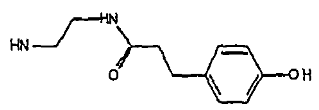 | 831(M-H)$^-$ | $^1H$ NMR ($CD_3OD$) δ 1.00(s, 3H), 5.07(s, 1H), 5.17(s, 1H), 6.69(d, 2H), 7.03(d, 2H) |
| 40 | 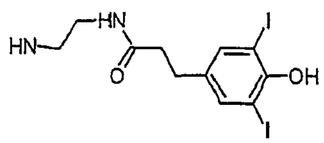 | 1083 (M-H)$^-$ 1105(M-2H+Na)$^-$ | |

EXAMPLE 12

ANT LIGAND BINDING ASSAYS

The atractyloside analogs of the preceding Examples are or were used in pseudo-homologous competition binding assays using *T. ni*/*ANT* mitochondria or bovine mitochondria. Mitochondria from noninfected *T. ni* cells, or *T. ni* cells infected with a baculovirus expressing an ANT isoform (see preceding Examples), are prepared as described in the preceding Examples.

Bovine mitochondria were prepared as follows. Essentially all of the fat and cholesterol in clogged arteries was removed from two bovine hearts which were then cut into 1-inch cubes. The cubes were ground in a meat grinder using the fine setting. Three hundred (300) gm portions of the ground heart were weighed out and, to each was added 400 ml of Isolation Buffer (IB; 250 mM sucrose, 1 mM sodium succinate, 0.2 mM $K^+$ EDTA, 10 mM Tris-base, pH 7.8). (All buffers were filter. sterilized, and column buffers were degassed, and, unless otherwise noted, all steps were carried out at 0 to 4˚C on ice or in pre-cooled rotors and centrifuges.) The preparations were mixed in a blender two times for 15 seconds on high setting and, in between and after blends, the pH was adjusted to 7.8 with 2M Tris-base. The homogenate was centrifuged for 20. minutes at 1,200 x g, and the supernatant was poured through two layers of cheese cloth and adjusted to pH 7.8 with 2M Tris-base. The supernatant was then centrifuged for 30 minutes at 11,000 x g. The supernatant was decanted, and the buff-colored outer pellet was dislodged with about 10 ml of IB and discarded. The brown inner pellet (heavy mitochondria) was resuspended in IB (about 10 ml per pellet). The pellets were homogenized in a glass-teflon homogenizer (2 passes at high drill speed). Samples were combined and centrifuged for 30 minutes at 11,000 x g. The supernatant was decanted, and the pellets were resuspended in 60 ml of IB per 900 gm of ground heart. This centrifugation step was repeated and the pellets were finally resuspended in IB (60 ml per 900 gm of ground heart). One kilogram of beef heart typically yields about one (1) gram of mitochondria.

The mitochondrial preparations are or were divided into aliquots (typically, 50 μl for *T. ni* mitochondria and 20 μl for bovine mitochondria) and then either used directly in assays or flash frozen and stored at -80˚C. Total protein content in the mitochondrial preparations was determined using the enhanced protocol (30 minutes at 60˚C; see http://www.ruf.rice.edu/~bioslabs/methods/protein/BCA.html) of the bicinchoninic acid (BCA) assay (available in kit form from Pierce, Rockford, IL).

In the "Tube Assay," mitochondria (from about 1 to 10 μg of total protein) were resuspended in 100 μl of Tris-KCl buffer with 0.1 % BSA, pH 7.4. $^{125}I$-labeled compound 24 (Example 11) was added to a final concentration of 0.5 nM. When used, competitors were added at these concentration ranges: unlabeled atractyloside or compound 24, final concentration from 5 nM to 10 μM; unlabeled ADP (a lower affinity competitor) was added at a final concentration of 500 nM to 1 mM.

The reaction mixes were incubated on ice for 60 minutes and then pelleted by centrifugation (approximately 16,000 x g) for 11 minutes at 4˚C. Unbound 125 1-compound 24 was removed by aspiration. The pellets were contact-washed with Tris-KCl buffer, pH 7.4, and recentrifuged. The resultant pellets were aspirated and the radioactivity (dpm) in each was determined by gamma counting.

Representative results with bovine mitochondria are shown in Figure 4. The data presented in Figure 4 show that mitochondria (5 μg of protein/tube) from bovine cardiac tissue specifically bind $^{125}I$-compound 24 in a manner that is inhibited by increasing concentrations of unlabeled compound 24 but, as expected, little or no binding is seen when mitochondria are excluded from the reaction mixes.

Figure 5 show competitive inhibition of $^{125}I$- compound 24 binding to mitochondria (1 μg of protein/tube) from bovine cardiac tissue by compound 24 that is not detectably labeled, unlabeled atractyloside (ATR), and unlabeled adenosine diphosphate (ADP). In both instances, ATR and compound 24 yield comparable competition curves, although

ATR appears to have a slightly higher affinity than compound 24. However, both ATR and compound 24 bind with much higher (about 1,000 fold) affinity than the low affinity ANT ligand ADP.

Figure 6 shows competitive inhibition, by unlabeled compound 24 and by bongkrekic acid (BKA), of $^{125}$I-compound 24 binding to mitochondria (1 μg of protein/tube) from beef heart. BKA effectively displaced labeled compound 24, albeit with a slightly lower affinity than unlabeled compound 24. Figure 7 shows competitive inhibition of $^{125}$I-compound 24 binding to beef heart mitochondria by either of the ATR derivatives, compound 23 and compound 28 *(see* Example 11). As shown in Figure 7, ATR exhibited an $IC_{50}$ of approximately 44 nM, compound 23 an $IC_{50}$ of approximately 105 nM, and compound 28 an $IC_{50}$ of approximately 695 nM.

In Figure 8, data are presented depicting competitive inhibition of $^{125}$I-compound 24 binding to beef heart mitochondria by the ATR derivative compound 5 (*see* preceding Examples). As shown in Figure 8, the $IC_{50}$ for compound 5 was approximately 3.3 μM.

Competitive binding assays are also performed using recombinant His-tagged ANT isoforms immobilized on Ni beads instead of mitochondria. To prepare the bead-immobilized ANT protein, mitochondria from *T. ni* cells are infected with a baculovirus expressing an ANT protein and are then solubilized with 0.5% O-glucopyranoside in the presence of 0.5 nM $^{125}$I-compound 24 Ni-agarose beads (Qiagen, Hilden, Germany), and various concentrations of ATR or BKA as unlabeled competitors. After 1 hour at 4˚C, the beads are washed and radioactivity that remained associated with the beads was counted.

EXAMPLE 13

HIGH THROUGHPUT SCREENING ASSAY FOR COMPOUNDS TARGETED TO ANT PROTEINS AND POLYPEPTIDES

The recombinantly produced ANT proteins, ANT fusion proteins and detectably labeled ANT ligands described herein are incorporated into automated assay systems. Such automated systems are useful for high throughput screening (HTS) of candidate ANT-binding compounds or chemical libraries comprising such compounds. Such compounds may be further characterized and developed as drug candidates and drugs useful for preventing, treating or curing diseases or disorders resulting from the overexpression or dysfunction of one or more ANT proteins or from the overexpression or dysfunction of a factor that positively regulates or stimulates ANT proteins.

A preferred element of many automated assay systems is the incorporation of a target molecule (in the present instance, an ANT protein) into a 96-well plate. This format is readily adaptable for use in a variety of automated label detection systems. For HTS assays, robotic label detection systems are preferred.

In control experiments, unlabeled atractyloside (ATR; Sigma) is used as a 'mock' drug at a concentration of from about 1 to about 10,000 nM. That is, unlabeled ATR is used to displace a labeled atractyloside derivative (e.g., $^{125}$I-ATR). Unlabeled ATR thus acts as a positive control for an HTS in which various compounds are screened for their ability to displace a labeled ANT ligand.

As an example of the automated label detection systems used in the HTS assays of the Example, when the detectably labeled ANT ligand of the assay is $^{125}$I-ATR, an automatic gamma counter is used. Alternatively, $^{125}$I-ATR can be used in scintillation proximity assays (SPA). For example, an ANT protein or fusion protein is contacted with ScintiStrip 96-well plates (EG&G Wallac). The polystyrene of these plates contains a scintillating agent that emits beta radiation when excited by a gamma emitter in close proximity thereto. The beta radiation is then detected by any appropriate automatic beta counter. When fluorescent ANT ligands are used in the HTS assay, an automatic fluorescence counter is used and may be, for example, a FLUOROCOUNT™ Counter (Packard Instrument Company, Meriden, CT).

EXAMPLE 14

DETECTION AND QUANTIFICATION OF LEVELS AND ACTIVITY OF ANT PROTEINS

The compositions and methods described herein can be used by one skilled in the art to determine the amounts (relative and absolute) of ANT protein and ANT activity *in toto,* and/or the amounts of specific ANT isoforms, in different cell lines, organisms, tissues, organs, and the like, at different stages of development or differentiation of such.

For example, the compositions and methods of the present disclosure were used to examine the levels and activities of ANT proteins in SH-SY5Y cells, a useful and generally accepted cellular neuronal cell that can be induced to undergo differentiation. Mitochondrially-enriched lysates from bovine cardiac tissue and from cultured SH-SY5Y cells were probed in western analyses (as described in the preceding Examples), and the results are shown in Figure 9. The results shown on the right side of Figure 9 demonstrate that lysates from SH-SY5Y cells contain roughly the same amount of the mitochondrial protein VDAC. In contrast, the left side of Figure 9 shows the results from a western where the bovine cardiac and SH-SY5Y extracts were probed with the Pan ANT antibody described above. The amount of

ANT in SH-SYSY cells is undetectable in this analysis, even though the results with anti-VDAC demonstrates that at least one mitochondrial protein is present in roughly normal amounts in SH-SY5Y cells. This result suggests that the low amount of ANT proteins in the SH-SY5Y preparations is not due to some failure to isolate equivalent amounts of mitochondrial protein from the cells.

ANT ligand-binding activity in SH-SY5Y cells was examined as follows. Carboxyactractyloside (C-ATR; Calbiochem) is a compound that is structurally related to actractyloside. However, unlike actractyloside, C-ATR binds irreversibly to ANT. C-ATR binding curves for isolated mitochondria from SH-SY5Y cells and bovine cardiac tissue are shown in Figure 10. As is readily seen, SH-SY5Y cells have little detectable C-ATR binding capacity, a result that is congruent with these cells having relatively slight amounts of ANT. In contrast, bovine cardiac tissue had a binding curve expected for ANT-containing mitochondria.

In another experiment, actractyloside specific binding (as measured by the above competitive assays using an $^{125}$I-labeled actractyloside-derived ligand) in isolated SH-SY5Y mitochondria and compared to results from the same assay using isolated mitochondria from bovine cardiac tissue. The results are shown in Figure 11. As in the C-ATR binding experiment (Figure 10), bovine cardiac mitochondria displayed a dose-dependent capacity for actractyloside specific binding (the righthand bars for each protein concentration correspond to the results with bovine mitochondria at that concentration). In contrast, SH-SY5Y mitochondria exhibited a slight capacity for actractyloside specific binding, even at high concentrations of protein (the lefthand bars for each protein concentration correspond to the results with SH-SY5Y mitochondria at that concentration).

EXAMPLE 15

HUMAN ANT EXPRESSION CONTROLLED BY A REGULATED PROMOTER IN MAMMALIAN CELLS

This example describes human ANT expression using a recombinant expression construct comprising a regulated promoter operably linked to a nucleic acid encoding an ANT polypeptide. Standard molecular biology reagents and methodologies were used as known in the art and as described, for example, in Ausubel et al. (Current Protocols in Molecular Biology, Greene Publishing, 1987); and in Sambrook et al. (Molecular Cloning: A Laboratory Manual, Cold Spring Harbor Press, 1989).

Preparation of regulated recombinant ANT expression constructs and Tet$^R$ host cells. Human ANT cDNAs were amplified by PCR using human brain RNA as a template as described in Example 1, except the following primers were used wherein underlined nucleotides indicate sequences complementary to the 5'-ends and 3'-ends of the ANT cDNAs and double-underlined nucleotides indicate recognition sequences for the restriction enzymes Asp718 (recognition sequence: 5'-GGTACC) and ApaI (recognition sequence 5'- GGGCCC):
For human ANT-1, the following primers were used:

Forward (sense):
5'-TTATATGGTACCATGGGTGATCACGCTTGGAGCTTCCTAAAG (SEQ ID NO:41);
and
Reverse (antisense):
5'-TATATAGGGCCCTTAGACATATTTT TTGATCTCA TCC AAC (SEQ ID NO:42).

For human ANT-2, the following sequences were used:

Forward (sense):
TTATATGGTACCATGACAGATGCCGCTGTGTCCTTCGCCAAG (SEQ ID NO:43);
and
Reverse (antisense):
TATATAGGGCCCTTATGTGTACTTCTTGATTTCATCATACAAG (SEQ ID NO:44).

For human ANT-3, the following sequences were used:

Forward (sense):
5'-TTATATGGTACCATGACGGAACAGGCCATCTCCTTCGCCAAA (SEQ ID NO:45);
and
Reverse (antisense):
TATATAGGGCCCTTAGATCACCTTCTTGAGCTCGTCGTACAGG (SEQ ID NO:46).

PCR amplification was performed using reagents and buffers supplied in an Expand™ Long PCR reagent kit

(Roche Molecular Biochemicals, Indianapolis, IN), according to the manufacturer's instructions. PCR products were digested with restriction endonucleases Asp718 and ApaI (both enzymes from New England Biolabs, Inc., Beverly, MA) according to the manufacturer's recommendations and using manufacturer-supplied reaction buffers. Restricted cDNAs were purified by horizontal agarose gel electrophoresis and band extraction using the UltraClean™ GelSpin kit (Mo Bio Laboratories, Inc., Solana Beach, CA) as instructed by the supplier.

Purified, restricted cDNAs encoding ANT-1, ANT-2 and ANT-3 were separately ligated into the expression vector pcDNA4/TO (Invitrogen, Carlsbad, CA). As provided by the supplier, the vector included the following elements operably linked in a 5' to 3' orientation as follows: the strong CMV promoter, tandem tetracycline ("tet")-operator regions (providing a binding site for a tet-repressor, which blocks transcription), a multiple cloning site, and a poly-adenylation region. Plasmid pcDNA4/TO DNA was prepared by digestion with the restriction endonucleases Asp718 and ApaI according to the manufacturer's instructions, resolved by horizontal agarose gel electrophoresis and recovered following band extraction using the UltraClean™ GelSpin kit (Mo Bio laboratories). Purified, restricted ANT cDNAs were ligated into the linearized, restricted plasmid expression vector DNA using T4 DNA ligase (New England Biolabs, Beverly, MA) with the manufacturer's reaction buffer and according to the manufacturer's instructions. The resulting constructs contained the human ANT coding sequences under the control of a tetracycline-regulated promoter.

SH-SY5Y neuroblastoma cells were propagated and maintained according to the supplier's recommendations (ATCC, Manassas, VA). As described in the preceding Example (Figures 10 and 11), based on detectable binding of ANT ligands to mitochondria isolated from SH-SY5Y cells, these cells exhibited extremely low levels of expression of endogenous ANT. Hence, these are suitable host cells for a recombinant expression construct comprising a regulated promoter operably linked to a nucleic acid encoding an ANT polypeptide, for example, pcDNA4/TO encoding an ANT polypeptide that can be controllably expressed in a tetracycline-inducible manner. The tetracycline repressor was stably integrated into the cells using the pcDNA6/TR vector (Invitrogen, Carlsbad, CA) and blasticidin selection according to the instructions accompanying the vector.

Transformation and transfection. The pcDNA4/TO-ANT cDNA ligation mixtures are delivered into competent *rec*A1 *hsd*R *end*A1 *E. coli* cells, for transformation by heat shock. Single colonies arising from the transformation plated onto LB-agar containing 50 μg/ml ampicillin are grown in LB-broth supplemented with 50 μg/ml ampicillin, and plasmid DNA is isolated according to standard protocols as described by Sambrook *et al.,* (1989). The presence in the expression constructs of the recombinant nucleotide sequences encoding ANT-1, ANT-2 and ANT-3 is confirmed by standard methods (*e.g.,* nucleotide sequencing). $Tet^R$ SH-SY5Y clones are then stably transfected with the modified prDNA4/TO vectors (Invitrogen) into the multiple cloning site of which the coding sequence for human ANT-1, ANT-2 or ANT-3 amplified by PCR as described above, has been ligated. Colonies are selected for zeocin resistance and individual clonal populations are isolated. Tetracycline dosages are titrated on the transfected cells and human ANT expression is monitored by analysis of transcription, by functional or phenotypic characterization of the cells, and/or by determination of ANT ligand binding to ANT in intact or non-intact (*e.g.*, permeabilized) cells and/or in isolated mitochondria.

EXAMPLE 16

EXPRESSION AND LOCALIZATION TO MITOCHONDRIA OF ANT ISOFORMS IN INSECT CELLS

This example describes recombinant expression of ANT isoforms that localize to mitochondria in *T. ni* cells and that exhibit ANT ligand binding. Materials and methods were essentially as described above in Examples 4, 12 and 15 except as otherwise noted. The coding region for human ANT-1 was amplified by PCR from human brain cDNA (Clontech; Palo Alto, CA), using the following forward and reverse primers complementary to the published sequence for human ANT-1:

Forward primer:
5'- TATATAGGGCCCTTAGATCACCTTCTTGAGCTCGTCGTACAGG' (SEQ ID NO:47); and
reverse primer:
5'- TATATAGGGCCCTTAGACATATTTTTTGATCTCATCCAAC-3'. (SEQ ID NO:48).

Underlined regions correspond to introduced restriction sites. An extra nucleotide was included 5' to the ATG start sequence of the ANT to assure the construct would be in-frame with the selected expression vector. Electrophoresis of the PCR reaction products demonstrated a single amplicon of the predicted size. The PCR product was ligated into the pBlueBacHis2 transfer vector "B" version, (Invitrogen; Carlsbad, CA), that had been linearized with Xho I and Kpn I restriction endonucleases (New England Biolabs; Beverly, MA).

Competent E. *coli* cells (TOP10 F', Invitrogen) were transformed with the ligation mixture by heat-shock and the resultant transformation was plated on LB plates containing 100μg/ml ampicillin for selection. Single colonies were selected and grown in LB broth containing 100 μg/ml ampicillin, and plasmid DNA was isolated using a QIAFilter™

plasmid purification kit (Qiagen; Valencia, CA). The in-frame nucleotide sequence of the insert was determined using a forward primer to the pBlueBacHis2 polyhedrin promoter and the corresponding baculovirus reverse primer to the same vector. The DNA sequence of the insert was confirmed to be human ANT-1, by reference to the published sequence for the human ANT-1 isoform.

For expression and detection of ectopically expressed human ANT-1, purified pBlueBacHis2/ANT-1 plasmid DNA was supplied to the Invitrogen Corporation (Carlsbad, CA), for production of high titer stock via the Bac-N-Blue™ baculovirus expression system. The fusion protein resulting from baculovirus infection with pBlueBacHis2/ANT-1 comprised a six tandem histidine residue repeat at the amino terminus followed by an enterokinase recognition site, and the in-frame human ANT-1 isoform was verified by western blot analysis (described below). High titer viral stocks were prepared and provided to Pharmingen, Inc. (San Diego, CA.) for infection into Sf9 and *Trichoplusia ni* (*T. ni)* cell lines. The resulting *T. ni* cell line expressing human ANT-1 was designated Tn/ANT-1.

Aliquots of frozen Tn/ANT-1 insect cells were resuspended in mannitol-sucrose buffer (MSB; 210 mM mannitol, 70 mM sucrose, 5 mM HEPES, 5 mM EGTA, 5mM glutamate, 5 mM malate, 0.1 mM PMSF, 1 μg/ml leupeptin, 1 μg / ml pepstatin, I μg/ml aprotinin, pH 7.4), and subjected to three freeze-thaw cycles. The lysates were centrifuged at 1,200 x g at 4 °C to pellet cellular debris, and supernatants were removed and held on ice. Pellets were resuspended in MSB and again cleared by centrifugation. The pellets remaining following these two centrifugation steps ("plasma membrane fraction") contained primarily plasma membranes, but also nuclei, and a small component of unbroken cells. Both supernatants were pooled and used for preparation of a mitochondrial assay fraction. Mitochondria were collected by subjecting the pooled supernatants to an additional 20,000 x g centrifugation step. Equivalent amounts (on the basis of quantitative protein assays) of Tn/ANT-1 cell lysate, Tn/ANT-1 mitochondrial fraction, and mitochondria purified as described above from the SH-SY5Y line (as a positive control for human ANT expression) were resolved by SDS-PAGE. Separated proteins were electrophoretically transferred to a nitrocellulose membrane and blocked overnight in western blot immunostaining buffer (Tris/NaCl) containing 5% BSA/ 3% nonfat dry milk and subsequently probed with the anti-(pan)ANT antibody described in Example 3 (elicited following immunization of rabbits with a peptide corresponding to amino acid residues 257-271 of human ANT-3) diluted in the same buffer. Detection of bound primary antibody was accomplished with a goat anti-mouse secondary antibody conjugated to horseradish peroxidase (HRP; Amersham-Pharmacia, Piscataway, NJ), followed by immersion in ECL™ substrate (Amersham) and detection of chemiluminescent signal according to the supplier's instructions. Figure 12 shows detection of the His-tagged ANT-1 fusion protein in Tn/ANT1 cell lysates and its enrichment in mitochondria isolated from Tn/ANT1 cells.

An assay to measure displacement by BKA of $^{125}$I-labeled compound 24 (ATR-ligand) binding to recombinantly expressed human ANT-1 present on isolated Tn/ANT1 mitochondria was also performed, according to the procedure described in Example 12; the results are shown in Figure 13.

From the foregoing, it will be appreciated that, although specific embodiments of the invention have been described herein for purposes of illustration, various modifications may be made without deviating from the spirit and scope of the invention. Accordingly, the invention is not limited except as by the appended claims. The present invention will now be further described with reference to the accompanying numbered clauses:

1. A recombinant expression construct comprising at least one regulated promoter operably linked to a first nucleic acid encoding an adenine nucleotide translocator polypeptide.

2. The expression construct of clause further comprising at least one additional nucleic acid sequence that regulates transcription.

3. The expression construct of clause 2 wherein the additional nucleic acid sequence that regulates transcription encodes a repressor of said regulated promoter.

4. The expression construct of clause 1 wherein the adenine nucleotide translocator polypeptide comprises an adenine nucleotide translocator polypeptide from a mammal.

5. The expression construct of clause 4 wherein the adenine nucleotide translocator polypeptide is ANT1.

6. The expression construct of clause 4 wherein the adenine nucleotide translocator polypeptide is ANT2.

7. The expression construct of clause 4 wherein the human adenine nucleotide translocator polypeptide is ANT3.

8. The expression construct of clause 4 wherein the mammal is selected from the group consisting of a rat, a mouse, a bovine and a human.

9. The expression construct of clause 8 wherein the polypeptide product of said second nucleic acid sequence is an enzyme.

10. The expression construct of clause 8 wherein said fusion protein localizes to membranes.

11. The expression construct of clause 10 wherein said membranes are mitochondrial membranes.

12. An expression construct according to clause; 1 wherein the adenine nucleotide translocator polypeptide is expressed as a fusion protein with at least one product of a second nucleic acid sequence encoding a polypeptide cleavable by a protease, said adenine nucleotide translocator polypeptide being separable from the fusion protein by cleavage with the protease.

13. A host cell comprising a recombinant expression construct according to clause 1.

14. A host cell according to clause 13 wherein the host cell is a prokaryotic cell.

15. A host cell according to clause 13 wherein the host cell is a eukaryotic cell.

16. The host cell of clause 15 wherein the eukaryotic cell is selected from the group consisting of a yeast cell, an insect cell and a mammalian cell.

17. The host cell of clause 16 wherein the insect cell is selected from the group consisting of an Sf9 cell and a *Trichoplusia ni* cell.

18. A host cell according to clause 13 that lacks at least one isoform of an endogenous adenine nucleotide translocator.

19. A host cell according to clause 13 in which expression of at least one gene encoding an endogenous adenine nucleotide translocator isoform is substantially impaired.

20. A recombinant expression construct comprising at least one promoter operably linked to a nucleic acid molecule comprising a first nucleic acid sequence and a second nucleic acid sequence, said first nucleic acid sequence encoding an animal adenine nucleotide translocator polypeptide wherein the adenine nucleotide translocator polypeptide is expressed as a fusion protein with a polypeptide product of said second nucleic acid sequence.

21. The expression construct of clause 20 wherein the polypeptide product of said second nucleic acid sequence is an enzyme.

22. The expression construct of clause 20 wherein said fusion protein localizes to membranes.

23. The expression construct of clause 22 wherein said membranes are mitochondrial membranes.

24. The expression construct of clause 20 further comprising at least one additional nucleic acid sequence that regulates transcription.

25. The expression construct of clause 24 wherein the additional nucleic acid sequence that regulates transcription encodes a repressor of said promoter.

26. The expression construct of clause 20 wherein the adenine nucleotide translocator polypeptide comprises an adenine nucleotide translocator polypeptide from a mammal.

27. The expression construct of clause 26 wherein the human adenine nucleotide translocator polypeptide is ANT1.

28. The expression construct of clause 26 wherein the human adenine nucleotide translocator polypeptide is ANT2.

29. The expression construct of clause26 wherein the human adenine nucleotide translocator polypeptide is ANT3.

30. The expression construct of clause: 26 wherein the adenine nucleotide translocator polypeptide is ANT1.

31. The expression construct of clause 26 wherein the adenine nucleotide translocator polypeptide is ANT2.

32. The expression construct of clause 26 wherein said mammal is selected from the group consisting of a rat, a mouse, a bovine and a human.

33. A host cell according to clause 31 wherein the host cell is a eukaryotic cell.

34. The host cell of clause 33 wherein the eukaryotic cell is selected from the group consisting of a yeast cell, an insect cell and a mammalian cell.

35. The host cell of clause 34 wherein the insect cell is selected from the group consisting of an Sf9 cell and a *Trichoplusia ni* cell.

36. A host cell according to clause 20 that lacks at least one isoform of an endogenous adenine nucleotide translocator.

37. A host cell according to clause 20 in which expression of at least one gene encoding an endogenous adenine nucleotide translocator isoform is substantially impaired.

38. A recombinant expression construct according to either clause 1 or clause 20 wherein the expression construct is a recombinant viral expression construct.

39. A method of producing a recombinant adenine nucleotide translocator polypeptide, comprising:

culturing a host cell comprising a recombinant expression construct comprising at least one regulated promoter operably linked to a first nucleic acid encoding an adenine nucleotide translocator polypeptide.

40. A method of producing a recombinant adenine nucleotide translocator polypeptide, comprising:

culturing a host cell comprising a recombinant expression construct comprising at least one promoter operably linked to a nucleic acid molecule comprising a first nucleic acid sequence and a second nucleic acid sequence, said first nucleic acid sequence encoding an animal adenine nucleotide translocator polypeptide wherein the adenine nucleotide translocator polypeptide is expressed as a fusion protein with a polypeptide product of said second nucleic acid sequence.

41. A method of producing a recombinant adenine nucleotide translocator polypeptide, comprising:

culturing a host cell infected with the recombinant viral expression construct of clause 38.

42. An ANT polypeptide produced by the method of any one of clauses 39-41.

43. An isolated adenine nucleotide translocator isoform polypeptide.

44. The isolated polypeptide of clause 43 wherein the adenine nucleotide translocator isoform polypeptide is selected from the group consisting of (a) recombinant ANT1 or a variant or fragment thereof, and (b) recombinant ANT2 or a variant or fragment thereof.

45. The isolated polypeptide of clause 43 wherein the-adenine nucleotide translocator isoform polypeptide is derived from a mammal.

46. The isolated polypeptide of clause 45 wherein the mammal is selected from the group consisting of a rat, a mouse, a bovine and a human.

47. An isolated adenine nucleotide translocator isoform fusion protein comprising an adenine translocator isoform polypeptide fused to at least one additional polypeptide sequence.

48. The fusion protein of clause 47 wherein said one additional polypeptide sequence is an enzyme sequence or a variant or fragment thereof.

49. The fusion protein of clause 47 wherein said fusion protein localizes to membranes.

50. The fusion protein of clause 49 wherein said membranes are mitochondrial membranes.

51. An isolated human adenine nucleotide translocator fusion protein comprising an adenine translocator polypeptide fused to at least one additional polypeptide sequence cleavable by a protease, said adenine nucleotide translocator polypeptide being separable from the fusion protein by cleavage with the protease.

52. An isolated adenine nucleotide translocator fusion protein comprising a first polypeptide that is an animal adenine translocator polypeptide fused to at least one additional polypeptide sequence.

53. The fusion protein of clause. 52 wherein said one additional polypeptide sequence is an enzyme sequence or a variant or fragment thereof.

54. A fusion protein according to clause 52 that localizes to membranes.

55. A fusion protein according to clause 54 wherein said membranes are mitochondrial membranes.

56. An isolated recombinant animal adenine nucleotide translocator fusion protein comprising an adenine translocator polypeptide fused to at least one additional polypeptide sequence cleavable by a protease, said adenine nucleotide translocator polypeptide being separable from the fusion protein by cleavage with the protease.

57. The fusion protein of either clause 47 or clause 52 wherein the additional polypeptide sequence is a polypeptide having affinity for a ligand.

58. A method .for determining the presence of an ANT polypeptide in a biological sample comprising:

contacting a biological sample suspected of containing an ANT polypeptide with an ANT ligand under conditions and for a time sufficient to allow binding of the ANT ligand to an ANT polypeptide; and
detecting the binding of the ANT ligand to an ANT polypeptide, and therefrom determining the presence of an ANT polypeptide in said biological sample.

59. The method of clause 58 wherein the adenine nucleotide translocator polypeptide comprises a adenine nucleotide translocator polypeptide from a mammal.

60. The method of clause 59 wherein the adenine nucleotide translocator polypeptide is ANT1.

61. The method of clause 59 wherein the adenine nucleotide translocator polypeptide is ANT2.

62. The method of clause 59 wherein the adenine nucleotide translocator polypeptide is ANT3.

63. The method of clause 59 wherein the adenine nucleotide translocator polypeptide is ANT1 or ANT2.

64. A method for determining the presence of an ANT polypeptide in a biological sample comprising:

contacting a biological sample suspected of containing an ANT polypeptide with an ANT antibody under conditions and for a time sufficient to allow binding of the ANT antibody to an ANT polypeptide; and
detecting the binding of the ANT antibody, and therefrom determining the presence of an ANT polypeptide in said biological sample.

65. The method of clause 61 wherein the ANT polypeptide is an ANT isoform, and the ANT antibody is specific for said ANT isoform.

66. The method of clause 65 wherein the radiolabeled substituent is selected from the group consisting of $^{125}I$, $^{131}I$, $^{3}H$, $^{14}C$ and $^{35}S$.

67. The method of clause 64 wherein the detectable atractyloside derivative comprises a fluorescent substituent.

68. The method of clause 67 wherein the ANT ligand further comprises a $Eu^{3+}$ atom complexed to the atractyloside derivative.

69. The method of clause 64 wherein the detectable atractyloside derivative comprises covalently bound biotin.

70. The method of clause 63 wherein the atractyloside molecule is substituted at 6' hydroxyl with an amine or an amine containing functionality to form an amine modified atractyloside derivative.

71. The method of any one of clauses 63-70 wherein the atractyloside molecule is a carboxyatractyloside molecule that is substituted at 6' hydroxyl to form an atractyloside derivative that is a carboxyatractyloside derivative.

72. A method for isolating ANT from a biological sample, comprising:

contacting a biological sample suspected of containing an ANT polypeptide with an ANT ligand under conditions and for a time sufficient to allow binding of the ANT ligand to an ANT polypeptide; and
recovering the ANT polypeptide, and thereby isolating ANT from a biological sample.

73. The method of clause 72 wherein the ANT ligand is covalently bound to a solid phase.

74. The method of clause 72 wherein the ANT ligand is non-covalently bound to a solid phase.

75. A method for identifying an agent that binds to an ANT polypeptide, comprising:

contacting a candidate agent with a host cell expressing at least one recombinant ANT polypeptide under conditions and for a time sufficient to permit binding of the agent to said recombinant ANT polypeptide; and
detecting binding of said agent to the recombinant ANT.

76. The method of clause 75 wherein the host cell is a prokaryotic cell.

77. The method of clause 76 wherein the prokaryotic cell is an *E. coli* cell.

78. The method of clause 75 wherein the host cell is a eukaryotic cell.

79. The method of clause 78 wherein the eukaryotic cell is selected from the group consisting of a yeast cell, an insect cell and a mammalian cell.

80. The method of clause 79 wherein the insect cell is selected from the group consisting of an Sf9 cell and a *Trichoplusia ni* cell.

81. The method of any one of clauses 75-80 wherein the host cell lacks at least one isoform of an endogenous adenine nucleotide translocator.

82. The method of any one of clauses 75-80 wherein host cell expression of at least one gene encoding an endogenous adenine nucleotide translocator isoform is substantially impaired.

83. A method for identifying an agent that binds to an ANT polypeptide, comprising:

contacting a candidate agent with a biological sample containing at least one recombinant ANT polypeptide under conditions and for a time sufficient to permit binding of the agent to said ANT polypeptide; and
detecting binding of said agent to the recombinant ANT polypeptide.

84. A method for identifying an agent that interacts with an ANT polypeptide comprising:

contacting a biological sample containing recombinant ANT with a detectable ANT ligand in the presence of a candidate agent; and
comparing binding of the detectable ANT ligand to recombinant ANT in the absence of said agent to binding of the detectable ANT ligand to recombinant ANT in the presence of said agent, and therefrom identifying an agent that interacts with an ANT polypeptide.

85. An ANT ligand comprising atractyloside substituted at the 6' hydroxyl to form an atractyloside derivative.

86. The ANT ligand of clause 85 wherein the atractyloside is detectably substituted at the 6' hydroxyl to form a detectable atractyloside derivative.

87. The ANT ligand ofclause86 wherein the detectable atractyloside derivative comprises a radioloabeled substituent.

88. The ANT ligand of clause87 wherein the radiolabeled substituent is selected from the group consisting of $^{125}I$, $^{131}I$, $^{3}H$, $^{14}C$ and $^{35}S$.

89. The ANT ligand of clause86 wherein the detectable atractyloside derivative comprises a fluorescent substituent.

90. The ANT ligand of clause 89 further comprising a $Eu^{3+}$ atom complexed to the atractyloside derivative.

91. The ANT ligand of clause 86 wherein the detectable atractyloside derivative comprises covalently bound biotin.

92. The ANT ligand of clause 85 wherein the atractyloside molecule is substituted at 6' hydroxyl with an amine or an amine containing functionality to form an amine modified atractyloside derivative.

93. The ANT ligand according to any one of clauses85-92 wherein the atractyloside molecule is a carboxyatractyloside molecule that is substituted at 6' hydroxyl to form an atractyloside derivative that is a carboxyatractyloside derivative.

94. An ANT ligand having the following structure:

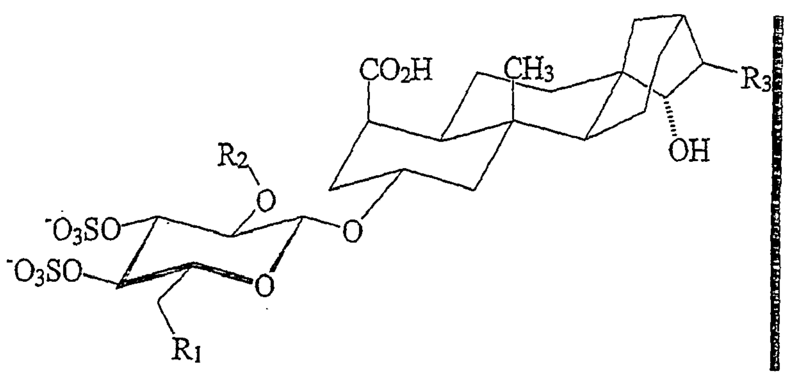

and stereoisomers and pharmaceutically acceptable salts thereof,
wherein

- $R_1$ is hydroxyl, halogen, -OC(=O)$R_4$ or -NH$R_4$;
- $R_2$ is hydrogen or -C(=O)$R_5$;
- $R_3$ is -$CH_3$ or =$CH_2$;
- $R_4$ is -X-aryl, -X-substituted aryl, -X-arylalkyl, -X-substituted arylalkyl, X-heteroaryl, or -X-heteroarylalkyl, wherein X is an optional amido or alkylamido linker moiety; and
- $R_5$ is alkyl.

95. The ANT ligand of clause 94 wheriein $R_1$ is hydroxyl.

96. The ANT ligand of clause 94 wherein $R_1$ is -C(=O)$R_4$.

97. The ANT ligand of clause 94 wherein $R_1$ is -NH$R_4$.

98. The ANT ligand of clause 94 wherein $R_2$ is hydrogen.

99. The ANT ligand of clause 94 wherein $R_2$ is $-C(=O)R_5$.

100. The ANT ligand of clause 94 wherein $R_3$ is $-CH_3$.

101. The ANT ligand of clause 94 wherein $R_3$ is $=CH_2$.

102. The ANT ligand of clause 94 wherien $R_4$ is -X-aryl, -X-substituted aryl, -X-arylalkyl or -X-substituted arylalkyl.

103. The ANT ligand of clause95 wherein $R_5$ is $-CH_2CH(CH_3)_2$.

104. An assay plate for high throughput screening of candidate agents that bind to at least one ANT polypeptide, comprising:

an assay plate having a plurality of wells, each of said wells further comprising at least one immobilized recombinant ANT polypeptide or a variant or fragment thereof.

105. A method of targeting a polypeptide of interest to a mitochondrial membrane, comprising:

expressing a recombinant expression construct encoding a fusion protein in a host cell, said construct comprising at least one regulated promoter operably linked to a nucleic acid molecule comprising a first nucleic acid sequence and a second nucleic acid sequence, said first nucleic acid sequence encoding an adenine nucleotide translocator polypeptide that is expressed as a fusion protein with a polypeptide product of said second nucleic acid sequence, wherein said second nucleic acid sequence encodes the polypeptide of interest.

106. A method of targeting a polypeptide of interest to a mitochondrial membrane, comprising:

expressing a recombinant expression construct encoding a fusion protein in a host cell, said construct comprising at least one promoter operably linked to a nucleic acid molecule comprising a first nucleic acid sequence and a second nucleic acid sequence, said first nucleic acid sequence encoding an animal adenine nucleotide translocator polypeptide that is expressed as a fusion protein with a polypeptide product of said second nucleic acid sequence, wherein said second nucleic acid sequence encodes the polypeptide of interest.

107. A pharmaceutical composition comprising an ANT ligand of clause 85..

108. A pharmaceutical composition comprising an ANT ligand of clause 94.

109. A pharmaceutical composition comprising an agent that binds to an ANT polypeptide identified according to clause 75.

110. A pharmaceutical composition comprising an agent that binds to an ANT polypeptide identified according to clause 83.

111. A pharmaceutical composition comprising an agent that interacts with an ANT polypeptide identified according to clause 84.

112. A method of treatment comprising administering to a subject the pharmaceutical composition of any one of causes 107-111.

## Claims

1. A Pan-ANT antibody that recognizes (i) a human adenine nucleotide translocator 1 polypeptide having an amino acid sequence as set forth in SEQ ID NO:31, (ii) a human adenine nucleotide translocator 2 polypeptide having an amino acid sequence as set forth in SEQ ID NO:32, and (iii) a human adenine nucleotide translocator 3 polypeptide having an amino acid sequence as set forth in SEQ ID NO:33.

2. An isoform-specific antibody that recognizes a human adenine nucleotide translocator 1 polypeptide having an amino acid sequence as set forth in SEQ ID NO:31.

3. An isoform-specific antibody that recognizes a human adenine nucleotide translocator 2 polypeptide having an amino acid sequence as set forth in SEQ ID NO:32.

4. An isoform-specific antibody that recognizes a human adenine nucleotide translocator 3 polypeptide having an amino acid sequence as set forth in SEQ ID NO:33.

5. A polypeptide for use in the preparation of an antibody according to Claim 1 comprising a polypeptide having the amino acid sequence set forth in SEQ ID NO:30.

6. A method for determining the presence of an adeninine nucleotide translocator (ANT) polypeptide in a biological sample comprising:

   contacting a biological sample suspected of containing an ANT polypeptide with an antibody according to any one of Claims 1 to 4, under conditions and for a time sufficient to allow binding of the antibody to the ANT polypeptide; and
   detecting the binding of the antibody to the ANT polypeptide, and therefrom determining the presence of the ANT polypeptide in said biological sample.

7. The method of claim 6 wherein the adenine nucleotide translocator polypeptide is a human adenine nucleotide translocator 1 polypeptide having an amino acid sequence as set forth in SEQ ID NO:31.

8. The method of claim 6 wherein the adenine nucleotide translocator polypeptide is a human adenine nucleotide translocator 2 polypeptide having an amino acid sequence as set forth in SEQ ID NO:32.

9. The method of claim 6 wherein the adenine nucleotide translocator polypeptide is a human adenine nucleotide translocator 3 polypeptide having an amino acid sequence as set forth in SEQ ID NO:33.

10. A method for isolating an adenine nucleotide translocator (ANT) polypeptide from a biological sample, comprising:

    contacting a biological sample suspected of containing an ANT polypeptide with an antibody according to any one of Claims 1 to 4, under conditions and for a time sufficient to allow binding of the antibody to the ANT polypeptide; and

recovering the ANT polypeptide, and thereby isolating the ANT polypeptide from the biological sample

**11.** The method of claim 10 wherein the antibody is covalently bound to a solid phase.

**12.** The method of claim 10 wherein the antibody is non-covalently bound to a solid phase.

**13.** An antibody according to Claim 1 wherein said antibody is prepared by the steps of

(a) conjugating a polypeptide having the amino acid sequence set forth in SEQ ID NO:30 to a carrier molecule to obtain a conjugated material;
(b) immunizing a rabbit with the conjugated material of (a); and
(c) collecting blood from the rabbit of (b) at least seven weeks post-inoculation to obtain the Pan-ANT antibody therein.

**14.** An antibody according to Claim 2, wherein said antibody is prepared by the steps of

(a) conjugating a polypeptide having the amino acid sequence set forth in SEQ ID NO:40 to a carrier molecule to obtain a conjugated material;
(b) immunizing a rabbit with the conjugated material of (a); and
(c) collecting blood from the rabbit of (b) at least seven weeks post-inoculation to obtain the isoform-specific ANT antibody therein.

**15.** An antibody according to Claim 3, wherein said antibody is prepared by the steps of

(a) conjugating a polypeptide having the amino acid sequence set forth in SEQ ID NO:39 to a carrier molecule to obtain a conjugated material;
(b) immunizing a rabbit with the conjugated material of (a); and
(c) collecting blood from the rabbit of (b) at least seven weeks post-inoculation to obtain the isoform-specific ANT antibody therein.

**16.** An antibody according to Claim 4, wherein said antibody is prepared by the steps of

(a) conjugating a polypeptide having the amino acid sequence set forth in SEQ ID NO:38 to a carrier molecule to obtain a conjugated material;
(b) immunizing a rabbit with the conjugated material of (a); and
(c) collecting blood from the rabbit of (b) at least seven weeks post-inoculation to obtain the isoform-specific ANT antibody therein.

```
ANT1m  ATGGGTGATCACGCTTGGAGCTTCCTAAAGGACTTCCTGGCCGGGGCGGTCGCCGCTGCCGTCTCCAAGACCGCGGTCGC  80
ANT2m  ATGACAGATGCCGCTGTGTCCTTCGCCAAGGACTTCCTGGCAGGTGGAGTGGCCGCAGCCATCTCCAAGACGGCGGTAGC  80
ANT3m  ATGACGGAACAGGCCATCTCCTTCGCCAAAGACTTCTTGGCCGGAGGCATCGCCGCCGCCATCTCCAAGACGGCCGTGGC  80

ANT1m  CCCCATCGAGAGGGTCAAACTGCTGCTGCAGGTCCAGCATGCCAGCAAACAGATCAGTGCTGAGAAGCAGTACAAAGGGA  160
ANT2m  GCCCATCGAGCGGGTCAAGCTGCTGCTGCAGGTGCAGCATGCCAGCAAGCAGATCACTGCAGATAAGCAATACAAAGGCA  160
ANT3m  TCCGATCGAGCGGGTCAAGCTGCTGCTGCAGGTCCAGCACGCCAGCAAGCAGATCGCCGCCGACAAGCAGTACAAGGGCA  160

ANT1m  TCATTGATTGTGTGGTGAGAATCCCTAAGGAGCAGGGCTTCCTCTCCTTCTGGAGGGGTAACCTGGCCAACGTGATCCGT  240
ANT2m  TTATAGACTGCGTGGTCCGTATTCCCAAGGAGCAGGAAGTTCTGTCCTTCTGGCGCGGTAACCTGGCCAATGTCATCAGA  240
ANT3m  TCGTGGACTGCATTGTCCGCATCCCCAAGGAGCAGGGCGTGCTGTCCTTCTGGAGGGGCAACCTTGCCAACGTCATTCGC  240

ANT1m  TACTTCCCCACCCAAGCTCTCAACTTCGCCTTCAAGGACAAGTACAAGCAGCTCTTCCTTAGGGGGTGTGGATCGGCATAA  320
ANT2m  TACTTCCCCACCCAGGCTCTTAACTTCGCCTTCAAAGATAAATACAAGCAGATCTTCCTGGGTGGTGTGGACAAGAGAAC  320
ANT3m  TACTTCCCCACTCAAGCCCTCAACTTCGCCTTCAAGGATAAGTACAAGCAGATCTTCCTGGGGGGCGTGGACAAGCACAC  320

ANT1m  GCAGTTCTGGCGCTACTTTGCTGGTAACCTGGCGTCCGGTGGGGCCGCTGGGGCCACCTCCCTTTTGCTTTGTCTACCCGC  400
ANT2m  CCAGTTTTGGCTCTACTTTGCAGGGAATCTGGCATCGGGTGGTGCCGCAGGGGCCACATCCCTGTGTTTTGTGTACCCTC  400
ANT3m  GCAGTTCTGGAGGTACTTTGCGGGCAACCTGGCCTCCGGCGGTGCGGCCGGCGCGGACCTCCCTCTGCTTCGTGTACCCGC  400

ANT1m  TGGACTTTGCTAGGACCAGGTTGGCTGCTGATGTGGGCAGGC---GCGCCCAGCGTGAGTTCCATGGTCTGGGCGACTGT  477
ANT2m  TTGATTTTGCCCGTACCCGTCTAGCAGCTGATGTGGGTAAAGCTGGAGCTGAAAGGGAATTCCGAGGCCTCGGTGACTGC  480
ANT3m  TGGATTTTGCCAGAACCCGCCTGGCAGCGGACGTGGGAAAGTCAGGCACAGAGCGCGAGTTCCGAGGCCTGGGAGACTGC  480
```

*Fig. 1A*

```
ANT1m  ATCATCAAGATCTTTCAAGTCTGATGGCCTTGAGGGGGCTCTACCAGGGTTTCAACGTCTCTGTCCAAGGCATCATTATCTA  557
ANT2m  CTGGTTAAGATCTACAAATCTGATGGGATTAAGGGCCTGTACCAAGGCTTTAACGTGTCTGTGCAGGGTATTATCATCTA  560
ANT3m  CTGGTGAAGATCACCAAGTCCGACGGCATCCGGGGCCTGTACCAGGGCTTCAGTGTCTCCGTGCAGGGCATCATCATCTA  560

ANT1m  TAGAGCTGCCTACTTCGGAGTCTATGATACTGCCAAGGGGATGCTGCCTGACCCCAAGAACGTGCACATTTTTGTGAGCT  637
ANT2m  CCGAGCCGCCTACTTCGGTATCTATGACACTGCAAAGGGAATGCTTCCGGATCCCAAGAACACTCACATCGTCATCAGCT  640
ANT3m  CCGGGCGGCCTACTTCGGCGTGTACGATACGGCCAAGGGCATGCTCCCCGACCCCAAGAACACGCACATCGTGGTGAGCT  640

ANT1m  GGATGATTGCCCAGAGTGTGACGGCAGTCGCAGGGCTGCTGTCCTACCCCTTTGACACTGTTCGTCGTAGAATGATGATG  717
ANT2m  GGATGATCGCACAGACTGTCACTGCTGTTGCCGGGTTGACTTCCTATCCATTTGACACTGTTCGCCGCCGCATGATGATG  720
ANT3m  GGATGATCGCGCAGACCGTGACGGCCGTGGCCGGCGTGGTGTCCTACCCCTTCGACACCGTGCGGCGGCGCATGATGATG  720

ANT1m  CAGTCCGGCCGGAAAGGGGCCGATATTATGTACACGGGGACAGTTGACTGCTGGAGGAAGATTGCAAAAGACGAAGGAGC  797
ANT2m  CAGTCAGGGCGCAAAGGAACTGACATCATGTACACAGGCACGCTTGACTGCTGGCGGAAGATTGCTCGTGATGAAGGAGG  800
ANT3m  CAGTCCGGGCGCAAAGGAGCTGACATCATGTACACGGGCACCGTCGACTGTTGGAGGAAGATCTTCAGAGATGAGGGCGG  800

ANT1m  CAAGGCCTTCTTCAAAGGTGCCTGGTCCAATGTGCTGAGAGGCATGGGCGGTGCTTTTGTATTGGTGTTGTATGATGAGA  877
ANT2m  CAAAGCTTTTTTCAAGGGTGCATGGTCCAATGTTCTCAGAGGCATGGGTGGTGCTTTTGTGCTTGTCTTGTATGATGAAA  880
ANT3m  CAAGGCCTTCTTCAAGGGTGCGTGGTCCAACGTCCTGCGGGGCATGGGGGCGCCTTCGTGCTGGTCCTGTACGACGAGC  880

ANT1m  TCAAAAAATATGTCTAA  894
ANT2m  TCAAGAAGTACACATAA  897
ANT3m  TCAAGAAGGTGATCTAA  897
```

*Fig. 1B*

```
HANT1p  MGDHAWSFLKDFLAGAVAAAVSKTAVAPIERVKLLLQVQHASKQISAEKQ  50
HANT2p  MTDAAVSFAKDFLAGGVAAAISKTAVAPIERVKLLLQVQHASKQITADKQ  50
HANT3p  MTEQAISFAKDFLAGGIAAAISKTAVAPIERVKLLLQVQHASKQIAADKQ  50

HANT1p  YKGIIDCVVRIPKEQGFLSFWRGNLANVIRYFPTQALNFAFKDKYKQLFL  100
HANT2p  YKGIIDCVVRIPKEQEVLSFWRGNLANVIRYFPTQALNFAFKDKYKQIFL  100
HANT3p  YKGIVDCIVRIPKEQGVLSFWRGNLANVIRYFPTQALNFAFKDKYKQIFL  100

HANT1p  GGVDRHKQFWRYFAGNLASGGAAGATSLCFVYPLDFARTRLAADVGRR-A  149
HANT2p  GGVDKRTQFWLYFAGNLASGGAAGATSLCFVYPLDFARTRLAADVGKAGA  150
HANT3p  GGVDKHTQFWRYFAGNLASGGAAGATSLCFVYPLDFARTRLAADVGKSGT  150

HANT1p  QREFHGLGDCIIKIFKSDGLRGLYQGFNVSVQGIIIYRAAYFGVYDTAKG  199
HANT2p  EREFRGLGDCLVKIYKSDGIKGLYQGFNVSVQGIIIYRAAYFGIYDTAKG  200
HANT3p  EREFRGLGDCLVKITKSDGIRGLYQGFSVSVQGIIIYRAAYFGVYDTAKG  200

HANT1p  MLPDPKNVHIFVSWMIACSVTAVAGLLSYPFDTVRRRMMMQSGRKGADIM  249
HANT2p  MLPDPKNTHIVISWMIAQTVTAVAGLTSYPFDTVRRRMMMQSGRKGTDIM  250
HANT3p  MLPDPKNTHIVVSWMIAQTVTAVAGVVSYPFDTVRRRMMMQSGRKGADIM  250

HANT1p  YTGTVDCWRKIAKDEGAKAFFKGAWSNVLRGMGGAFVLVLYDEIKKYV.  298
HANT2p  YTGTLDCWRKIARDEGGKAFFKGAWSNVLRGMGGAFVLVLYDEIKKYT.  299
HANT3p  YTGTVDCWRKIFRDEGGKAFFKGAWSNVLRGMGGAFVLVLYDELKKVI.  299
```

*Fig. 2*

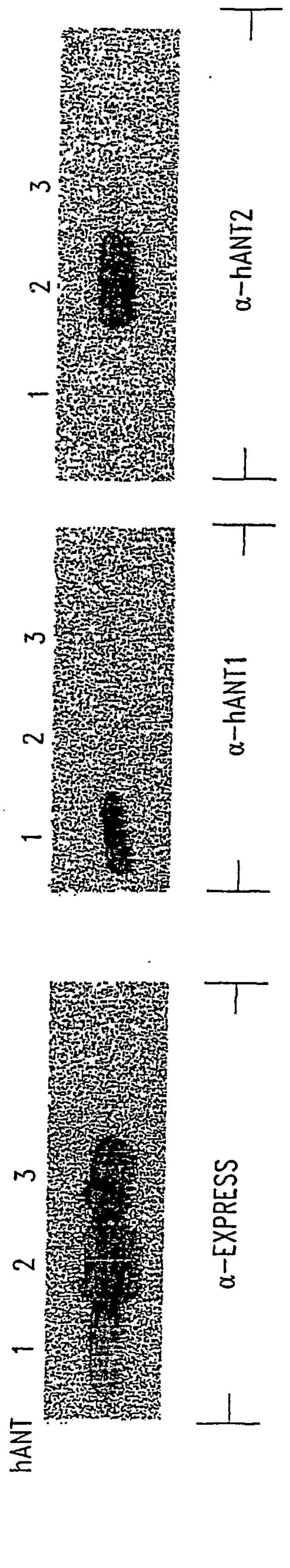


*Fig. 3*

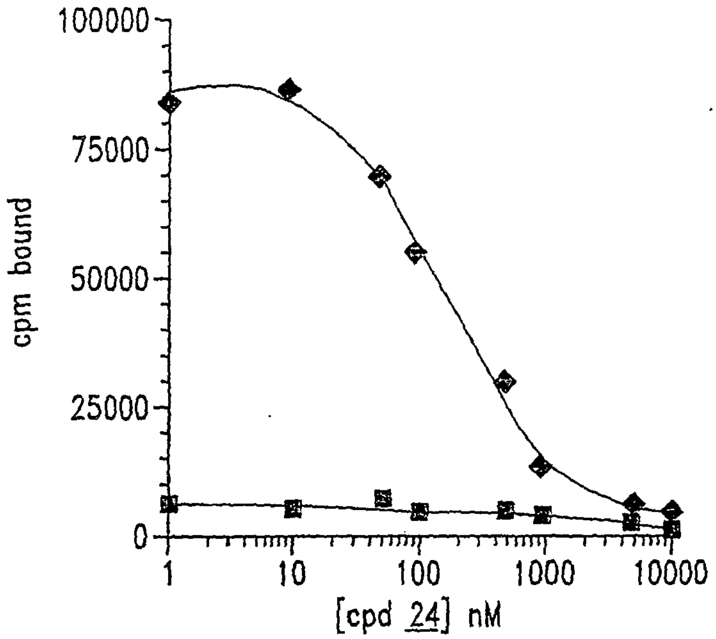


*Fig. 4*

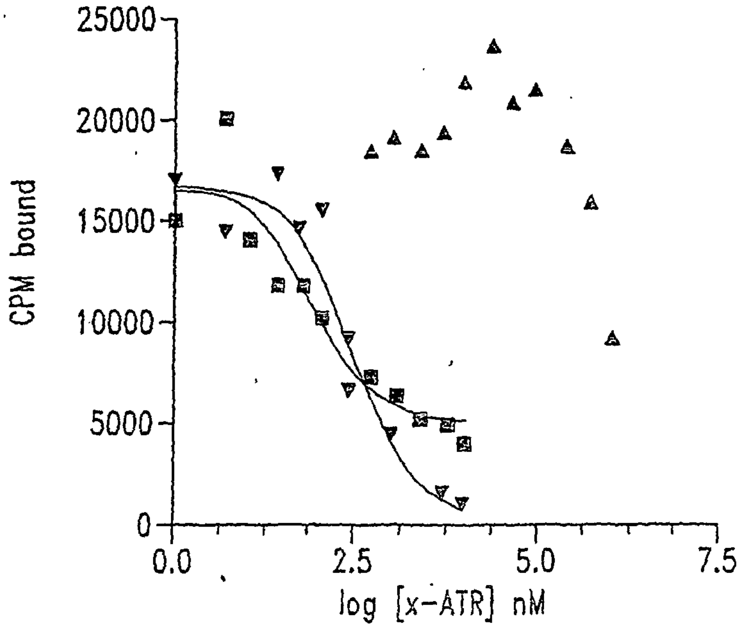


[cpd 24] nM

*Fig. 5*

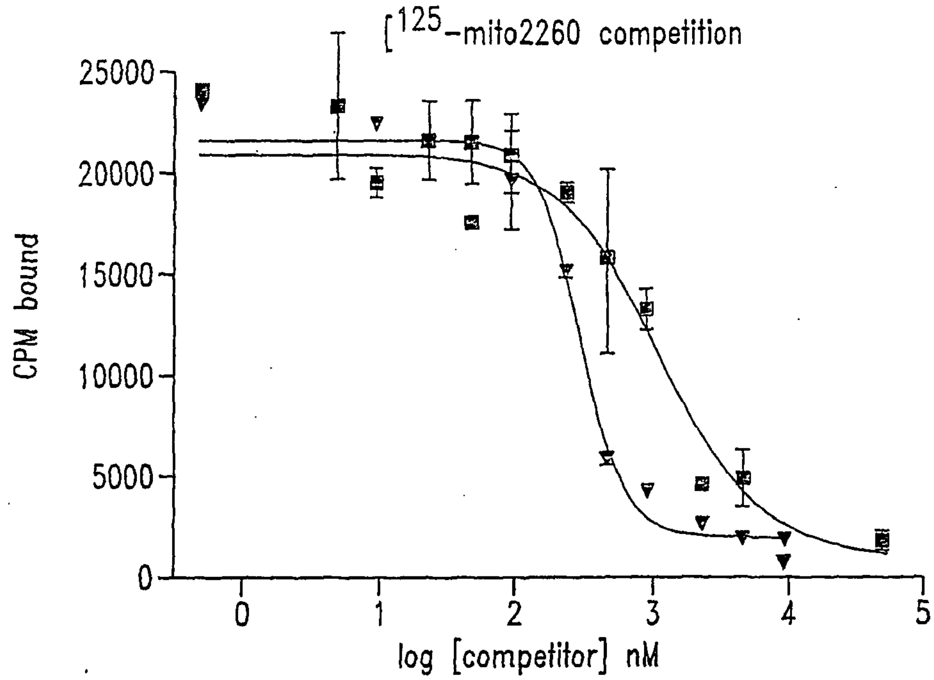
[125-mito2260 competition
25000
20000
15000
10000
5000
0
CPM bound
0
1
2
3
4
5
log [competitor] nM

*Fig. 6*

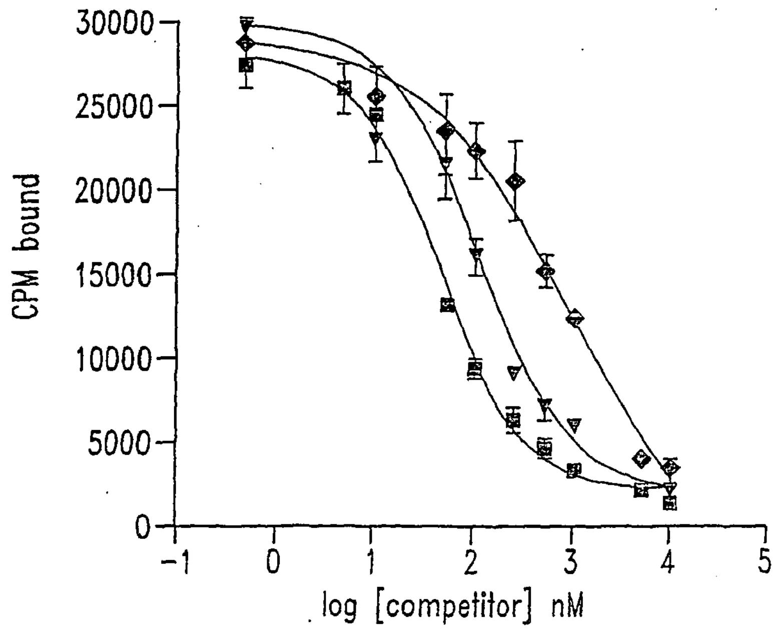
30000
25000
20000
15000
10000
5000
0
CPM bound
−1
0
1
2
3
4
5
log [competitor] nM

*Fig. 7*

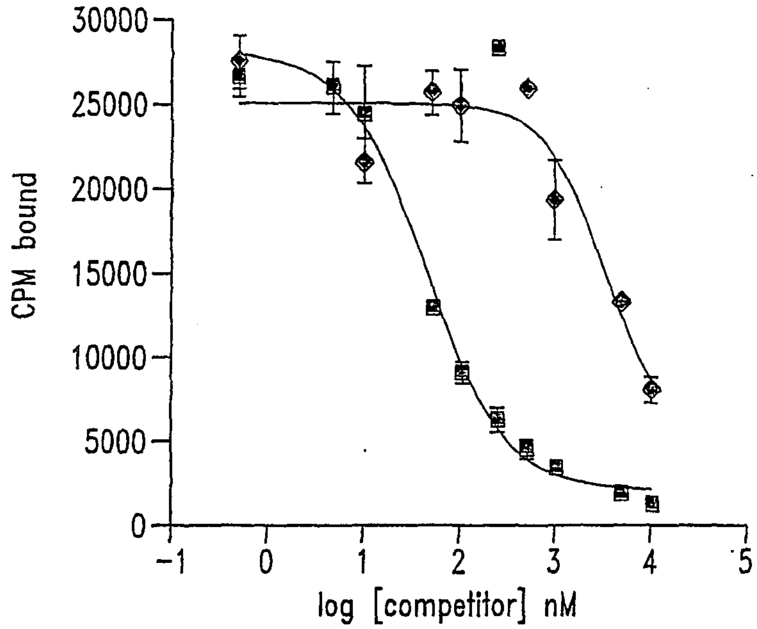
30000
25000
20000
15000
10000
5000
0
CPM bound
-1
0
1
2
3
4
5
log [competitor] nM

*Fig. 8*

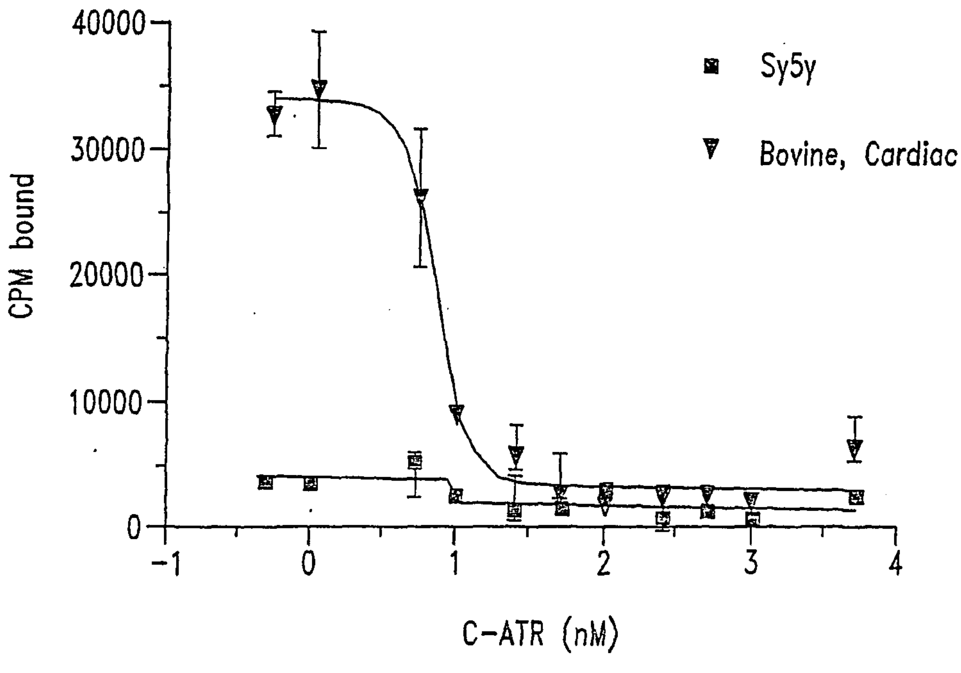
40000
30000
20000
10000
0
CPM bound
Sy5y
Bovine, Cardiac
-1
0
1
2
3
4
C-ATR (nM)

*Fig. 10*

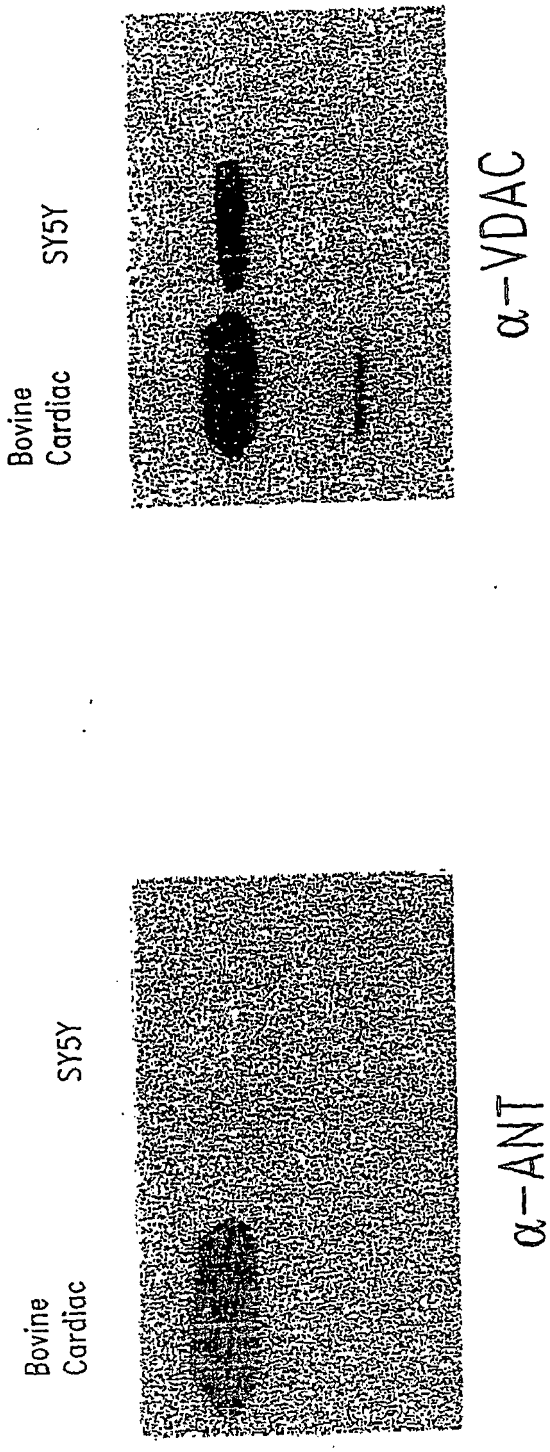


*Fig. 9*

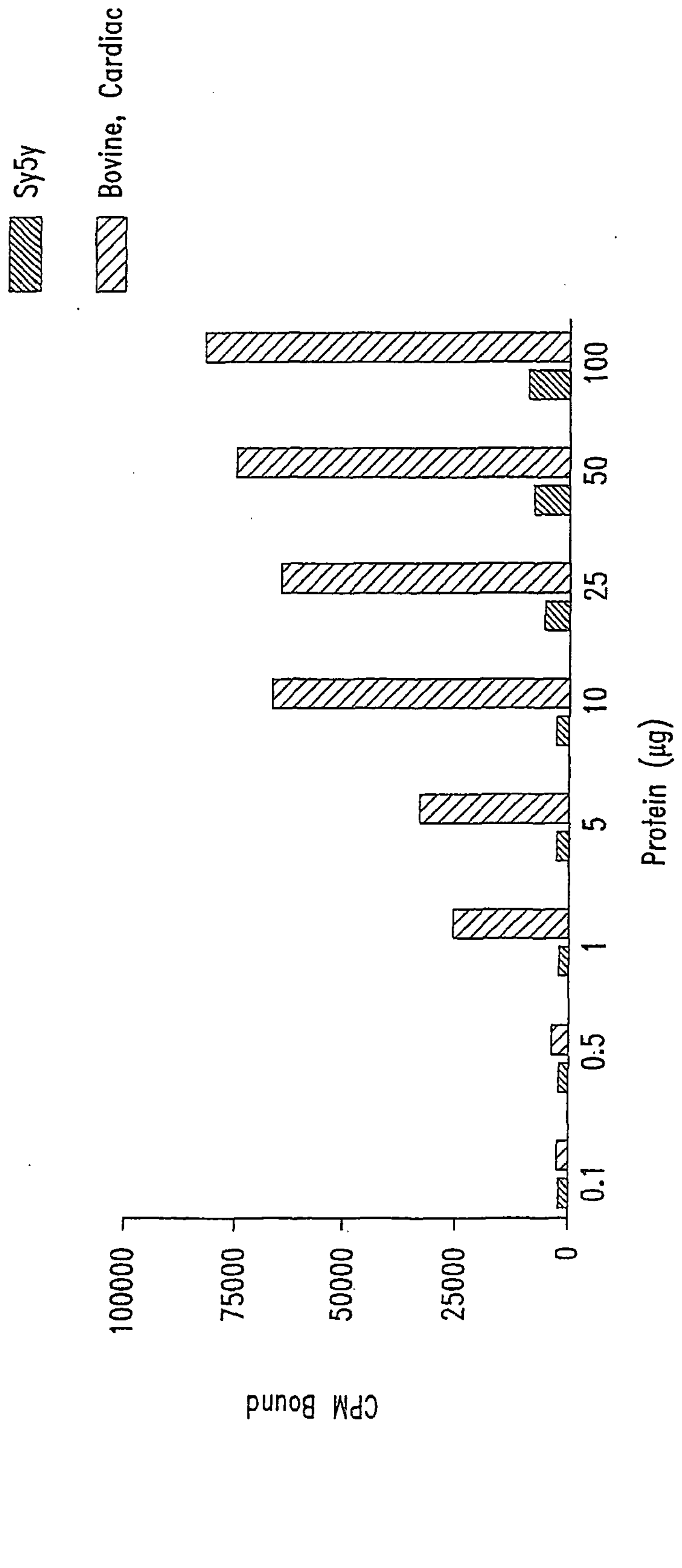
Sy5y
Bovine, Cardiac
CPM Bound
100000
75000
50000
25000
0
0.1
0.5
1
5
10
25
50
100
Protein (µg)

Fig. 11

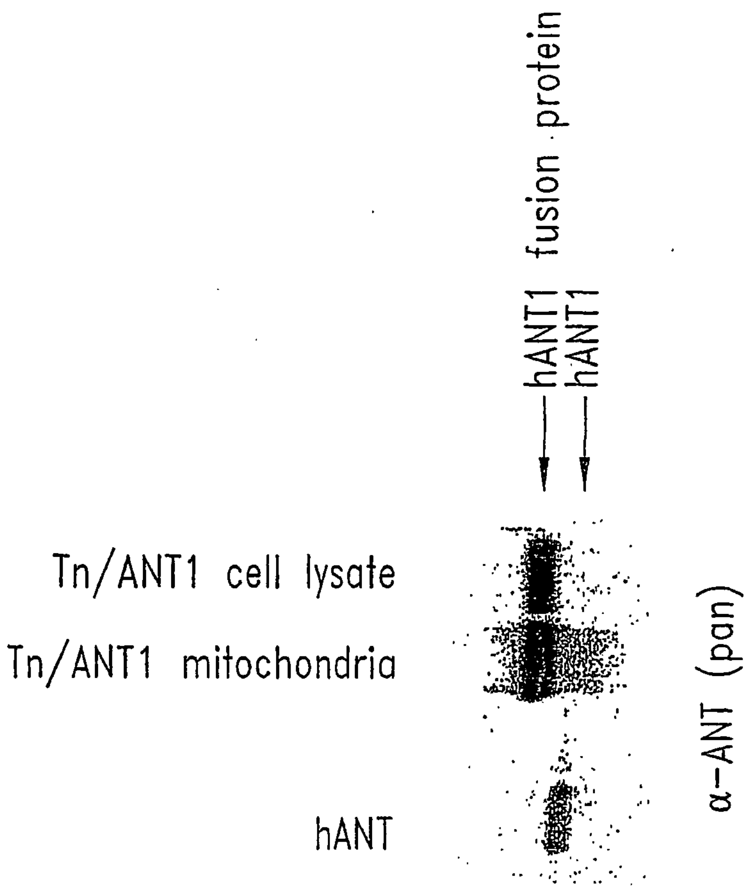
Tn/ANT1 cell lysate
Tn/ANT1 mitochondria
hANT
hANT1 fusion protein
hANT1
α-ANT (pan)

*Fig. 12*

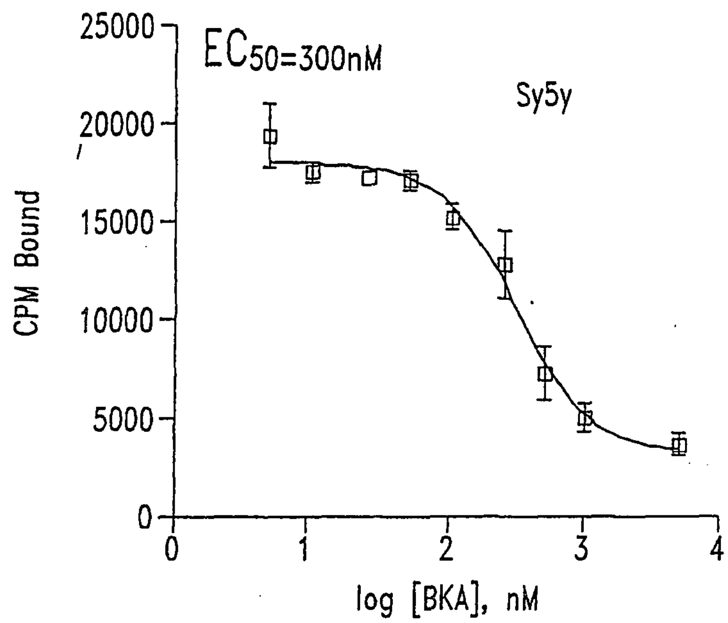
EC50=300nM
Sy5y
CPM Bound
25000
20000
15000
10000
5000
0
0
1
2
3
4
log [BKA], nM

*Fig. 13*

European Patent Office

# EUROPEAN SEARCH REPORT

Application Number
EP 06 02 2533

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| P,X | MANCHADO CARLOS ET AL: "Epitope mapping of mitochondrial adenine nucleotide translocase-1 in idiopathic dilated cardiomyopathy." JOURNAL OF MOLECULAR AND CELLULAR CARDIOLOGY MAY 2002, vol. 34, no. 5, May 2002 (2002-05), pages 571-582, XP002426776 ISSN: 0022-2828 * page 576, left-hand column, paragraph 2 - page 578, left-hand column, paragraph 1 * * figure 3; table 3 * | 1 | INV. C12N15/12 C07K14/47 C12N15/62 C12N5/10 C12N9/00 C12N15/86 G01N33/50 C07H15/24 |
| P,X | WO 00/26370 A (ANDERSON CHRISTEN M ;CLEVENGER WILLIAM (US); WILEY SANDRA EILEEN () 11 May 2000 (2000-05-11) * page 85, line 10 - page 86, line 15 * | 4,16 | |
| P,X | WO 01/32876 A (CLEVENGER WILLIAM ;FRIGERI LUCIANO G (US); MITOKOR (US); MURPHY AN) 10 May 2001 (2001-05-10) * example 14.A * | 4,16 | TECHNICAL FIELDS SEARCHED (IPC) |
| X | GIRAUD, S. ET AL.: "Expression of human ANT2 gene in highly proliferative cells: GRBOX, a new transcriptional element, is involved in the regulation of glycolytic ATP import into mitochondria." JOURNAL OF MOLECULAR BIOLOGY, vol. 281, no. 3, 21 August 1998 (1998-08-21), pages 409-18, XP000901735 * page 415, right-hand column, paragraph 3 * * figure 2 * | 3,6,8, 10-12,15 | C12N C07K G01N C07H |

-/--

The present search report has been drawn up for all claims

| Place of search | Date of completion of the search | Examiner |
|---|---|---|
| Munich | 30 March 2007 | Tudor, Mark |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

7

European Patent Office

# EUROPEAN SEARCH REPORT

Application Number
EP 06 02 2533

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | COZENS, A.L. ET AL.: "DNA sequence of two expressed nuclear genes for human mitochondrial ADP-ATP translocase." JOURNAL OF MOLECULAR BIOLOGY, vol. 206, 5 March 1989 (1989-03-05), - 20 April 1989 (1989-04-20) pages 261-80, XP000905070 * figure 7 * ----- | 2,4,6,7, 9-12,14, 16 | |
| X | KU, D.-H. ET AL.: "The human fibroblast adenine nucleotide translocator gene." JOURNAL OF BIOLOGICAL CHEMISTRY, vol. 265, no. 24, 25 August 1989 (1989-08-25), pages 16060-3, XP002136075 * abstract * * page 16062, left-hand column, paragraph 5 * * figure 1 * ----- | 2,6,7, 10-12,14 | |
| X | LI ET AL: "A human muscle adenine nucleotide translocator gene has four exons, is located on chromosome 4, and is differentially expressed" JOURNAL OF BIOLOGICAL CHEMISTRY, AMERICAN SOCIETY OF BIOLOCHEMICAL BIOLOGISTS, BIRMINGHAM,, US, vol. 264, no. 24, 25 August 1989 (1989-08-25), pages 13998-14004, XP002136076 ISSN: 0021-9258 * figure 2 * ----- -/-- | 2,6,7, 10-12,14 | TECHNICAL FIELDS SEARCHED (IPC) |

The present search report has been drawn up for all claims

| Place of search | Date of completion of the search | Examiner |
|---|---|---|
| Munich | 30 March 2007 | Tudor, Mark |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

7

European Patent Office

# EUROPEAN SEARCH REPORT

Application Number
EP 06 02 2533

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE UniProt [Online] 1 October 1989 (1989-10-01), "ADP/ATP translocase 3 (Adenine nucleotide translocator 2) (ANT 3) (ADP,ATP carrier protein 3) (Solute carrier family 25 member 6) (ADP,ATP carrier protein, isoform T2)." XP002426785 retrieved from EBI accession no. UNIPROT:P12236 Database accession no. P12236 * abstract * ----- | 5 | |

TECHNICAL FIELDS SEARCHED (IPC)

The present search report has been drawn up for all claims

| Place of search | Date of completion of the search | Examiner |
|---|---|---|
| Munich | 30 March 2007 | Tudor, Mark |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

7

EPO FORM 1503 03.82 (P04C01)

European Patent Office

Application Number

EP 06 02 2533

## CLAIMS INCURRING FEES

The present European patent application comprised at the time of filing more than ten claims.

- [ ] Only part of the claims have been paid within the prescribed time limit. The present European search report has been drawn up for the first ten claims and for those claims for which claims fees have been paid, namely claim(s):

- [ ] No claims fees have been paid within the prescribed time limit. The present European search report has been drawn up for the first ten claims.

## LACK OF UNITY OF INVENTION

The Search Division considers that the present European patent application does not comply with the requirements of unity of invention and relates to several inventions or groups of inventions, namely:

see sheet B

- [ ] All further search fees have been paid within the fixed time limit. The present European search report has been drawn up for all claims.

- [X] As all searchable claims could be searched without effort justifying an additional fee, the Search Division did not invite payment of any additional fee.

- [ ] Only part of the further search fees have been paid within the fixed time limit. The present European search report has been drawn up for those parts of the European patent application which relate to the inventions in respect of which search fees have been paid, namely claims:

- [ ] None of the further search fees have been paid within the fixed time limit. The present European search report has been drawn up for those parts of the European patent application which relate to the invention first mentioned in the claims, namely claims:

European Patent Office

LACK OF UNITY OF INVENTION
SHEET B

Application Number
EP 06 02 2533

The Search Division considers that the present European patent application does not comply with the requirements of unity of invention and relates to several inventions or groups of inventions, namely:

1. claims: 1,5,13 (completely), 6, 10-12 (partially)

   An antibody which recognises human ANT1, human ANT2 and human ANT3; a polypeptide for use in the preparation of said antibody; the use of said antibody in determining the presence of ANT in a biological sample; and, a method for isolating ANT using said antibody.

   ---

2. claims: 2,7,14 (completely), 6, 10-12 (partially)

   An antibody which specifically recognises human ANT1; the use of said antibody in determining the presence of human ANT1 in a biological sample; and, a method for isolating human ANT1 using said antibody.

   ---

3. claims: 3,8,15 (completely), 6, 10-12 (partially)

   An antibody which specifically recognises human ANT2; the use of said antibody in determining the presence of human ANT2 in a biological sample; and, a method for isolating human ANT2 using said antibody.

   ---

4. claims: 4,9,16 (completely), 6, 10-12 (partially)

   An antibody which specifically recognises human ANT3; the use of said antibody in determining the presence of human ANT3 in a biological sample; and, a method for isolating human ANT3 using said antibody.

   ---

## ANNEX TO THE EUROPEAN SEARCH REPORT ON EUROPEAN PATENT APPLICATION NO. EP 06 02 2533

This annex lists the patent family members relating to the patent documents cited in the above-mentioned European search report.
The members are as contained in the European Patent Office EDP file on
The European Patent Office is in no way liable for these particulars which are merely given for the purpose of information.

30-03-2007

| Patent document cited in search report | Publication date | Patent family member(s) | Publication date |
|---|---|---|---|
| WO 0026370 A | 11-05-2000 | AU 769756 B2 | 05-02-2004 |
| | | AU 2472900 A | 22-05-2000 |
| | | CA 2349444 A1 | 11-05-2000 |
| | | EP 1049780 A1 | 08-11-2000 |
| | | JP 2002539761 T | 26-11-2002 |
| | | JP 2004154139 A | 03-06-2004 |
| | | US 2005003352 A1 | 06-01-2005 |
| | | US 2005003353 A1 | 06-01-2005 |
| | | US 2002012992 A1 | 31-01-2002 |
| | | US 2001044144 A1 | 22-11-2001 |
| | | US 2005019835 A1 | 27-01-2005 |
| | | US 2005064530 A1 | 24-03-2005 |
| WO 0132876 A | 10-05-2001 | AU 1362201 A | 14-05-2001 |
| | | CA 2389754 A1 | 10-05-2001 |
| | | EP 1228206 A2 | 07-08-2002 |
| | | JP 2003516128 T | 13-05-2003 |
| | | US 6562563 B1 | 13-05-2003 |
| | | US 6797467 B1 | 28-09-2004 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 09161172 B
- WO 9819714 A
- US 4518584 A
- US 4737462 A
- EP 212914 A
- US 5011912 A
- EP 0318554 A
- US 5132405 A
- US 5091513 A
- US 5476786 A
- US 5100788 A
- WO 8903422 A
- US 5489528 A
- US 5672691 A
- WO 9324631 A
- US 5168049 A
- US 5272254 A
- EP 511747 A
- US 5168053 A, Altman
- US 5190931 A, Inouye
- US 5135917 A, Burch
- US 5087617 A, Smith
- US 5176996 A, Hogan
- US 5272262 A
- US 5144019 A
- US 5180818 A
- US 5116742 A
- US 5093246 A, Cech
- US 9504063 W


### Non-patent literature cited in the description


- **ERNSTER et al.** *J. Cell Biol.,* 1981, vol. 91, 227s
- **WILSON.** *Cell Death Differen.,* 1998, vol. 5, 646-652
- **KROEMER et al.** *FASEB J.,* 1995, vol. 9, 1277-87
- **ZAMZAMI et al.** *J. Exp. Med.,* 1995, vol. 182, 367-77
- **ZAMZAMI et al.** *J. Exp. Med,* 1995, vol. 181, 1661-72
- **AUSSERER et al.** *Mol Cell. Biol.,* 1994, vol. 14, 5032-42
- **NEWMEYER et al.** *Cell,* 1994, vol. 79
- **LIU et al.** *Cell,* 1996, vol. 86, 147-157
- **GREEN ; REED.** *Science,* 1998, vol. 281, 1309-1312
- **GREEN.** *Cell,* 1998, vol. 94, 695-698
- **KROMER.** *Nature Medicine,* 1997, vol. 3, 614-620
- **KLINGENBERG.** *J. Bioenergetics and Biomembranes,* 1993, vol. 25, 447-457
- **WALLACE et al.** Mitochondria & Free Radicals in Neurodegenerative Diseases. Wiley-Liss, 1998, 283-307
- **MIROUX et al.** *J. Mol. Biol.,* 1996, vol. 260, 289
- **BEUTNER et al.** *Biochim. Biophys. Acta,* 1998, vol. 1368, 7
- **OBATOMI ; BACH.** *Toxicol. Lett.,* 1996, vol. 89, 155
- **GREEN ; REED.** *Science,* 1998, vol. 281, 1309
- **KROEMER et al.** *Annu. Rev. Physiol.,* 1998, vol. 60, 619
- **FIORE et al.** *Biochimie,* 1998, vol. 80, 137-150
- **DOERNER et al.** *Biochim. Biophys. Acta,* 1999, vol. 141, 16-24
- **DOERNER et al.** *FEBS Lett.,* 1997, vol. 414, 258
- **DRUMMLER et al.** *Biochem. J.,* 1996, vol. 317, 913-918
- **WOLDEGIORGIS et al.** *J. Biol. Chem.,* 1985, vol. 260, 7585
- **GIRAUD et al.** *J. Mol. Biol.,* 1998, vol. 281, 409
- **FAURE-VIGNY et al.** *Molec. Carcinogen.,* 1996, vol. 16, 165-172
- **STEPIEN et al.** *J. Biol. Chem.,* 1992, vol. 267, 14592
- **GRAHAM et al.** *Nat. Genet.,* 1997, vol. 16, 226
- **NECKELMANN et al.** *Proc. Nat'l. Acad. Sci. U.S.A.,* 1987, vol. 84, 7580-7.584
- **COZENS et al.** *J. Mol. Biol.,* 1989, vol. 206, 261-280
- **LI et al.** *J. Biol. Chem.,* 1989, vol. 264, 13998
- **LI et al.** *J. Biol. Chem.,* 1990, vol. 265, 20585
- **SHINOHARA et al.** *Biochim. Biophys. Acta,* 1993, vol. 1152, 192

- **KU et al.** *J. Biol. Chem.,* 1990, vol. 265, 16060
- **ADAMS et al.** *Science,* 1991, vol. 252, 1651
- **WALDER et al.** *Gene,* 1986
- **BAUER et al.** *Gene,* 1985, vol. 37, 73
- **CRAIK.** *BioTechniques,* January 1985, 12-19
- **SMITH et al.** Genetic Engineering: Principles and Methods. Plenum Press, 1981
- **KUNKEL.** *Proc. Natl. Acad. Sci. USA,* 1985, vol. 82, 488
- **KUNKEL et al.** *Methods in Enzymol.,* 1987, vol. 154, 367
- **HOPP et al.** *Bio/Technology,* 1988, vol. 6, 1204
- **WILSON et al.** *Cell,* 1984, vol. 37, 767
- **ASHKENAZI et al.** *PNAS USA,* 1991, vol. 88, 10535
- **BYRN et al.** *Nature,* 1990, vol. 344, 677
- **SAMBROOK et al.** Molecular Cloning: A Laboratory Manual. Cold Spring Harbor, 1989
- **AUSUBEL et al.** Current Protocols in Molecular Biology. Greene Publ. Assoc. Inc. & John Wiley & Sons, Inc, 1993
- **SAMBROOK et al.** Molecular Cloning. Cold Spring Harbor Laboratory, 1989
- **MANIATIS et al.** Molecular Cloning. Cold Spring Harbor Laboratory, 1982
- **GUZMAN et al.** *J. Bacteriol.,* 1995, vol. 177, 4121
- **CARRA et al.** *EMBO J.,* 1993, vol. 12, 35
- **MAYER.** *Gene,* 1995, vol. 163, 41
- **HALDIMANN et al.** *J. Bacteriol.,* 1998, vol. 180, 1277
- **LUTZ et al.** *Nuc. Ac. Res.,* 1997, vol. 25, 1203
- **ALLGOOD et al.** *Curr. Opin. Biotechnol.,* 1997, vol. 8, 474
- **MAKRIDES.** *Microbiol. Rev.,* 1996, vol. 60, 512
- **MILLER et al.** *Biotechniques,* 1989, vol. 7, 980-990
- Baculovirus Expression Protocols, Methods in Molecular Biology. Human Press, 1995, vol. 39
- Expression of Proteins in Insect Cells Using Baculoviral Vectors. **PIWNICA-WORMS et al.** Short Protocols in Molecular Biology. John Wiley & Sons, 1992, 16-3216-48
- **GLUZMAN.** *Cell,* 1981, vol. 23, 175
- **DAVIS et al.** *Basic Methods in Molecular Biology,* 1986
- **AGRWAL et al.** *Tetrehedron Lett.,* 1987, vol. 28, 3539-3542
- **MILLER et al.** *J. Am. Chem. Soc.,* 1971, vol. 93, 6657-6665
- **STEC et al.** *Tetrehedron Lett.,* 1985, vol. 26, 2191-2194
- **MOODY et al.** *Nucl. Acids Res.,* 1989, vol. 12, 4769-4782
- **UZNANSKI et al.** *Nucl. Acids Res.,* 1989
- **LETSINGER et al.** *Tetrahedron,* 1984, vol. 40, 137-143
- **ECKSTEIN.** *Annu. Rev. Biochem.,* 1985, vol. 54, 367-402
- **ECKSTEIN.** *Trends Biol. Sci.,* 1989, vol. 14, 97-100
- **STEIN.** Oligodeoxynucleotides. Antisense Inhibitors of Gene Expression. Macmillan Press, 1989, 97-117
- **JAGER et al.** *Biochemistry,* 1988, vol. 27, 7237-7246
- **CLUSEL et al.** *Nucl. Acids Res.,* 1993, vol. 21, 3405-3411
- **GIRAUD et al.** *J. Mol Biol.,* 1998, vol. 281, 409
- **HAUGLAND.** Handbook of Fluorescent Probes and Research Chemicals- Sixth Ed. 1996, 266-274589-594
- Pierce Catalog and Handbook. Pierce Chemical Company, 1994
- **SCOPES, R.K.** Protein Purification: Principles and Practice. Springer-Verlag, 1987
- **HERMANSON, G.T. et al.** Immobilized Affinity Ligand Techniques. Academic Press, Inc, 1992
- **BLOCK et al.** *Meths. Enzymol.,* 1986, vol. 125, 658
- **STUBBS.** *Pharm. Ther.,* 1979, vol. 7, 329
- **KLINGENBERG et al.** *Biochim. Biophys. Acta,* 1978, vol. 503, 193
- **STERLING.** *Endocrinol,* 1986, vol. 119, 292
- **MAJIMA et al.** *Biochem.,* 1998, vol. 37, 424
- **BEAVIS et al.** *J. Biol. Chem.,* 1993, vol. 268, 997
- **POWERS et al.** *J. Biol. Chem.,* 1994, vol. 269, 10614
- **BOULAY et al.** *Analytical Biochemistry,* 1983, vol. 128, 323-330
- **ROUX et al.** *Analytical Biochemistry,* 1996, vol. 234, 31-37
- **LAUQUIN et al.** *FEBS Letters,* 1976, vol. 67, 306-31
- **GOTTIKH et al.** *Tetrahedron,* 1970, vol. 26, 4419-4433
- **HAUGLAND.** Handbook of Fluorescent Probes and Research Chemicals- Sixth Ed. 1996
- **SMITH, A.L.** *Meths. Enzymol.,* vol. 10, 81-86
- Mitochondria: A Practical Approach. IRL Press
- **STORRIE et al.** *Meths. Enzymol.,* 1990, vol. 182, 203-255
- **JEZEK et al.** *J. Biol. Chem.,* 1990, vol. 265, 10522-10526
- **CHENG ; PRUSOFF.** *Biochem. Pharmacol.,* 1973, vol. 22, 3099
- **BLOCK et al.** *Meths. Enzymol,* 1986, vol. 125, 658
- Remingtons Pharmaceutical Sciences. Mack Publishing Co, 1985

- **SAMBROOK, J. ; FRITSCH, E.F. ; MANIATIS, T.** Molecular Cloning: A Laboratory Manual. Cold Spring Harbor Laboratory Press, 1989
- **NECKELMANN et al.** *Proc. Nat'l. Acad. Sci. U.S.A.,* 1987, vol. 84, 7580-7584
- **BATTINI et al.** *J. Biol. Chem.,* 1987, vol. 262, 4355-4359
- **AQUILA et al.** *Hoppe-Seyler's Z. Physiol. Chem.,* 1982, vol. 363, 345-349
- **STERLING.** *Endocrinology,* 1986, vol. 119, 292-295
- **KLINGENBERG et al.** *Biochim. et Biophys. Acta,* 1978, vol. 503, 193-210
- **STERLING.** *Endrocrinol.,* 1986, vol. 119, 292-295
- **BERMAN et al.** *Biochemistry,* 1985, vol. 24, 7140-7147
- **KLINGENBERG et al.** *Biochim. Biophys. Acta,* 1978, vol. 503, 193-210
- **STERLING.** *Endocrinol,* 1986, vol. 119, 292-295
- **WOLF et al.** *Comput. Appl. Biosci.,* 1988, vol. 4, 187-191
- **COLLAWN ; PATERSON et al.** Short Protocols in Molecular Biology. John Wiley & Sons, 1992, vol. 11, 37-41
- **SMITH et al.** *Anal. Biochem.,* 1985, vol. 150, 76-85
- Expression of Proteins in Insect Cells Using Baculovirus Vectors. **PIWNICA-WORMS et al.** Short Protocols in Molecular Biology. John Wiley & Sons, 1992, 16-32-16-48
- Baculovirus Expression Protocols. **KITTS.** Methods in Molecular Biology. Humana Press, 1995, vol. 39, 129-142
- **STEPIEN et al.** *J. Biol. Chem.,* 1992, vol. 267, 14592-14597
- **FIORE et al.** *Biochimie,* 1998, vol. 80, 137
- **LI et al.** J. Biol. Chem. 1989, vol. 264, 13998
- Transfection of DNA into Eukaryotic Cells. **KINGSTON et al.** Short Protocols in Molecular Biology. John Wiley & Sons, 1992, 9-3-9-16
- **CEPKO et al.** Short Protocols in Molecular Biology. John Wiley & Sons, 1992, 9-30-9-35
- **CEPKO et al.** Short Protocols in Molecular Biology. John Wiley & Sons, 1992, 9-36-9-45
- **CEPKO et al.** Short Protocols in Molecular Biology. John Wiley & Sons, 1992, 9-45-9-48
- **LAUQUIN et al.** *FEBS Letters,* 1976, vol. 67, 306-311
- **BRANDOLIN et al.** *FEBS Lett.,* 1974, vol. 46, 149
- *Acta Chem, Scand.,* 1958, vol. 12, 188
- *J. Org. Chem.,* 1990, vol. 55, 5287
- **AUSUBEL et al.** Current Protocols in Molecular Biology. Greene Publishing, 1987
- **SAMBROOK et al.** Molecular Cloning: A Laboratory Manual. Cold Spring Harbor Press, 1989

SEQUENCE LISTING

<110> MitoKor
Anderson, Christen M.
Davis, Robert E.
Clevenger, William
Wiley, Sandra Eileen
Willer, Scott W.
Szabo, Tomas R.
Ghosh, Soumitra S.

<120> PRODUCTION OF ADENINE NUCLEOTIDE TRANSLOCATOR (ANT), NOVEL ANT LIGANDS AND SCREENING ASSAYS THEREFOR

<130> 660088.443PC

<140> PCT/US
<141> 2001-05-11

<160> 48

<170> FastSEQ for Windows Version 3.0

<210> 1
<211> 894
<212> DNA
<213> Homo sapien

<400> 1
```
atgggtgatc acgcttggag cttcctaaag gacttcctgg ccggggcggt cgccgctgcc     60
gtctccaaga ccgcggtcgc ccccatcgag agggtcaaac tgctgctgca ggtccagcat    120
gccagcaaac agatcagtgc tgagaagcag tacaaaggga tcattgattg tgtggtgaga    180
atccctaagg agcagggctt cctctccttc tggaggggta acctggccaa cgtgatccgt    240
tacttcccca cccaagctct caacttcgcc ttcaaggaca agtacaagca gctcttctta    300
gggggtgtgg atcggcataa gcagttctgg cgctactttg ctggtaacct ggcgtccggt    360
ggggccgctg gggccacctc cctttgcttt gtctacccgc tggactttgc taggaccagg    420
ttggctgctg atgtgggcag gcgcgcccag cgtgagttcc atggtctggg cgactgtatc    480
atcaagatct tcaagtctga tggcctgagg gggctctacc agggtttcaa cgtctctgtc    540
caaggcatca ttatctatag agctgcctac ttcggagtct atgatactgc caaggggatg    600
ctgcctgacc ccaagaacgt gcacattttt gtgagctgga tgattgccca gagtgtgacg    660
gcagtcgcag ggctgctgtc ctaccccttt gacactgttc gtcgtagaat gatgatgcag    720
tccggccgga aaggggccga tattatgtac acggggacag ttgactgctg gaggaagatt    780
gcaaaagacg aaggagccaa ggccttcttc aaaggtgcct ggtccaatgt gctgagaggc    840
atgggcggtg cttttgtatt ggtgttgtat gatgagatca aaaaatatgt ctaa          894
```

<210> 2
<211> 897
<212> DNA
<213> Homo sapien

<400> 2
```
atgacagatg ccgcattgtc cttcgccaag gacttcctgg caggtggagt ggccgcagcc     60
atctccaaga cggcggtagc gcccatcgag cgggtcaagc tgctgctgca ggtgcagcat    120
gccagcaagc agatcactgc agataagcaa tacaaaggca ttatagactg cgtggtccgt    180
attcccaagg agcaggaagt tctgtccttc tggcgcggta acctggccaa tgtcatcaga    240
tacttcccca cccaggctct taacttcgcc ttcaaagata aatacaagca gatcttcctg    300
ggtggtgtgg acaagagaac ccagttttgg cgctactttg cagggaatct ggcatcgggt    360
ggtgccgcag ggccacatc cctgtgtttt gtgtaccctc ttgattttgc ccgtacccgt     420
ctagcagctg atgtgggtaa agctggagct gaaagggaat tccgaggcct cggtgactgc    480
ctggttaaga tctacaaatc tgatgggatt aagggcctgt accaaggctt taacgtgtct    540
```

gtgcagggta ttatcatcta ccgagccgcc tacttcggta tctatgacac tgcaaaggga 600
atgcttccgg atcccaagaa cactcacatc gtcatcagct ggatgatcgc acagactgtc 660
actgctgttg ccgggttgac ttcctatcca tttgacaccg ttcgccgccg catgatgatg 720
cagtcagggc gcaaaggaac tgacatcatg tacacaggca cgcttgactg ctggcggaag 780
attgctcgtg atgaaggagg caaagctttt ttcaagggtg catggtccaa tgttctcaga 840
ggcatgggtg gtgcttttgt gcttgtcttg tatgatgaaa tcaagaagta cacataa 897

<210> 3
<211> 897
<212> DNA
<213> Homo sapien

<400> 3
atgacggaac aggccatctc cttcgccaaa gacttcttgg ccggaggcat cgccgccgcc 60
atctccaaga cggccgtggc tccgatcgag cgggtcaagc tgctgctgca ggtccagcac 120
gccagcaagc agatcgccgc cgacaagcag tacaagggca tcgtggactg cattgtccgc 180
atccccaagg agcagggcgt gctgtccttc tggaggggca accttgccaa cgtcattcgc 240
tacttcccca ctcaagccct caacttcgcc ttcaaggata agtacaagca gatcttcctg 300
gggggcgtgg acaagcacac gcagttctgg aggtactttg cgggcaacct ggcctccggc 360
ggtgcggccg gcgcgacctc cctctgcttc gtgtacccgc tggattttgc cagaacccgc 420
ctggcagcgg acgtgggaaa gtcaggcaca gagcgcgagt tccgaggcct gggagactgc 480
ctggtgaaga tcaccaagtc cgacggcatc cggggcctgt accagggctt cagtgtctcc 540
gtgcagggca tcatcatcta ccgggcggcc tacttcggcg tgtacgatac ggccaagggc 600
atgctccccg accccaagaa cacgcacatc gtggtgagct ggatgatcgc gcagaccgtg 660
acggccgtgg ccggcgtggt gtcctacccc ttcgacacgg tgcggcggcg catgatgatg 720
cagtccgggc gcaaaggagc tgacatcatg tacacgggca ccgtcgactg ttggaggaag 780
atcttcagag atgagggggg caaggccttc ttcaagggtg cgtggtccaa cgtcctgcgg 840
ggcatggggg gcgccttcgt gctggtcctg tacgacgagc tcaagaaggt gatctaa 897

<210> 4
<211> 43
<212> DNA
<213> PCRArtificial Sequence

<220>
<223> PCR Primer

<400> 4
ttatatctcg agtatgggtg atcacgcttg gagcttccta aag 43

<210> 5
<211> 43
<212> DNA
<213> Artificial Sequence

<220>
<223> PCR Primer

<400> 5
tatataggta ccttagacat attttttgat ctcatcatac aac 43

<210> 6
<211> 43
<212> DNA
<213> Artificial Sequence

<220>
<223> PCR Primer

<400> 6
ttatatctcg agtatgacag atgccgctgt gtccttcgcc aag 43

<210> 7
<211> 43
<212> DNA
<213> Artificial Sequence

<220>
<223> PCR Primer

<400> 7
tatataggta ccttatgtgt acttcttgat ttcatcatac aag 43

<210> 8
<211> 43
<212> DNA
<213> Artificial Sequence

<220>
<223> PCR Primer

<400> 8
ttatatctcg agtatgacgg aacaggccat ctccttcgcc aaa 43

<210> 9
<211> 44
<212> DNA
<213> Artificial Sequence

<220>
<223> PCR Primer

<400> 9
tatataggta ccttagagtc accttcttga gctcgtcgta cagg 44

<210> 10
<211> 21
<212> DNA
<213> Artificial Sequence

<220>
<223> Sequence primer

<400> 10
tatgccatag catttttatc c 21

<210> 11
<211> 18
<212> DNA
<213> Artificial Sequence

<220>
<223> PCR Primer

<400> 11
cgccaaaaca gccaagct 18

<210> 12
<211> 45
<212> DNA
<213> Artificial Sequence

<220>
<223> Mutagenic oligonucleotide primer

<400> 12
ggagatggcc tgttccgtca tcttatcgtc atcgtcgtac agatc 45

<210> 13
<211> 45
<212> DNA
<213> Artificial Sequence

<220>
<223> PCR Primer

<400> 13
gatctgtacg acgatgacga taagatgacg gaacaggcca tctcc 45

<210> 14
<211> 35
<212> DNA
<213> Artificial Sequence

<220>
<223> PCR primer

<400> 14
cccggggaat tctgatgacg gaacaggcca tctcc 35

<210> 15
<211> 34
<212> DNA
<213> Artificial Sequence

<220>
<223> PCR Primer

<400> 15
cccgggctcg agttagagtc accttcttga gctc 34

<210> 16
<211> 41
<212> DNA
<213> Artificial Sequence

<220>
<223> PCR Primer

<400> 16
ttataggatc catgacggaa caggccatct ccttcgccaa a 41

<210> 17
<211> 41
<212> DNA
<213> Artificial Sequence

<220>
<223> PCR Primer

<400> 17
ttaaagaatt cttagatcac cttcttgagc tcgtcgtaca g 41

<210> 18
<211> 18
<212> DNA

<213> Artificial Sequence

<220>
<223> Sequencing primer

<400> 18
aaatgataac catctcgc 18

<210> 19
<211> 18
<212> DNA
<213> Artificial Sequence

<220>
<223> PCR Primer

<400> 19
acttcaagga gaatttcc 18

<210> 20
<211> 18
<212> DNA
<213> Artificial Sequence

<220>
<223> PCR Primer

<400> 20
acttcgcctt cacggata 18

<210> 21
<211> 18
<212> DNA
<213> Artificial Sequence

<220>
<223> PCR Primer

<400> 21
tacggccaag ggcattct 18

<210> 22
<211> 18
<212> DNA
<213> Artificial Sequence

<220>
<223> PCR Primer

<400> 22
tgaagcggaa gttcctat 18

<210> 23
<211> 18
<212> DNA
<213> Artificial Sequence

<220>
<223> PCR Primer

<400> 23
atgccggttc ccgtacga 18

<210> 24
<211> 31
<212> DNA
<213> Artificial Sequence

<220>
<223> Mutagenic oligonucleotide primer

<400> 24
ggcctgttcc gtcatcttat cgtcatcgtc g 31

<210> 25
<211> 31
<212> DNA
<213> Artificial Sequence

<220>
<223> PCR Primer

<400> 25
cgacgatgac gataagatga cggaacaggc c 31

<210> 26
<211> 41
<212> DNA
<213> Artificial Sequence

<220>
<223> PCR primer

<400> 26
ttaaagaatt catgacggaa caggccatct ccttcgccaa a 41

<210> 27
<211> 41
<212> DNA
<213> Artificial Sequence

<220>
<223> PCR Primer

<400> 27
ttataggatc cttagatcac cttcttgagc tcgtcgtaca g 41

<210> 28
<211> 42
<212> DNA
<213> Artificial Sequence

<220>
<223> PCR Primer

<400> 28
ttaatgggta ccatgacgga acaggccatc tccttcgcca aa 42

<210> 29
<211> 42
<212> DNA
<213> Artificial Sequence

<220>

<223> PCR Primer

<400> 29
ttatactcga gttagatcac cttcttgagc tcgtcgtaca gg 42

<210> 30
<211> 15
<212> PRT
<213> Artificial Sequence

<220>
<223> Synthetic polypeptide

<400> 30
Cys Trp Arg Lys Ile Phe Arg Asp Glu Gly Gly Lys Ala Phe Phe
1 5 10 15

<210> 31
<211> 297
<212> PRT
<213> Homo sapien

<400> 31
Met Gly Asp His Ala Trp Ser Phe Leu Lys Asp Phe Leu Ala Gly Ala
1 5 10 15
Val Ala Ala Ala Val Ser Lys Thr Ala Val Ala Pro Ile Glu Arg Val
20 25 30
Lys Leu Leu Leu Gln Val Gln His Ala Ser Lys Gln Ile Ser Ala Glu
35 40 45
Lys Gln Tyr Lys Gly Ile Ile Asp Cys Val Val Arg Ile Pro Lys Glu
50 55 60
Gln Gly Phe Leu Ser Phe Trp Arg Gly Asn Leu Ala Asn Val Ile Arg
65 70 75 80
Tyr Phe Pro Thr Gln Ala Leu Asn Phe Ala Phe Lys Asp Lys Tyr Lys
85 90 95
Gln Leu Phe Leu Gly Gly Val Asp Arg His Lys Gln Phe Trp Arg Tyr
100 105 110
Phe Ala Gly Asn Leu Ala Ser Gly Gly Ala Ala Gly Ala Thr Ser Leu
115 120 125
Cys Phe Val Tyr Pro Leu Asp Phe Ala Arg Thr Arg Leu Ala Ala Asp
130 135 140
Val Gly Arg Arg Ala Gln Arg Glu Phe His Gly Leu Gly Asp Cys Ile
145 150 155 160
Ile Lys Ile Phe Lys Ser Asp Gly Leu Arg Gly Leu Tyr Gln Gly Phe
165 170 175
Asn Val Ser Val Gln Gly Ile Ile Ile Tyr Arg Ala Ala Tyr Phe Gly
180 185 190
Val Tyr Asp Thr Ala Lys Gly Met Leu Pro Asp Pro Lys Asn Val His
195 200 205
Ile Phe Val Ser Trp Met Ile Ala Gln Ser Val Thr Ala Val Ala Gly
210 215 220
Leu Leu Ser Tyr Pro Phe Asp Thr Val Arg Arg Arg Met Met Met Gln
225 230 235 240
Ser Gly Arg Lys Gly Ala Asp Ile Met Tyr Thr Gly Thr Val Asp Cys
245 250 255
Trp Arg Lys Ile Ala Lys Asp Glu Gly Ala Lys Ala Phe Phe Lys Gly
260 265 270
Ala Trp Ser Asn Val Leu Arg Gly Met Gly Gly Ala Phe Val Leu Val
275 280 285
Leu Tyr Asp Glu Ile Lys Lys Tyr Val
290 295

<210> 32
<211> 298
<212> PRT
<213> Homo sapien

<400> 32
Met Thr Asp Ala Ala Leu Ser Phe Ala Lys Asp Phe Leu Ala Gly Gly
1               5                   10                  15
Val Ala Ala Ala Ile Ser Lys Thr Ala Val Ala Pro Ile Glu Arg Val
            20                  25                  30
Lys Leu Leu Leu Gln Val Gln His Ala Ser Lys Gln Ile Thr Ala Asp
        35                  40                  45
Lys Gln Tyr Lys Gly Ile Ile Asp Cys Val Val Arg Ile Pro Lys Glu
    50                  55                  60
Gln Glu Val Leu Ser Phe Trp Arg Gly Asn Leu Ala Asn Val Ile Arg
65                  70                  75                  80
Tyr Phe Pro Thr Gln Ala Leu Asn Phe Ala Phe Lys Asp Lys Tyr Lys
                85                  90                  95
Gln Ile Phe Leu Gly Gly Val Asp Lys Arg Thr Gln Phe Trp Arg Tyr
            100                 105                 110
Phe Ala Gly Asn Leu Ala Ser Gly Gly Ala Ala Gly Ala Thr Ser Leu
        115                 120                 125
Cys Phe Val Tyr Pro Leu Asp Phe Ala Arg Thr Arg Leu Ala Ala Asp
    130                 135                 140
Val Gly Lys Ala Gly Ala Glu Arg Glu Phe Arg Gly Leu Gly Asp Cys
145                 150                 155                 160
Leu Val Lys Ile Tyr Lys Ser Asp Gly Ile Lys Gly Leu Tyr Gln Gly
                165                 170                 175
Phe Asn Val Ser Val Gln Gly Ile Ile Ile Tyr Arg Ala Ala Tyr Phe
            180                 185                 190
Gly Ile Tyr Asp Thr Ala Lys Gly Met Leu Pro Asp Pro Lys Asn Thr
        195                 200                 205
His Ile Val Ile Ser Trp Met Ile Ala Gln Thr Val Thr Ala Val Ala
    210                 215                 220
Gly Leu Thr Ser Tyr Pro Phe Asp Thr Val Arg Arg Arg Met Met Met
225                 230                 235                 240
Gln Ser Gly Arg Lys Gly Thr Asp Ile Met Tyr Thr Gly Thr Leu Asp
                245                 250                 255
Cys Trp Arg Lys Ile Ala Arg Asp Glu Gly Gly Lys Ala Phe Phe Lys
            260                 265                 270
Gly Ala Trp Ser Asn Val Leu Arg Gly Met Gly Gly Ala Phe Val Leu
        275                 280                 285
Val Leu Tyr Asp Glu Ile Lys Lys Tyr Thr
    290                 295

<210> 33
<211> 298
<212> PRT
<213> Homo sapien

<400> 33
Met Thr Glu Gln Ala Ile Ser Phe Ala Lys Asp Phe Leu Ala Gly Gly
1               5                   10                  15
Ile Ala Ala Ala Ile Ser Lys Thr Ala Val Ala Pro Ile Glu Arg Val
            20                  25                  30
Lys Leu Leu Leu Gln Val Gln His Ala Ser Lys Gln Ile Ala Ala Asp
        35                  40                  45
Lys Gln Tyr Lys Gly Ile Val Asp Cys Ile Val Arg Ile Pro Lys Glu
    50                  55                  60
Gln Gly Val Leu Ser Phe Trp Arg Gly Asn Leu Ala Asn Val Ile Arg
65                  70                  75                  80
Tyr Phe Pro Thr Gln Ala Leu Asn Phe Ala Phe Lys Asp Lys Tyr Lys

```
                        85                  90                  95
Gln Ile Phe Leu Gly Gly Val Asp Lys His Thr Gln Phe Trp Arg Tyr
                100                 105                 110
Phe Ala Gly Asn Leu Ala Ser Gly Gly Ala Ala Gly Ala Thr Ser Leu
            115                 120                 125
Cys Phe Val Tyr Pro Leu Asp Phe Ala Arg Thr Arg Leu Ala Ala Asp
    130                 135                 140
Val Gly Lys Ser Gly Thr Glu Arg Glu Phe Arg Gly Leu Gly Asp Cys
145                 150                 155                 160
Leu Val Lys Ile Thr Lys Ser Asp Gly Ile Arg Gly Leu Tyr Gln Gly
                165                 170                 175
Phe Ser Val Ser Val Gln Gly Ile Ile Ile Tyr Arg Ala Ala Tyr Phe
            180                 185                 190
Gly Val Tyr Asp Thr Ala Lys Gly Met Leu Pro Asp Pro Lys Asn Thr
        195                 200                 205
His Ile Val Val Ser Trp Met Ile Ala Gln Thr Val Thr Ala Val Ala
    210                 215                 220
Gly Val Val Ser Tyr Pro Phe Asp Thr Val Arg Arg Arg Met Met Met
225                 230                 235                 240
Gln Ser Gly Arg Lys Gly Ala Asp Ile Met Tyr Thr Gly Thr Val Asp
                245                 250                 255
Cys Trp Arg Lys Ile Phe Arg Asp Glu Gly Gly Lys Ala Phe Phe Lys
            260                 265                 270
Gly Ala Trp Ser Asn Val Leu Arg Gly Met Gly Gly Ala Phe Val Leu
        275                 280                 285
Val Leu Tyr Asp Glu Leu Lys Lys Val Ile
    290                 295
```

<210> 34
<211> 28
<212> DNA
<213> Artificial Sequence

<220>
<223> PCR Primer

<400> 34
ctcgagatgg gggatcaggc tttgagct 28

<210> 35
<211> 33
<212> DNA
<213> Artificial Sequence

<220>
<223> PCR Primer

<400> 35
ggtaccttac acatattttt tgatctcatc ata 33

<210> 36
<211> 28
<212> DNA
<213> Artificial Sequence

<220>
<223> PCR Primer

<400> 36
ctcgagatga cagatgccgc tgtgtcct 28

<210> 37

<211> 31
<212> DNA
<213> Artificial Sequence

<220>
<223> PCR Primer

<400> 37
ggtaccttat gtgtacttct tgatttcatc a 31

<210> 38
<211> 19
<212> PRT
<213> Artificial Sequence

<220>
<223> Synthetic peptide derived from ANT3 polypeptide sequence

<400> 38
Cys Ser Gly Thr Glu Arg Glu Phe Arg Gly Leu Gly Asp Cys Leu Val
1 5 10 15
Lys Ile Thr

<210> 39
<211> 16
<212> PRT
<213> Artificial Sequence

<220>
<223> Synthetic peptide derived from ANT2 polypeptide sequence

<400> 39
Met Thr Asp Ala Ala Val Ser Phe Ala Lys Asp Phe Leu Ala Gly Cys
1 5 10 15

<210> 40
<211> 16
<212> PRT
<213> Artificial Sequence

<220>
<223> Synthetic peptide derived from ANT1 polypeptide sequence

<400> 40
Met Gly Asp His Ala Trp Ser Phe Leu Lys Asp Leu Leu Ala Gly Cys
1 5 10 15

<210> 41
<211> 42
<212> DNA
<213> Artificial Sequence

<220>
<223> PCR Primer

<400> 41
ttatatggta ccatgggtga tcacgcttgg agcttcctaa ag 42

<210> 42
<211> 40
<212> DNA
<213> Artificial Sequence

<220>
<223> PCR Primer

<400> 42
tatatagggc ccttagacat attttttgat ctcatccaac 40

<210> 43
<211> 42
<212> DNA
<213> Artificial Sequence

<220>
<223> PCR Primer

<400> 43
ttatatggta ccatgacaga tgccgctgtg tccttcgcca ag 42

<210> 44
<211> 43
<212> DNA
<213> Artificial Sequence

<220>
<223> PCR Primer

<400> 44
tatatagggc ccttatgtgt acttcttgat ttcatcatac aag 43

<210> 45
<211> 42
<212> DNA
<213> Artificial Sequence

<220>
<223> PCR Primer

<400> 45
ttatatggta ccatgacgga acaggccatc tccttcgcca aa 42

<210> 46
<211> 43
<212> DNA
<213> Artificial Sequence

<220>
<223> PCR Primer

<400> 46
tatatagggc ccttagatca ccttcttgag ctcgtcgtac agg 43

<210> 47
<211> 42
<212> DNA
<213> Artificial Sequence

<220>
<223> PCR Primer

```
<400> 47
ttatatggta ccatgggtga tcacgcttgg agcttcctaa ag                    42

<210> 48
<211> 40
<212> DNA
<213> Artificial Sequence

<220>
<223> PCR Primer

<400> 48
tatatagggc ccttagacat attttttgat ctcatccaac                       40
```